(19) Europäisches Patentamt
European Patent Office
Office européen des brevets

(11) EP 1 663 244 B1

(12) **EUROPEAN PATENT SPECIFICATION**

(45) Date of publication and mention of the grant of the patent:
**15.08.2007 Bulletin 2007/33**

(21) Application number: **04765120.3**

(22) Date of filing: **13.09.2004**

(51) Int Cl.:
*A61K 31/519* (2006.01) *A61K 31/5377* (2006.01)
*A61K 31/541* (2006.01) *A61P 29/00* (2006.01)
*A61P 31/00* (2006.01) *A61P 39/00* (2006.01)
*A61P 3/00* (2006.01) *A61P 25/28* (2006.01)
*A61P 35/02* (2006.01) *A61P 1/16* (2006.01)

(86) International application number:
**PCT/EP2004/010198**

(87) International publication number:
**WO 2005/025574 (24.03.2005 Gazette 2005/12)**

(54) **PTERIDINE DERIVATIVES FOR THE TREATMENT OF TNF-ALPHA-RELATED DISEASES.**

PTERIDIN-DERIVATE ZUR BEHANDLUNG VON ERKRANKUNGEN IM ZUSAMMENHANG MIT TNF-ALPHA

DERIVES DE PTERIDINE POUR LE TRAITEMENT DE MALADIES LIEES AU TNF-ALPHA

(84) Designated Contracting States:
**AT BE BG CH CY CZ DE DK EE ES FI FR GB GR HU IE IT LI LU MC NL PL PT RO SE SI SK TR**

(30) Priority: **12.09.2003 GB 0321384**
**22.04.2004 GB 0408955**

(43) Date of publication of application:
**07.06.2006 Bulletin 2006/23**

(73) Proprietor: **4 AZA IP NV**
**3000 Leuven (BE)**

(72) Inventors:
- **WAER, Mark, Jozef, Albert**
  **B-3001 Heverlee (BE)**
- **HERDEWIJN, Piet, André, Maurits, Maria**
  **B-3111 Rotselaar/Wezemaal (BE)**
- **DE JONGHE, Steven, Cesar, Alfons**
  **B-1030 Brussel (BE)**
- **MARCHAND, Arnaud, Didier, Marie**
  **B-3001 Heverlee (BE)**
- **YUAN, Lin**
  **Arlington, MA 02474 (US)**
- **EL HASSANE, Sefrioui**
  **B-3000 Leuven (BE)**

(74) Representative: **Bird, Ariane et al**
**Bird Goen & Co,**
**Klein Dalenstraat 42A**
**3020 Winksele (BE)**

(56) References cited:

| | |
|---|---|
| **EP-A- 0 290 819** | **EP-A- 0 362 645** |
| **EP-A- 0 544 445** | **WO-A-00/39129** |
| **WO-A-01/21619** | **WO-A-02/32507** |
| **WO-A-03/062240** | **DD-A1- 267 495** |
| **US-A- 3 122 546** | **US-A- 5 929 046** |

- **MATTER HANS ET AL: "Structural requirements for inhibition of the neuronal nitric oxide synthase (NOS-I): 3D-QSAR analysis of 4-oxo- and 4-amino-pteridine-based inhibitors" JOURNAL OF MEDICINAL CHEMISTRY, vol. 45, no. 14, 4 July 2002 (2002-07-04), pages 2923-2941, XP002313348 ISSN: 0022-2623**
- **Y. LANDRY, J.-P. GIES: "Pharmacologie. Des cibles vers l'indication thérapeutique. Cours et exercices." 2003, DUNOD , PARIS , XP002313503 page 177, paragraphs 2,3**
- **NICOLAUS B J R: "Symbiotic Approach to Drug Design" DECISION MAKING IN DRUG RESEARCH, XX, XX, 1983, pages 173-186, XP002197412**
- **M.H. BEERS, R. BERKOW: "The Merck Manual of Diagnosis and Therapy, Seventeenth Edition" 1999, MERCK RESEARCH LABORATORIES , WHITEHOUSE STATION, N.J. , XP002313611 page 1474, column 1, paragraph 4**
- **M. H. BEERS, R. BERKOW: "The Merck Manual of Diagnosis and Therapy, Seventeenth Edition" 1999, MERCK RESEARCH LABORATORIES , WHITEHOUSE STATION N.J. , XP002313612 page 953, column 2, paragraphs 1,2**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- DATABASE BIOSIS [Online] BIOSCIENCES INFORMATION SERVICE, PHILADELPHIA, PA, US; 1992, MOREB JAN ET AL: "The therapeutic potential of interleukin-1 and tumor necrosis factor on hematopoietic stem cells" XP002313613 Database accession no. PREV199395064200 & LEUKEMIA AND LYMPHOMA, vol. 8, no. 4-5, 1992, pages 267-275, ISSN: 1042-8194
- FROEHLICH LOTHAR G ET AL: "Inhibition of neuronal nitric oxide synthase by 4-amino pteridine derivatives: Structure-activity relationship of antagonists of (6R)-5,6,7,8-tetrahydrobiopterin cofactor" JOURNAL OF MEDICINAL CHEMISTRY, vol. 42, no. 20, 7 October 1999 (1999-10-07), pages 4108-4121, XP002324246 ISSN: 0022-2623

## Description

FIELD OF THE INVENTION

The present invention relates to the use of polysubstituted pteridines for the prevention and/or the treatment of toxic side effects, disorders and diseases related to or resulting from the exposure of patients to abnormally high levels of tumor necrosis factor-alpha (hereinafter referred as TNF-$\alpha$) in general, and particularly following the administration of TNF-$\alpha$ in cancer treatment in humans. This invention also relates to the use of polysubstituted pteridines for the treatment or prevention of alcohol-induced hepatitis and of cachexia.

BACKGROUND OF THE INVENTION

Several 2,4-diaminopteridine derivatives, including methotrexat, are known in the art (for instance, see U.S. Patent No. 2,512,572) as being useful as antineoplastic agents.

Nevertheless, there still is a need in the art for specific and highly therapeutically active compounds, such as drugs for preventing or treating cell proliferative disorders, including cancer. In particular, there is a need in the art to provide anti-cancer drugs which are active in a minor dose or in minimizing the side effects of other known and efficient anti-cancer drugs or radio-active treatments.

Septic shock is a major cause of death in intensive care units (about 150,000 estimated deaths annually in the United States of America, despite treatment with intravenous antibiotics and supportive care) for which very little effective treatment is available at present. Patients with severe sepsis often experience failures of various systems in the body, including the circulatory system, as well as kidney failure, bleeding and clotting. Lipopolysaccharide (hereinafter referred as LPS) is the primary mediator of Gramm-negative sepsis, the most common form of sepsis, by inducing the production of a whole array of macrophage-derived cytokines (such as TNF-$\alpha$; interleukins such as IL-1, IL-6, IL-12; interferon-gamma (hereinafter referred IFN-$\gamma$), etc.). These cytokines may induce other cells. (e.g. T cells, NK cells) to make cytokines as well (e.g. IFN-$\gamma$). In addition, other macrophage products (e.g. nitric oxide, hereinafter referred as NO) may also play a role in the pathogenesis of toxic shock. These substances (e.g. NO) may be induced directly due to microbial interactions or indirectly through the action of proinflammatory cytokines. LPS binds to a serum protein known as LPB and the LPS-LPB complex thus formed is recognized by the CD14 toll-like receptor 4 (hereinafter referred as Tlr 4) complex on mononuclear phagocytes. Tlr4 is a signal transducing unit, the activation of which results in the release of mediators such as TNF-$\alpha$, IL-1$\alpha$, IL-1$\beta$ and IL-6. These cytokines are important for the pathogenesis of shock. Their administration produces the clinical symptoms of septic shock and their blockade partially protects against LPS-induced lethal shock.

Current therapeutic strategies for the treatment of septic shock are directed against LPS (e.g. antibodies against LPS or LBP-34-23) or against the cytokines induced by LPS (e.g. TNF antibodies) or against the receptor for LPS (e.a. CD14). Unfortunately the initial clinical data of these approaches are very disappointing and illustrate the redundancy of receptors and mediators involved in the pathogenesis of toxic shock. For instance flagellin seems to be another toxin that plays a role in Gramm-negative Salmonella shock syndrome and that cannot be prevented or treated by therapeutic strategies directed specifically at LPS.

Clinical trials in humans with TNF-$\alpha$, blocking antibodies (such as the IL-1 receptor antagonist or PAF receptor antagonists) have been unsuccessful yet, as have been approaches to down regulate inflammation (e.g. using prednisolone) or to block endotoxins. These products must be administered very early after the onset of the disease, which is in most cases not possible.

The only drug currently approved by health authorities for the treatment of adult patients with the most serious forms of sepsis, including septic shock, is a genetically engineered version of a naturally occurring human protein, Activated Protein C, known as Xigris® or drotecogin-alpha which shows only moderate efficacy. Furthermore, because Activated Protein C interferes with blood clotting, the most serious side effect associated with Xigris® is bleeding, including bleeding that causes stroke. Thus Xigris® is contra-indicated for patients who have active internal bleeding, or who are more likely to bleed because of certain medical conditions including recent strokes, recent head or spinal surgery or severe head trauma. Beacause treatment with Xigris® comes with potentially serious risks, the benefits and risks of treatment with Xigris® must be carefully weighed for each individual patient.

Therefore there is a strong need in the art for new medications, either alone or in combination with the currently suggested treatments, for treating the most serious forms of life-threatening illnesses caused by severe infection, such as septic shock.

TNF-$\alpha$ is generally considered to be the key mediator in the mammalian response to bacterial infection. It is a strong pro-inflammatory agent that will affect the function of almost any organ system, either directly or by inducing the formation of other cytokines like IL-1 or prostaglandines. TNF-$\alpha$ is also a potent anti-tumor agent. If administered in small quantities to humans, it causes fever, headache, anorexia, myalgia, hypotension, capillary leak syndrome, increased rates of lipolysis and skeletal muscle protein degradation (including cachexia). Its use in cancer treatment is therefore very much limited by its severe side effects.

TNF-α, a pleiotropic cytokine produced mainly by activated macrophages, exerts an *in vitro* cytotoxic action against transformed cells and *in vivo* anti-tumor activities in animal models. However, despite the fact that TNF-α is used in cancer patients especially to treat melanoma and sarcoma, the major problem hampering its use is toxicity. Indeed, TNF-α induces shock-like symptoms such as bowel swelling and damage, liver cell necrosis, enhanced release of inflammatory cytokines such as IL-1 or IL-6, and hypotension probably due to the release of inducers of vessels dilatation such nitric oxide and other proinflammatory cytokines. Cardiovascular toxicity is usually dose-limiting. Hypotension can be severe with systolic blood pressure below 60 mm Hg. Respiratory compromise is common after treatment with TNF-α and may require mechanical ventilation. Upper as well as lower digestive tract symptoms are also common in this type of treatment. Nausea and vomiting can be distressing and in some cases dose-limiting. Watery diarrhea is frequently observed. Neurological sequelae of treatment with TNF-α can also occur.

Hence, compounds that inhibit the toxic effects of TNF-α but that do not inhibit TNF-α anti-tumor effect are highly desirable for the treatment of cancer patients. Presently, several clinical trials involving TNF-α are being developed for the cancer of organs such as liver, lung, kidney and pancreas, which are based on a procedure including the steps of organ isolation, injection of TNF-α into the isolated organ, and reperfusion of the treated organ. However, even for isolated organ perfusion, some TNF-α usually escapes to the general blood circulation and leads to the mortality of about 10% of the patients thus treated. Many patients treated by this procedure also require intensive care unit rescue to cope with the toxic side-effects of such TNF-α treatment.

Combined treatment of TNF-α with alkylating drugs in an isolated organ perfusion model has received considerable attention. TNF-α is currently successfully used in isolated limb perfusion of human cancer patients and, in combination with melphalan arid interferon-gamma, against melanoma, sarcomas and carcinomas.

The gastrointestinal mucosa is very sensitive to chemotherapeutic drugs. Mucositis caused by chemotherapy usually begins rapidly after initiation of the treatment with inflammation and ulceration of the gastrointestinal tract and leading to diarrhea. Severe, potentially life-threatening, diarrhea may require interruption of the chemotheraputic treatment and subsequent dose reduction of the therapeutic agent. The oral cavity is often the place of severe side effects from cancer therapy that adversely affects the quality of life of the patient and its ability to tolerate the therapy. These side effects can be caused by radiotherapy as well as chemotherapy. A relationship between both serum and mucosal levels of TNF-α and IL-1 correlates with nonhematologic toxicities, including mucositis.

Radiation injuries occurring e.g. after a single high-dose irradiation include apoptosis as well as radiation necrosis. Even normal tissues protected by shielding during irradiation may be considerably damaged. It was found in experimental animal models that the radiation injuries after a single high-dose irradiation typically used for the treatment of various malignant tumors consist of radiation necrosis and apoptosis, which were correlated with the expression of TNF-α and TGF-β1.

Irradiation may induce graft-versus-host disease (hereinafter referred as GVHD) in cancer patients. This disease may occur especially in patients receiving allogeneic bone marrow transplantation as a treatment for cancers such as leukemia or lymphoma and can lead to the death of about 25% of the relevant patients. Before bone marrow transplantation, leukaemia patients for example receive either total body or total lymphoid irradiation to suppress their immune system. However, such irradiation induces not only necrosis but also the release of proinflammatory cytokines mainly TNF-α, IL-1 and IL-6 which in turn induce direct host tissues inflammation and activation of donor cells against host antigens leading to GVHD.

Cisplatin is an effective chemotherapeutic agent used in the treatment of a wide variety of both pediatric and adult malignancies, including testicular, germ cell, head and neck (cervical), bladder and lung cancer. Dose-dependent and cumulative nephrotoxicity is the major side effect of cisplatin, sometimes requiring a reduction in dose or discontinuation of the treatment. Other side effects of cisplatin include kidney damage, loss of fertility, harmful effect on a developing baby, temporary drop in bone marrow function causing drop in white blood cell count, anaemia, drop in platelets causing bleeding, loss of appetite, numbness or tingling in limbs, loss of taste, allergic reactions, and hearing disorders (difficulty in hearing some high-pitched sounds, experiencing ringing in the ears). Blurred vision may also be a side effect with high doses of cisplatin. It was shown that TNF-α is a key element in a network of proinflammatory chemokines and cytokines activated in the kidney by cisplatin. Blockade of TNF-α action would prevent the activation of this cytokine network and would provide protection against cisplatin nephrotoxicity. Hence, compounds that inhibit the toxic effects of cisplatin but that do not inhibit cisplatin anti-tumor effects are highly desirable for the treatment of cancer patients.

A surplus of TNF-α also causes a dramatic change of endothelial cells. In particular, TNF-α is an important mediator of skeletal muscle degeneration associated with cachexia, a debilitating syndrome characterized by extreme weight loss and whole-body wasting. Cachexia is usually a secondary condition whereby there is excessive tissue catabolism in combination with deficient anabolism. It is frequently seen in patients afflicted with chronic diseases such as cancer, cardiopulmonary diseases, aging, malabsortive disorders, excessive physical stress, easting disorders and acquired immmuno-deficiency syndrome (AIDS). Some authors consider that the elevated TNF-α values found in at

least 50% of cancer patients in the active stage of the disease can result in cachexia. TNF-α levels in clinically healthy adults, as well as in adult cancer patients, are well documented, for instance by Nenova et al. in Archives of Hellenic Medicine (2000) 17:619-621. Serum TNF-α concentrations in healthy children as well as in children with malignancies are documented for instance by Saarinen et al. in Cancer Research (1990) 50:592-595. A very significant proportion of cancer mortalities result from cachexia rather than from tumor burden. Chronic wasting disease (cachexia) may result when excessive cellular damage results in the release of substances (TNF-α, collagenase, hyaluronidase) that further catabolize the so-called healthy tissue resulting in an inability to assimilate nutrients required for anabolic restructuring of associated tissue.

Infants infected with human immunodeficiency virus type 1 (HIV-1) show growth retardation and severe weight loss that can lead to death. The overproduction of certain cytokines has been implicated as a possible cause for this. For instance, according to Rautonen et al. in AIDS (1991) 5:1319-1325, serum IL-6 concentrations are elevated and associated with elevated TNF-α concentrations in children with HIV infection. Swapan et al. in Journal of Virology (2002) 76:11710-11714 have shown that reduction of TNF-α levels by either anti-TNF-α antibodies or human chorionic gonadotropin inhibits the expression of HIV-1 proteins and prevents cachexia and death.

Very few drugs have been suggested at present for the treatment of cachexia. Some high-dose progestins like megestrol acetate, an agent used for the treatment of metastatic breast cancer, and medroxyprogesterone acetate were shown in randomized clinical trials to provide a statistically significant advantage as regards improved appetite and body weight gain. Hence, compounds that stimulate appetite and body weight gain without inhibiting the anti-tumor effect or anti-viral effect of co-administered drugs are highly desirable for the treatment of cachexia. More specifically, there is a need in the art for treating cachexia by the administration of compounds that reduce TNF-α levels in the serum of humans.

TNF-α is also suspected to play a role, through a possible dual action in the hematopoietic environment, in the development of hematologic malignancies such as idiopathic myelodysplastic syndromes occurring most often in elderly people but also occasionally in children, these syndromes being currently regarded as the early phase of acute leukemia.

WO 00/39129 discloses the use of substituted pteridines for the treatment of *inter alia* autoimmune disorders, inflammation and cancer. WO 01/21619 discloses substituted pteridines for use in the treatment of disorders associated with a disturbed NO metabolism, such as autoimmune, cardiovascular or central nervous system diseases.

There is a strong need in the art to improve, or to provide alternatives to, the existing prophylactic or therapeutic solutions to all the aforesaid diseases. Meeting this need in the art constitutes the main goal of the present invention.

## SUMMARY OF THE INVENTION

The present invention relates to the unexpected finding that a first class of pteridine derivatives having the general formula (I):

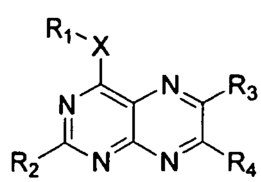

wherein X represents an oxygen atom or a group with the formula $S(O)_m$ wherein m is an integer from 0 to 2, or a group with the formula NZ and wherein:

- $R_1$ is a group selected from the group consisting of methyl, ethyl, isopropyl and pentyl;
- Z is a group independently defined as $R_1$ or Z is hydrogen or the group NZ together with $R_1$ is either hydroxylamino or an optionally substituted heterocyclic group containing at least one nitrogen atom;
- $R_2$ is selected from the group consisting of amino; acylamino;
- $R_4$ is an atom or a group selected from the group consisting of hydrogen; halogen; $C_{1-7}$ alkyl; $C_{2-7}$ alkenyl; $C_{2-7}$ alkynyl; halo $C_{1-7}$ alkyl; carboxy $C_{1-7}$ alkyl; acetoxy $C_{1-7}$ alkyl; carboxyaryl; $C_{1-7}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; amino; hydroxylamino; mercaptoamino; acylamino; thioacylamino; alkoxy-amino; thioalkylamino; acetal; thioacetal; carboxylic acid; carboxylic acid esters, thioesters,

halides, anhydrides, amides and thioamides; thiocarboxylic acid; thiocarboxylic acid esters, thioesters, halides, anhydrides, amides and thioamides; hydroxyl; sulfhydryl; nitro; cyano; carbamoyl; thiocarbamoyl, ureido; thio-ureido; alkylamino; cycloalkylamino; alkenylamino; cycloalkenylamino; alkynylamino; arylamino; arylalkylamino; hydroxyalkylamino; mercaptoalkylamino; heterocyclic amino; heterocyclic-substituted alkylamino; oximino; alkyloximino; hydrazino; alkylhydrazino; phenylhydrazino; cysteinyl acid, esters, thioesters, halides, anhydrides, amides and thioamides thereof; aryl groups optionally substituted with one or more substituents independently selected from the group consisting of halogen, $C_{1-7}$ alkyl, $C_{1-7}$ alkoxy; optionally substituted heterocyclic radicals; aromatic or heterocyclic substituents substituted with an aliphatic spacer between the pteridine ring and the aromatic or heterocyclic substituent, whereby said aliphatic spacer is a branched or straight, saturated or unsaturated aliphatic chain of 1 to 4 carbon atoms; branched or straight, saturated or unsaturated aliphatic chains of 1 to 7 carbon atoms; and

- $R_3$ is an atom or a group defined as $R_4$, or $R_3$ together with $R_4$ forms a homocyclic or heterocyclic radical such as indolyl, dihydroxypyrimidyl or tetramethylene,

as well as pharmaceutically acceptable addition salts, stereoisomers, mono- or di-*N*-oxides, solvates and/or dihydro- or tetrahydropteridine derivatives thereof, are useful for the manufacture of a medicament for the prevention or treatment of a disorder in a mammal, the said disorder being selected from the group consisting of:

- alcohol-induced hepatitis,
- toxic effects of TNF-α, and
- cachexia.

## BRIEF DESCRIPTION OF THE DRAWINGS

Figure 1 represents a scheme for the preparation of 2,4,6-trisubstituted pteridine derivatives used according to one embodiment of this invention, and having various $R_2$ and $R_3$ substituents in the 2- and 6- positions of the pteridine ring, respectively.

Figures 2 to 5 represent alternative schemes for the preparation of 2,4,6- or 2,4,7-trisubstituted or 2,4,6,7-tetrasubstituted pteridine derivatives with various $R_1$, $R_2$, $R_3$ and/or $R_4$ substituents, used according to other embodiments of this invention.

Figure 6 represents a scheme for the preparation of symmetrical 2,4,6- trisubstituted pteridines and 2,4,7-trisubstituted as well as 2,4,6,7-tetrasubstituted pteridine derivatives, i.e. wherein substituents in the 2- and 4- positions of the pteridine ring are identical (reference).

Figure 7 represents a scheme for the preparation of 2,4,6-trisubstituted pteridine derivatives, wherein the substituent in the 6- position of the pteridine ring is a phenyl group substituted by a nitrogen-containing function, used according to one embodiment of this invention.

Figure 8 represents a scheme for the preparation of 2,4,6-trisubstituted pteridine derivatives, wherein the substituent in the 6- position of the pteridine ring is a phenyl group substituted by an oxygen-containing function, used according to another embodiment of this invention.

## DEFINITIONS

Unless otherwise stated herein, the term " trisubstituted " means that three of the carbon atoms being in positions 2, 4 and 6 or, alternatively, in positions 2, 4 and 7 of the pteridine ring (according to standard atom numbering for the pteridine ring) are substituted with an atom or group other than hydrogen. The term " tetrasubstituted " means that all four carbon atoms being in positions 2, 4, 6 and 7 of the pteridine ring are substituted with an atom or group other than hydrogen.
As used herein with respect to a substituting radical, and unless otherwise stated, the terms " $C_{1-7}$ alkyl" or " aliphatic saturated hydrocarbon radicals with 1 to 7 carbon atoms " means straight and branched chain saturated acyclic hydrocarbon monovalent radicals having from 1 to 7 carbon atoms such as, for example, methyl, ethyl, propyl,

n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl; the term " $C_{1-4}$ alkyl " designate the corresponding radicals with only 1 to 4 carbon atoms.

As used herein with respect to a substituting radical, and unless otherwise stated, the term $C_{1-7}$ alkylene means the divalent hydrocarbon radical corresponding to the above defined $C_{1-7}$ alkyl, such as methylene, bis(methylene), tris (methylene), tetramethylene, hexamethylene.

As used herein with respect to a substituting radical, and unless otherwise stated, the terms " $C_{3-10}$ cycloalkyl " and " cycloaliphatic saturated hydrocarbon radical with 3 to 10 carbon atoms " means a monocyclic saturated hydrocarbon monovalent radical having from 3 to 10 carbon atoms, such as for instance cyclopropyl, cyclobutyl, cyclopentyl, cyclohexyl, cycloheptyl, cyclooctyl, or a $C_{7-10}$ polycyclic saturated hydrocarbon monovalent radical having from 7 to 10 carbon atoms such as, for instance, norbornyl, fenchyl, trimethyltricycloheptyl or adamantyl.

As used herein with respect to a substituting radical, and unless otherwise stated, the term " $C_{3-10}$ cycloalkyl-alkyl " refers to an aliphatic saturated hydrocarbon monovalent radical (preferably a $C_{1-7}$ alkyl such as defined above) to which a $C_{3-10}$ cycloalkyl (such as defined above) is already linked such as cyclohexylmethyl and cyclopentylmethyl.

As used herein with respect to a substituting radical, and unless otherwise stated, the term " $C_{3-10}$ cycloalkylene" means the divalent hydrocarbon radical corresponding to the above defined $C_{3-10}$ cycloalkyl such as cyclohexylene.

As used herein with respect to a substituting radical, and unless otherwise stated, the term " aryl " designate any mono- or polycyclic aromatic monovalent hydrocarbon radical having from 6 to 30 carbon atoms such as phenyl, naphthyl, anthracenyl, phenantracyl, fluoranthenyl, chrysenyl, pyrenyl, biphenylyl, terphenyl, picenyl, indenyl, biphenyl, indacenyl, benzocyclobutenyl, benzocyclooctenyl, including fused benzo$C_{4-8}$ cycloalkyl radicals (the latter being as defined above) such as, for instance, indanyl, tetrahydronaphtyl, fluorenyl, each of said radicals being optionally substituted with one or more substituents independently selected from the group consisting of halogen, amino, trifluoromethyl, hydroxyl, sulfhydryl and nitro, such as for instance 4-fluorophenyl, 4-chlorophenyl, 3,4-dichlorophenyl, 4-cyanophenyl, 2,6-dichlorophenyl, 2-fluorophenyl, 3-chlorophenyl and 3,5-dichlorophenyl.

As used herein with respect to a substituting radical such as the combination of $R_3$ and $R_4$ together with the carbon atoms in positions 6 and 7 of the pteridine ring, and unless otherwise stated, the term " homocyclic" means a mono- or polycyclic, saturated or mono-unsaturated or polyunsaturated hydrocarbon radical having from 4 up to 15 carbon atoms but including no heteroatom in the said ring; for instance said combination of $R_3$ and $R_4$ may form a $C_{2-6}$ alkylene radical, such as tetramethylene, which cyclizes with the carbon atoms in positions 6 and 7 of the pteridine ring.

As used herein with respect to a substituting radical (including a combination of substituents in positions 6 and 7 of the pteridine ring together with the carbon atoms in positions 6 and 7 of the pteridine ring), and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in a 3 to 10 membered ring (and optionally one or more heteroatoms attached to one or more carbon atoms of said ring, for instance in the form of a carbonyl or thiocarbonyl group) and/or to one or more heteroatoms of said ring, for instance in the form of a sulfone, sulfoxide, N-oxide, phosphate, phosphonate or selenium oxide, each said heteroatom being independently selected from the group consisting of nitrogen, oxygen, sulfur, selenium and phosphorus, including benzo-fused heterocyclic radicals, such as diazepinyl, oxadiazinyl, thiadiazinyl, dithiazinyl, triazolonyl, diazepinonyl, triazepinyl, triazepinonyl, tetrazepinonyl, benzo-quinolinyl, benzothiazinyl, benzothiazinonyl, benzoxathiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzopyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoisoquinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpholinyl, azlactonyl, naphtindazolyl, naphtindolyl, naphtothiazolyl, naphtothioxolyl, naphtoxindolyl, naphtotriazolyl, naphtopyranyl, oxabicycloheptyl, azabenzimidazolyl, azacycloheptyl, aza-cyclooctyl, azacyclononyl, azabicyclononyl, tetrahydrofuryl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thiourazolyl, thiotriazolyl, thiopyranyl, thiopyronyl, coumarinyl, quinoleinyl, oxyquinoleinyl, quinuclidinyl, xanthinyl, dihydro-pyranyl, benzodihydrofuryl, benzothiopyronyl, benzothiopyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl (benzothiofuranyl), phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, oxetanonyl, thietyl, thietanyl, diazabicyclooctyl, diazetyl, diaziridinonyl, diaziridinethionyl, chromanyl, chromanonyl, thiochromanyl, thiochromanonyl, thiochromenyl, benzofuranyl, benzisothiazolyl, benzocarbazolyl, benzochromonyl, benzisoalloxazinyl, benzocoumarinyl, thiocoumarinyl, phenometoxazinyl, phenoparoxazinyl, phentriazinyl, thiodiazinyl, thiodiazolyl, indoxyl, thio-indoxyl, benzodiazinyl (e.g. phtalazinyl), phtalidyl, phtalimidinyl, phtalazonyl, alloxazinyl, dibenzopyronyl (i.e. xanthonyl), xanthionyl, isatyl, isopyrazolyl, isopyrazolonyl, urazolyl, urazinyl, uretinyl, uretidinyl, succinyl, succinimido, benzylsultimyl, benzylsultamyl and the like, including all possible isomeric forms thereof, wherein each carbon atom of the said ring may be substituted with a substituent selected from the group consisting of halogen, nitro, $C_{1-7}$ alkyl (optionally containing one or more functions or radicals selected from the group consisting of carbonyl (oxo), alcohol (hydroxyl), ether (alkoxy), acetal, amino, imino, oximino, alkyloximino, amino-acid, cyano, carboxylic acid ester or amide, nitro, thio $C_{1-7}$ alkyl, thio $C_{3-10}$ cycloalkyl, $C_{1-7}$ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfonyl, sulfonamido and halogen), $C_{3-7}$ alkenyl, $C_{2-7}$ alkynyl, halo $C_{1-7}$ alkyl, $C_{3-10}$ cycloalkyl, aryl, arylalkyl, alkylaryl, alkylacyl, arylacyl, hydroxyl, amino, $C_{1-7}$ alkylamino, cycloalkylamino, alkenylamino, cyclo-alkenylamino, alkynylamino, arylamino, arylalkylamino, hydroxyalkylamino, mercaptoalkylamino, hetero-cyclic amino, hydrazino, alkylhydrazino, phenylhydrazino, sulfhydryl, $C_{1-7}$ alkoxy, $C_{3-10}$ cycloalkoxy, aryloxy, arylalkyloxy, oxyheterocyclic, heterocyclic-substituted alkyloxy, thio $C_{1-7}$ alkyl, thio $C_{3-10}$ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof; depending upon the number of unsaturations in the 3 to 10 membered ring, heterocyclic radicals may be sub-divided into heteroaromatic (or " heteroaryl") radicals and non-aromatic heterocyclic radicals; when a heteroatom of the said non-aromatic heterocyclic radical is nitrogen, the latter may be substituted with a substituent selected from the group consisting of $C_{1-7}$ alkyl, $C_{3-10}$ cycloalkyl, aryl, arylalkyl and alkylaryl.

As used herein with respect to a substituting radical, and unless otherwise stated, the terms $C_{1-7}$ alkoxy ", " $C_{3-10}$ cycloalkoxy ", " aryloxy", " arylalkyloxy ", " oxyheterocyclic ", "thio $C_{1-7}$ alkyl", " thio $C_{3-10}$ cycloalkyl ", "arylthio ", " arylalkylthio " and " thioheterocyclic " refer to substituents wherein a $C_{1-7}$ alkyl radical, respectively a $C_{3-10}$ cycloalkyl, aryl, arylalkyl or heterocyclic radical (each of them such as defined herein), are attached to an oxygen atom or a sulfur atom through a single bond, such as methoxy, ethoxy, propoxy, butoxy, thioethyl, thiomethyl, phenyloxy, benzyloxy, mercaptobenzyl, cresoxy.

As used herein with respect to a substituting atom, and unless otherwise stated, the term halogen means any atom selected from the group consisting of fluorine, chlorine, bromine and iodine.

As used herein with respect to a substituting radical, and unless otherwise stated, the term " halo $C_{1-7}$ alkyl " means a $C_{1-7}$ alkyl radical (such as above defined) in which one or more hydrogen atoms are independently replaced by one or more halogens (preferably fluorine, chlorine or bromine), such as difluoromethyl, trifluoromethyl, trifluoroethyl, octafluoropentyl, dodecafluoroheptyl, dichloromethyl; the term "halo $C_{1-4}$ alkyl " designate the corresponding radical with only 1 to 4 carbon atoms.

As used herein with respect to a substituting radical, and unless otherwise stated, the terms " $C_{2-7}$ alkenyl " and " aliphatic unsaturated hydrocarbon radical with 2 to 7 carbon atoms " are interchangeable and designate a straight and branched acyclic hydrocarbon monovalent radical having one or more ethylenical unsaturations and having from 2 to 7 carbon atoms such as, for example, vinyl, 2-propenyl, 3-butenyl, 2-butenyl, 2-pentenyl, 3-pentenyl, 3-methyl-2-butenyl, 3-hexenyl, 2-hexenyl, 2-heptenyl, butadienyl, pentadienyl, hexadienyl, heptadienyl, heptatrienyl, including all possible isomers thereof; the term " $C_{3-7}$ alkenyl " designate the corresponding radical with only 3 to 7 carbon atoms.

As used herein with respect to a substituting radical, and unless otherwise stated, the terms " $C_{3-10}$ cycloalkenyl " and " cycloaliphatic unsaturated hydrocarbon radical with 3 to 10 carbon atoms " are interchangeable and mean a monocyclic mono- or polyunsaturated hydrocarbon monovalent radical having from 3 to 8 carbon atoms, such as for instance cyclopropenyl, cyclobutenyl, cyclopentenyl, cyclopentadienyl, cyclohexenyl, cyclohexadienyl, cycloheptenyl, cycloheptadienyl, cyclohepta-trienyl, cyclooctenyl, cyclooctadienyl, or a $C_{7-10}$ polycyclic mono- or polyunsaturated hydrocarbon mono-valent radical having from 7 to 10 carbon atoms such as dicyclopentadienyl, fenchenyl (including all isomers thereof, such as $\alpha$-pinolenyl), bicyclo[2.2.1]hept-2-enyl, bicyclo[2.2.1]hepta-2,5-dienyl, cyclo-fenchenyl.

As used herein with respect to a substituting radical, and unless otherwise stated, the term " $C_{2-7}$ alkynyl " defines straight and branched chain hydrocarbon radicals containing one or more triple bonds and having from 2 to 20 carbon atoms such as, for example, acetylenyl, 2-propynyl, 3-butynyl, 2-butynyl, 2-pentynyl, 3-pentynyl, 3-methyl-2-butynyl, 3-hexynyl, 2-hexynyl and all possible isomers thereof.

As used herein with respect to a substituting radical, and unless otherwise stated, the terms " arylalkyl " ,

" arylalkenyl " and "heterocyclic-substituted alkyl" refer to an aliphatic saturated or ethylenically unsaturated hydrocarbon monovalent radical (preferably a $C_{1-7}$ alkyl or a $C_{2-7}$ alkenyl such as defined above, onto which an aryl radical or respectively a heterocyclic radical (such as defined above) is already bonded, and wherein the said aliphatic radical and/or the said aryl or heterocyclic radical may be optionally substituted with one or more substituents independently selected from the group consisting of $C_{1-4}$ alkyl, trifluoromethyl, halogen, amino, nitro, hydroxyl, sulfhydryl and nitro, such as benzyl, 4-chlorobenzyl, 2-fluorobenzyl, 4-fluorobenzyl, 3,4-dichlorobenzyl, 2,6-dichlorobenzyl, 4-*ter*-butylbenzyl, 3-methylbenzyl, 4-methylbenzyl, phenylpropyl, 1-naphtylmethyl, phenylethyl, 1-amino-2-phenylethyl, 1-amino-2-[4-hydroxy-phenyl]ethyl, 1-amino-2-[indol-2-yl]ethyl, styryl, pyridylmethyl (including all isomers thereof), pyridylethyl, 2-(2-pyridyl)isopropyl, oxazolylbutyl, 2-thienylmethyl, pyrrolylethyl, morpholinyl-ethyl, imidazol-1-yl-ethyl, benzodioxolylmethyl and 2-furylmethyl.

As used herein with respect to a substituting radical, and unless otherwise stated, the term " alkylaryl " and " alkyl-substituted heterocyclic " refer to an aryl radical or respectively a heterocyclic radical (such as defined above) onto which is (are) already bonded one or more aliphatic saturated hydrocarbon monovalent radicals, preferably one or more $C_{1-7}$ alkyl radicals or $C_{3-10}$ cycloalkyl radicals as defined above such as o-toluyl, m-toluyl, p-toluyl, 2,3-xylyl, 2,4-xylyl, 3,4-xylyl, o-cumenyl, m-cumenyl, p-cumenyl, o-cymenyl, m-cymenyl, p-cymenyl, mesityl and 2,4,6-trimethylphenyl, *ter*-butylphenyl, lutidinyl (i.e. dimethylpyridyl), 2-methylaziridinyl, methylbenzimidazolyl, methylbenzofuranyl, methylbenzothiazolyl, methyl-benzotriazolyl, methylbenzoxazolyl and methylbenzselenazolyl.

As used herein with respect to a substituting radical, and unless otherwise stated, the term " alkoxyaryl " refers to an aryl radical (such as defined above) onto which is (are) bonded one or more $C_{1-7}$ alkoxy radicals as defined above, preferably one or more methoxy radicals, such as 2-methoxyphenyl, 3-methoxyphenyl, 4-methoxyphenyl, 3,4-dimethoxyphenyl, 2,4,6-trimethoxyphenyl, methoxynaphtyl.

As used herein with respect to a substituting radical, and unless otherwise stated, the terms " alkylamino ", " cycloalkylamino ", " alkenylamino ", " cycloalkenylamino " , " arylamino ", " arylalkylamino ", " heterocyclic amino " , " hydroxyalkylamino ", " mercaptoalkylamino " and " alkynylamino " mean that respectively one (thus monosubstituted amino) or even two (thus disubstituted amino) $C_{1-7}$ alkyl, $C_{3-10}$ cycloalkyl, $C_{2-7}$ alkenyl, $C_{3-10}$ cycloalkenyl, aryl, arylalkyl, heterocyclic, mono- or polyhydroxy $C_{1-7}$ alkyl, mono- or polymercapto $C_{1-7}$ alkyl or $C_{2-7}$ alkynyl radical(s) (each of them as defined herein, respectively) is/are attached to a nitrogen atom through a single bond or, in the case of heterocyclic, include a nitrogen atom, such as anilino, benzylamino, methylamino, dimethylamino, ethylamino, diethylamino, isopropylamino, propenylamino, n-butylamino, terbutylamino, dibutylamino, morpholino-alkylamino, morpholinyl, piperidinyl, piperazinyl, hydroxymethylamino, β-hydroxyethylamino and ethynylamino; this definition also includes mixed disubstituted amino radicals wherein the nitrogen atom is attached to two such radicals belonging to two different sub-set of radicals, e.g. an alkyl radical and an alkenyl radical, or to two different radicals within the same sub-set of radicals, e.g. methylethylamino; the term " $C_{3-7}$ alkylamino " designates the corresponding radical with only 3 to 7 carbon atoms in the alkyl group(s) attached to nitrogen, for instance di-isopropylamino, and so on; among disubstituted amino radicals, symetrically substituted are usually preferred and more easily accessible.

As used herein with respect to a substituting radical, and unless otherwise stated, the terms " (thio)carboxylic acid ester " , " (thio)carboxylic acid thioester " and " (thio)carboxylic acid amide " refer to radicals wherein the carboxyl or thiocarboxyl group is directly attached to the pteridine ring (e.g. in the 6- and/or 7-position) and wherein said carboxyl or thiocarboxyl group is bonded to the hydrocarbonyl residue of an alcohol, a thiol, a polyol, a phenol, a thiophenol, a primary or secondary amine, a polyamine, an amino-alcohol or ammonia, the said hydrocarbonyl residue being selected from the group consisting of alkyl, alkenyl, alkynyl, cycloalkyl, cycloalkenyl, aryl, arylalkyl, alkylaryl, alkylamino, cycloalkylamino, alkenylamino, cycloalkenylamino, arylamino, arylalkylamino, heterocyclic amino, hydroxyalkylamino, mercapto-alkylamino or alkynylamino (such as above defined, respectively).

As used herein with respect to a substituting radical, and unless otherwise stated, the term " amino-acid " refers to a radical derived from a molecule having the chemical formula $H_2N-CHR-COOH$, wherein R is the side group of atoms characterizing the amino-acid type; said molecule may be one of the 20 naturally-occurring amino-acids or any similar non naturally-occurring amino-acid.

As used herein and unless otherwise stated, the term " stereoisomer " refers to all possible different isomeric as well as conformational forms which the pteridine derivatives having the general formula (I) may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a pteridine derivative of this invention with a suitable inorganic solvent (e.g. hydrates) or organic solvent, such as

alcohols, ketones, esters.

As used herein and unless otherwise stated, the terms " dihydropteridine derivative " and " tetrahydropteridine derivative " refer to the hydrogenation products of the pteridine derivatives having the general formula (I), i.e. derivatives wherein two hydrogen atoms are present in positions 5 and 6, or 7 and 8, of the pteridine ring, or respectively wherein four hydrogen atoms are present in positions 5, 6, 7 and 8 of the said ring; such hydrogenated derivatives are easily accessible from the pteridine derivatives of formula (I) by using hydrogenation methods well known in the art.

DETAILED DESCRIPTION OF THE INVENTION

A main object of the invention is to provide a treatment for a class of TNF-$\alpha$- related disorders in a mammal, the said disorders being selected from the group consisting of:

- alcohol-induced hepatitis,
- toxic effects of TNF-$\alpha$, and
- cachexia.

This is achieved by manufacturing a medicament, or a pharmaceutical composition, including a pteridine derivative having the above mentioned general formula (I) as a biologically active ingredient.

According to the invention, the first class of active pteridine derivatives are as defined in the general formula (I), wherein each of the substituents X, Z, $R_1$, $R_2$, $R_3$ and $R_4$ may correspond to any of the definitions given above (and, when X includes sulfur, wherein m may be 0, 1 or 2), in particular with any of the individual meanings (such as illustrated above) of generic terms such as " $C_{1-7}$ alkyl ", " $C_{2-7}$ alkenyl ", " $C_{2-7}$ alkynyl ", " aryl ", " alkyl-aryl ", "arylalkyl ", " alkylamino ", " cycloalkylamino ", " alkenylamino ", " alkynylamino ", "arylamino ", " arylalkylamino ", " $C_{1-7}$ alkoxy ", " $C_{3-10}$ cycloalkoxy ", " thio $C_{1-7}$ alkyl ", " thio $C_{3-10}$ cycloalkyl ", " halo $C_{1-7}$ alkyl ".

When a mixture of enantiomers of a pteridine derivative having the general formula (I) according to the invention is obtained during synthesis, the said mixture may be separated by means and methods standard in the art, e.g. liquid chromatography using one or more suitable chiral stationary phases. The latter include, for example, polysaccharides, in particular cellulose or amylose derivatives. Commercially available polysaccharide-based chiral stationary phases suitable for this purpose are ChiralCel™ CA, OA, OB, OC, OD, OF, OG, OJ and OK, and Chiralpak™ AD, AS, OP(+) and OT(+). Appropriate eluents or mobile phases for use in combination with said polysaccharide-based chiral stationary phases are hydrocarbons such as hexane, optionally admixed with an alcohol such as ethanol, isopropanol. The above mixture of enantiomers may alternatively be separated by forming diastereoisomers, followed by separation of the diastereoisomers, e.g. by differential crystallization or chromatography. The resolving agent may be cleaved from the separated diastereoisomers, e.g. by treatment with acids or bases, in order to generate the pure enantiomers of the compounds of the invention.

Some preferred pteridine derivatives having the general formula (I) according to the invention are more specifically illustrated in the following examples. For instance, useful pteridine species disclosed below include these wherein:

- $R_1$ is selected from the group consisting of methyl, ethyl, isopropyl and pentyl and/or
- $R_2$ is amino, and/or
- $R_4$ is hydrogen or methoxy, and/or
- $R_3$ is 3-thienyl or 2-thienyl or a phenyl group with one or more substituents (in the latter case, such substituents are preferably each independently selected from the group consisting of fluoro, methoxy, ethoxy, trifluoromethyl, dimethyl-amino, chloro, cyano, methyl, ethyl, carboxymethyl, methylthio, dimethylcarboxamido, diethylcarboxamido and methylcarboxylate, and/or
- X is a sulfur atom (i.e. m is 0) or an oxygen atom, or
- X is NZ, wherein Z is selected from the group consisting of hydrogen, methyl, ethyl, n-propyl and benzyl, or NZ together with $R_1$ forms a radical selected from the group consisting of hydroxylamino, tetrahydropyridinyl, morpholinyl, piperidinyl, piperazinyl, N-methylpiperazinyl and 1,2,4-triazolyl.

Further described are processes and methods for making the pteridine derivatives having the general formula (I) As a general rule, the preparation of these compounds is based on the principle that, starting from a suitable pteridine precursor, each of the substituents $XR_1$, $NH_2$ , $R_2$, $R_3$ and $R_4$ (with respect to general formula (I)), may be introduced separately (except, of course, when $R_3$ together with $R_4$ and the carbon atoms at positions 6 and 7 of the pteridine ring forms a homocyclic or heterocyclic radical) without adversely influencing the presence of one or more substituents already introduced at other positions on the pteridine ring or the capacity to introduce further substituents later on.

Methods of manufacture have been developed by the present inventors which may be used alternatively to, or may be combined with, the methods of synthesis already known in the art of pteridine derivatives (depending upon the targeted final compound). For instance, methods for simultaneously introducing $R_3$ and $R_4$ in the form of a homocyclic or heterocyclic radical at positions 6 and 7 of the pteridine ring are already known from U.S. Patent No. 2,581,889. The synthesis of mono- and di-*N*-oxides of the pteridine derivatives of this invention can easily be achieved by treating the said derivatives with an oxidizing agent such as, but not limited to, hydrogen peroxide (e.g. in the presence of acetic acid) or a peracid such as chloroperbenzoic acid. Dihydro- and tetrahydropteridine derivatives of this invention can easily be obtained by catalytic hydrogenation of the corresponding pteridine derivatives, e.g. by placing the latter in a hydrogen atmosphere in the presence of platinum oxide or platinum. The methods for making the pteridine derivatives of the present invention will now be explained in more details by reference to the appended figures 1 to 8 wherein, unless otherwise stated hereinafter, each of the substituting groups or atoms X, Z, $R_1$, $R_2$, $R_3$ and $R_4$ is as defined in formula (I) of the summary of the invention and, more specifically, may correspond to any of the individual meanings disclosed above. The same manufacturing methods may also be applied, if need be, while starting from pteridine derivatives which are already known in the art. In the description of the reaction steps involved in each figure, reference is made to the use of certain catalysts and/or certain types of solvents. It should be understood that each catalyst mentioned should be used in a catalytic amount well known to the skilled person with respect to the type of reaction involved. Solvents that may be used in the following reaction steps include various kinds of organic solvents such as protic solvents, polar aprotic solvents and non-polar solvents as well as aqueous solvents which are inert under the relevant reaction conditions. More specific examples include aromatic hydrocarbons, chlorinated hydrocarbons, ethers, aliphatic hydrocarbons, alcohols, esters, ketones, amides, water or mixtures thereof, as well as supercritical solvents such as carbon dioxide (while performing the reaction under supercritical conditions). The suitable reaction temperature and pressure conditions applicable to each kind of reaction step will not be detailed herein but do not depart from the relevant conditions already known to the skilled person with respect to the type of reaction involved and the type of solvent used (in particular its boiling point).

Figure 1 represents a scheme for the preparation of 2,4,6-trisubstituted pteridines with various $R_2$ and $R_3$ substituents in the 2- and 6-positions of the pteridine ring. In the first step (a), a chloropyrimidine 1, wherein $R_2$ may be *inter alia* amino, alkylamino, arylamino, alkoxy, aryloxy, mercaptoalkyl, or mercaptoaryl, is reacted with an appropriate nucleophile $R_1XH$, the said nucleophile being selected from the group consisting of alcohols (e.g. methanol, ethanol, isopropanol or benzylalcohol), thiols, primary amines and secondary amines wherein $R_1$ may be *inter alia* alkyl, cycloalkyl, aryl, alkylaryl, heteroaryl or alkylheteroaryl. Introduction of a nitroso group into the pyrimidine intermediate 2 occurs in step (b) under acidic aqueous conditions in the presence of sodium nitrite $NaNO_2$. Reduction of the nitroso functionality of the pyrimidine intermediate 3 into a free amino group in intermediate 4 is then effected in step (c) by means of reducing agents (such as $Na_2S_2O_4$ or $(NH_4)_2S$) in water, or catalytically ($Pt/H_2$) in the presence of a protic solvent. In step (d), ring closure is performed by treating the diaminopyrimidine 4 with glyoxal in order to form a pteridine ring. In step (e), the nitrogen atom at position 8 of the pteridine ring of compound 5 is oxidized, e.g. using $H_2O_2$ under acidic conditions. In step (f), a chlorine atom is regioselectively introduced on the 6 position of the pteridine ring of compound 6 by treatment with a carboxylic acid chloride such as acetyl chloride under acidic conditions. Then in step (g) the 6-chlorosubstituted pteridine 7 is reacted with a boronic acid having the general formula $R_3B(OH)_2$, wherein $R_3$ may be alkyl, cycloalkyl, aryl or heteroaryl, under basic conditions (such as in the presence of an aqueous alkaline solution) and a palladium based catalyst, thus yielding the desired derivative 8 of the present invention.

Figure 2 represents a scheme for the preparation of 2,4,6- or 2,4,7-trisubstituted or 2,4,6,7-tetrasubstituted pteridine derivatives with various $R_1$, $R_2$, $R_3$ and/or $R_4$ substituents. In step (a), the thiol function of 2-mercapto-4,6-diaminopyrimidine is alkylated, preferably methylated by reaction with methyl iodide in the presence of a solvent such as ethanol, in order to yield 2-thiomethyl-4,6-diaminopyrimidine. Introduction of a nitroso group in the 5-position of the pyrimidine ring is then achieved in step (b) by using sodium nitrite under aqueous acidic conditions. In step (c), the methylthio group in the 2-position is exchanged for a group $R_2$ by reaction with an appropriate nucleophile, wherein $R_2$ is as defined above and preferably is primary or secondary amino, $C_{1-7}$ alkoxy, aryloxy, $C_{3-10}$ cycloalkoxy, heteroaryloxy, mercapto $C_{1-7}$alkyl, mercaptoaryl, mercapto $C_{3-10}$cycloalkyl or mercapto-heteroaryl. Reduction of the nitroso group is then achieved in step (d) either catalytically ($Pt/H_2$) in the presence of a protic solvent or chemically using sodium dithionite or ammonium sulfide in the presence of water. Then in step (e), the resulting 2-$R_2$-substituted-4,5,6-triaminopyrimidine is condensed, under acidic conditions in the presence of a solvent such as methanol, with an $\alpha$-ketoaldoxime bearing the group $R_3$, wherein $R_3$ may be $C_{1-7}$ alkyl, $C_{3-10}$ cycloalkyl, aryl or heteroaryl, into a 2,6-substituted-4-aminopteridine derivative. Alternatively, the corresponding 2,7-substituted-4-aminopteridine derivative can be obtained in step (f) by reacting the 2-$R_2$-substituted-4,5,6-triaminopyrimidine with a monosubstituted glyoxal bearing a group $R_4$, wherein $R_4$ may be inter alia $C_{1-7}$ alkyl, $C_{3-10}$ cycloalkyl, aryl or heteroaryl. Alternatively, a 2-$R_2$-substituted 4-amino-6,7-disubstituted pteridine derivative can be obtained in step (g) by reacting the 2-$R_2$-substituted 4,5,6-triaminopyrimidine with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under

neutral or basic conditions. In step (h), acidic or basic hydrolysis of the amino group at position 4 of the pteridine ring is performed and results in the corresponding 4-oxopteridine derivative. In step (i), the hydroxyl group of the tautomeric form of the latter is activated by nucleophilic displacement, e.g. by preparing the 4-[(1,2,4)-triazolyl] pteridine derivative. Finally in a first part of step (j), a nucleophilic displacement is performed by mixing the said 4-triazolylpteridine derivative with a nucleophile having the general formula $R_1XH$, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl yielding the desired final pteridine derivatives.

Figure 3 represents a scheme for the preparation of 2,4,6- or 2,4,7-trisubstituted or 2,4,6,7-tetrasubstituted pteridine derivatives with various substituents on the pteridine ring, starting from the 2-thiomethyl-5-nitroso-4,6-diaminopyrimidine obtained after step (b) of the scheme shown in figure 2. Reduction of the nitroso group is achieved in step (a) either catalytically ($Pt/H_2$) in the presence of a protic solvent or chemically using sodium dithionite or ammonium sulfide in the presence of water. Then in step (b), 2-thiomethyl-4,5,6-triamino-pyrimidine is condensed, under acidic conditions in the presence of a solvent such as methanol, with an $\alpha$-ketoaldoxime bearing the group $R_3$, wherein $R_3$ may be *inter alia* $C_{1-7}$ alkyl, $C_{3-10}$ cycloalkyl, aryl or heteroaryl, thus regioselectively yielding a 2-thiomethyl-4-amino-6-$R_3$-substituted-pteridine derivative. Alternatively, the corresponding 2-thiomethyl-4-amino-7-$R_4$-substituted pteridine is obtained in step (c) by reacting 2-thiomethyl-4,5,6-triamino-pyrimidine with a monosubstituted glyoxal bearing a group $R_4$, wherein $R_4$ may be *inter alia* $C_{1-7}$ alkyl, $C_{3-10}$ cycloalkyl, aryl or heteroaryl. Alternatively, the corresponding 2-thiomethyl-4-amino-6-$R_3$-7-$R_4$-substituted pteridine is obtained in step (d) by reacting 2-thiomethyl-4,5,6-triamino-pyrimidine with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under neutral or basic conditions. In step (e), the methylthio group in the 2-position is oxidized to the corresponding sulfone by using oxidizing agents such as chloroperoxybenzoic acid in chloroform or hydrogen peroxide in acetic acid. The methylsulfonyl group is easily exchanged in step (f) by reaction with a nucleophile, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl. In step (g), acidic or basic hydrolysis of the amino group at position 4 of the pteridine ring is performed and results in the corresponding 4-oxopteridine derivative. In step (h), the hydroxyl group of the tautomeric form of the latter is activated by nucleophilic displacement, e.g. by preparing the 4-[(1,2,4)-triazolyl] pteridine derivative. In the last step (i), a nucleophilic displacement is performed by mixing the said 4-triazolylpteridine derivative with a nucleophile having the general formula $R_1XH$, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl.

Figure 4 represents a scheme for the synthesis of unsymmetrical 2,4,6-trisubstituted and 2,4,7-trisubstituted, as well as 2,4,6,7-tetrasubstituted, pteridine derivatives with various $R_1$, $R_2$, $R_3$ and/or $R_4$ substituents in the 2-, 4-, 6- and/or 7-positions of the pteridine ring, respectively. In step (a), a 2-$R_2$-substituted-4,5,6-triamino-pyrimidine is condensed with an $\alpha$-keto-aldoxime bearing a radical $R_3$, wherein $R_3$ may be *inter alia* selected from the group consisting of $C_{1-7}$ alkyl, $C_{3-10}$ cycloalkyl, aryl and heteroaryl, in a protic solvent such as methanol under acidic conditions yielding regioselectively a 4-amino-pteridine bearing a $R_2$-substituent in position 2 and a $R_3$ substituent in position 6 of the pteridine ring. Alternatively, a 2-$R_2$-substituted-4-amino-7-$R_4$-substituted pteridine derivative can be obtained in step (b) by reacting a 2-$R_2$-substituted-4,5,6-triamino-pyrimidine with a monosubstituted glyoxal bearing the group $R_4$, wherein $R_4$ may be *inter alia* selected from the group consisting of $C_{1-7}$ alkyl, $C_{3-10}$ cycloalkyl, aryl and heteroaryl, under neutral or basic conditions. Alternatively, a 2-$R_2$-substituted-4-amino-6,7-di-substituted pteridine derivative can be obtained in step (c) by reacting a 2-$R_2$-substituted-4,5,6-triamino-pyrimidine with a disubstituted glyoxal bearing the groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl or heteroaryl, under neutral or basic conditions. In step (d), acidic or basic hydrolysis of the amino group at position 4 of the pteridine ring is performed and results in the corresponding 4-oxopteridine derivative. In step (e), the hydroxyl group of the tautomeric form of the latter is activated for a nucleophilic displacement reaction, e.g. by preparing the 4-[(1,2,4)-triazolyl]pteridine derivative. In the last step (f), the 4-triazolylpteridine derivative is reacted with a nucleophile having the general formula $R_1XH$, such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl.

Figure 5 represents a scheme for the synthesis of symmetrical 2,4,6-trisubstituted and 2,4,7-trisubstituted, as well as 2,4,6,7-tetrasubstituted pteridine derivatives with various $R_1$, $R_2$, $R_3$ and/or $R_4$ substituents in the 2-, 4-, 6- and/or 7-positions of the pteridine ring. In step (a), a nitroso group is introduced on position 5 of the pyrimidine ring of a 2-$R_2$-substituted 4-oxo-6-aminopyrimidine by using sodium nitrite under aqueous acidic conditions. Reduction of the nitroso group in step (b) is achieved either catalytically ($Pt/H_2$) in the presence of a protic solvent, or chemically using sodium dithionite or ammonium sulfide in water. Then in a next step (c), condensing the resulting 2-$R_2$-substituted 4-oxo-5,6-diamino-pyrimidine with an $\alpha$-ketoaldoxime bearing a radical $R_3$, wherein $R_3$ may be *inter alia* $C_{1-7}$ alkyl, $C_{3-10}$ cycloalkyl, aryl or heteroaryl, in a protic solvent such as methanol under acidic conditions regioselectively yields a 4-oxopteridine

bearing a $R_2$ substituent in position 2 and a $R_3$ substituent in position 6 of the pteridine ring. Alternatively, a 2-$R_2$-substituted 4-oxo-7-$R_4$-substituted pteridine derivative can be obtained in step (d) by reacting the 2-$R_2$-substituted 4-oxo-5,6-diaminopyrimidine with a monosubstituted glyoxal bearing the group $R_4$, wherein $R_4$ may be *inter alia* $C_{1-7}$ alkyl, $C_{3-10}$ cycloalkyl, aryl or heteroaryl, under neutral or basic conditions. Alternatively, a 2-$R_2$-substituted-4-oxo-6,7-disubstituted pteridine derivative can be obtained in step (e) by reacting the 2-$R_2$-substituted 4-oxo-5,6-diamino-pyrimidine with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under neutral or basic conditions. Activation of the (tautomeric) hydroxyl substituent in position 4 of the pteridine ring for a nucleophilic displacement reaction occurs in step (f) by preparing the corresponding 4-[(1,2,4)-triazolyl] pteridine derivative, e.g. using $POCl_3$ or 4-chlorophenyl phosphorodichloridate and 1,2,4-triazole in pyridine as solvent. When $R_2$ is an amino group, protection of $R_2$ may further be necessary before carrying out this reaction. The amino group can be protected for instance by an acetyl group, which can be hydrolysed back to the amino group in a next step. Nucleophilic substitution is performed in step (g) by mixing the triazolyl pteridine derivative with a nucleophile $R_1XH$, (such as for example such as for example a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl) at room temperature in a polar aprotic solvent such as 1,4-dioxane.

Figure 6 (reference) represents a scheme for the preparation of symmetrical 2,4,6- trisubstituted pteridines and 2,4,7-trisubstituted as well as 2,4,6,7-tetrasubstituted pteridine derivatives, i.e. wherein substituents in the 2- and 4- positions of the pteridine ring are identical. In step (a), a nitro group is introduced in position 5 of a 6-amino-2,4-dioxopyrimidine under strongly acidic conditions (e.g. $HNO_3$, $H_2SO_4$). Then, in step (b), both hydroxyl groups from the tautomeric form are converted to chloro groups by treatment with a chlorinating agent such as $POCl_3$ or $SOCl_2$. Both chloro groups are then displaced in step (c) with a nucleophile having the general formula $R_1XH$. The nitro group of the latter is then reduced in step (d) to an amino group by treatment with a reducing agent (e.g. $Pt/H_2$). Finally, reaction of the latter with an $\alpha$-ketoaldoxime bearing the group $R_3$, wherein $R_3$ may be *inter alia* aryl, $C_{1-7}$ alkyl, $C_{1-10}$ cycloalkyl or heteroaryl, regioselectively yields the desired 2,4,6-trisubstituted pteridine derivative in step (e). Alternatively, reaction of the 2,4-substituted-5,6-diaminopyrimidine from step (d) with a monosubstituted glyoxal bearing a group $R_4$ wherein $R_4$ may be *inter alia* $C_{1-7}$ alkyl, $C_{3-10}$ cycloalkyl, aryl or heteroaryl yields the desired 2,4,7-trisubstituted pteridine derivative in step (f). Alternatively, reaction of the 2,4-substituted-5,6-diaminopyrimidine from step (d) with a disubstituted glyoxal bearing groups $R_3$ and $R_4$, wherein each of $R_3$ and $R_4$ is independently selected (i.e. $R_3$ and $R_4$ may be identical or different) from the group consisting of $C_{1-7}$ alkyl, $C_{3-10}$ cycloalkyl, aryl and heteroaryl, under neutral or basic conditions, yields the desired 2,4,6,7-tetrasubstituted pteridine derivative in step (g).

Figure 7 represents a scheme for the preparation of 2,4,6-trisubstituted pteridine derivatives, wherein the substituent in the 6- position of the pteridine ring is a phenyl group substituted by a nitrogen-containing function. In step (a), the chlorine at position 4 of the pyrimidine ring is displaced by an appropriate nucleophile, having the general formula $R_1XH$ (such as a primary or secondary amine, $C_{1-7}$ alkoxy, aryloxy, $C_{3-10}$ cycloalkoxy, heteroaryloxy, mercapto $C_{1-7}$ alkyl, mercaptoaryl, mercapto $C_{3-10}$ cycloalkyl, or mercapto-heteroaryl). Introduction of the nitroso group at position 5 of the pyrimidine ring is achieved by treatment with sodium nitrite under aqueous acidic conditions. Reduction of the nitroso group in step (c) is achieved either catalytically ($Pt/H_2$) in the presence of a protic solvent, or chemically using sodium dithionite or ammonium sulfide in water. In step (d), the 2-$R_2$-4-$XR_1$-substituted-5,6-triamino-pyrimidine analogue is condensed with an acetamidophenylglyoxalmonoxime in a protic solvent such as methanol under acidic conditions to yield regioselectively a pteridine analogue bearing an acetamidophenyl group at position 6 of the pteridine ring. Acidic deprotection of the acetyl group leads to the formation of the free amino group. This amino group can be transformed into amides (by reaction with carboxylic acids or acid chlorides) or into sulfonamides (by reaction with sulfonyl chlorides) by reaction in a polar aprotic solvent (e.g. pyridine, dimethylformamide or dichloromethane) and optionally further in the presence of a base (e.g. triethylamine).

Figure 8 represents a scheme for the preparation of 2,4,6-trisubstituted pteridine derivatives, wherein the substituent in the 6- position of the pteridine ring is a phenyl group substituted by an oxygen-containing function. In step (a) a 2-$R_2$-4-$XR_1$-substituted-5,6-triaminopyrimidine, which may be obtained for instance after step (c) described in figure 7, is condensed with an hydroxyphenyl-glyoxalmonoxime in a protic solvent such as methanol under acidic conditions to yield regioselectively a pteridine analogue bearing an hydroxyphenyl group at position 6 of the pteridine ring. The free hydroxyl group can be alkylated in a polar protic solvent (e.g. dimethylformamide) using a base (such as potassium carbonate, cesium carbonate or sodium hydride) and an appropriate alkylhalide or arylalkylhalide.

When applicable, and depending upon the specific substituents being present, the pteridine derivatives having the general formula (I) may be in the form of a pharmaceutically acceptable salt. The latter include any therapeutically active non-toxic addition salt which compounds having the general formula (I) are able to form with a salt-forming agent. Such addition salts may conveniently be obtained by treating the pteridine derivatives of the invention with an appropriate salt-forming acid or base. For instance, pteridine derivatives having basic properties may be converted into the corresponding therapeutically active, non-toxic acid addition salt form by treating the free base form with a suitable amount of an appropiate acid following conventional procedures. Examples of such appropriate salt-forming acids include, for

instance, inorganic acids resulting in forming salts such as hydrohalides (e.g. hydrochloride and hydrobromide), sulfate, nitrate, phosphate, diphosphate, carbonate, bicarbonate; and organic monocarboxylic or dicarboxylic acids resulting in forming salts such as, for example, acetate, propanoate, hydroxyacetate, 2-hydroxypropanoate, 2-oxopropanoate, lactate, pyruvate, oxalate, malonate, succinate, maleate, fumarate, malate, tartrate, citrate, methanesulfonate, ethanesulfonate, benzoate, 2-hydroxybenzoate, 4-amino-2-hydroxybenzoate, benzenesulfo-nate, p-toluenesulfonate, salicylate, p-aminosalicylate, pamoate, bitartrate, camphorsulfonate, edetate, 1,2-ethanedisulfonate, fumarate, glucoheptonate, gluconate, glutamate, hexylresorcinate, hydroxynaphtoate, hydroxyethane-sulfonate, mandelate, methylsulfate, pantothenate, stearate, as well as salts derived from ethanedioic, propanedioic, butanedioic, (Z)-2-butenedioic, (E)2-butenedioic, 2-hydroxybutanedioic, 2,3-dihydroxy-butanedioic, 2-hydroxy-1,2,3-propanetricarboxylic and cyclohexanesulfamic acids.

Pteridine derivatives having acidic properties may be converted in a similar manner into the corresponding therapeutically active, non-toxic base addition salt form. Examples of appropriate salt-forming bases include, for instance, inorganic bases like metallic hydroxides such as those of alkali and alkaline-earth metals like calcium, lithium, magnesium, potassium and sodium, or zinc, resulting in the corresponding metal salt; organic bases such as ammonia, alkylamines, benzathine, hydrabamine, arginine, lysine, N,N'-dibenzylethylenediamine, chloroprocaine, choline, diethanolamine, ethylene-diamine, N-methylglucamine, procaine.

Reaction conditions for treating the pteridine derivatives having the general formula (I) of this invention with an appropriate salt-forming acid or base are similar to standard conditions involving the same acid or base but different organic compounds with basic or acidic properties, respectively. Preferably, in view of its use in a pharmaceutical composition or in the manufacture of medicament for treating specific diseases, the pharmaceutically acceptable salt will be designed, i.e. the salt-forming acid or base will be selected so as to impart greater water-solubility, lower toxicity, greater stability and/or slower dissolution rate to the pteridine derivative of this invention.

The present invention provides the use of the above-described pteridine derivatives as biologically-active ingredients, i.e. active principles, for the manufacture of a medicament for the prevention or treatment of a TNF-α-related disorder as defined in claim 1 in a mammal. The class of such disorders include the following:

- alcohol-induced hepatitis;
- toxic effects of TNF-α, especially side effects associated with TNF generation during neoplastic therapy, for instance following use of cisplatin;
- cachexia and similar chronic wasting diseases, whether associated with cancer or with other chronic diseases such as malabsortive disorders, excessive physical stress, eating disorders and AIDS.

The medicament of this invention may be for prophylactic use, i.e. where circumstances are such that an elevation in the TNF level might be expected or alternatively, may be for use in reducing the TNF level after it has reached an undesirably high level or as the TNF level is rising.

The medicament according to this invention may be administered orally or in any other suitable fashion. Oral administration is preferred and the preparation may have the form of a tablet, aqueous dispersion, dispersable powder or granule, emulsion, hard or soft capsule, syrup, elixir or gel. The dosing forms may be prepared using any method known in the art for manufacturing these pharmaceutical compositions and may comprise as additives sweeteners, flavoring agents, coloring agents, preservatives and the like. Carrier materials and excipients are detailed hereinbelow and may include, *inter alia,* calcium carbonate, sodium carbonate, lactose, calcium phosphate or sodium phosphate; granulating and disintegrating agents, binding agents and the like. The pharmaceutical composition or combined preparation of this invention may be included in a gelatin capsule mixed with any inert solid diluent or carrier material, or has the form of a soft gelatin capsule, in which the ingredient is mixed with a water or oil medium. Aqueous dispersions may comprise the biologically active composition or combined preparation in combination with a suspending agent, dispersing agent or wetting agent. Oil dispersions may comprise suspending agents such as a vegetable oil. Rectal administration is also applicable, for instance in the form of suppositories or gels. Injection (e.g. intramuscularly or intraperiteneously) is also applicable as a mode of administration, for instance in the form of injectable solutions or dispersions, depending upon the disorder to be treated and the condition of the patient.

Usually the medicament of the invention is in the form of a combination of the pteridine derivative active principle and one or more pharmaceutically acceptable carriers or excipients.

The term " pharmaceutically acceptable carrier or excipient " as used herein refers to any material or substance with which the active principle, i.e. the pteridine derivative having the general formula (I) may be formulated in order to facilitate its application or dissemination to the locus to be treated, for instance by dissolving, dispersing or diffusing the said composition, and/or to facilitate its storage, transport or handling without impairing its effectiveness. The pharmaceutically acceptable carrier may be a solid or a liquid or a gas which has been compressed to form a liquid, i.e. the

compositions of this invention can suitably be used as concentrates, emulsions, solutions, granulates, dusts, sprays, aerosols, pellets or powders.

Suitable pharmaceutical carriers for use in the said pharmaceutical compositions and their formulation are well known to those skilled in the art. There is no particular restriction to their selection within the present invention although, due to the usually low or very low water-solubility of the pteridine derivatives of this invention, special attention will be paid to the selection of suitable carrier combinations that can assist in properly formulating them in view of the expected time release profile. Suitable pharmaceutical carriers include additives such as wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, dissolving, spray-drying, coating and/or grinding the active ingredients, in a one-step or a multi-steps procedure, with the selected carrier material and, where appropriate, the other additives such as surface-active agents. may also be prepared by micronisation, for instance in view to obtain them in the form of microspheres usually having a diameter of about 1 to 10 μm, namely for the manufacture of microcapsules for controlled or sustained release of the biologically active ingredient(s).

Suitable surface-active agents to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids ($C_{10}$-$C_{22}$), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, Unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanyl-phosphatidylcholine, dipalmitoylphoshatidylcholine and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenol-polyethoxyethanol, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo, phenyl, substituted phenyl or hydroxy; for instance quaternary ammonium salts containing as N-substituent at least one $C_8$-$C_{22}$ alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbuch", 2nd ed. (Hanser Verlag, Vienna, 1981) and "Encyclopaedia of Surfactants (Chemical Publishing Co., New York, 1981).

Structure-forming, thickening or gel-forming agents may be included into the pharmaceutical compositions of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon

atoms; cetostearyl alcohol and modified castor oil products (e.g. the product commercially available under the trade name Antisettle).

Gelling agents which may be included into the pharmaceutical compositions of the present invention include cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof. Gelatin and modified celluloses represent a preferred class of gelling agents.

Other optional excipients which may be included in the pharmaceutical compositions of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethyl-cellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins and the like, and mixtures thereof.

The effective amount of a pteridine derivative having the general formula (I) as well as pharmaceutically acceptable addition salts, stereoisomers, mono- or di-*N*-oxides, solvates and/or dihydro- or tetrahydropteridine derivatives thereof for treating or preventing a TNF-$\alpha$ related disease as defined above is usually in the range of 0.01 mg to 20 mg, preferably 0.1 mg to 5 mg, per day per kg bodyweight for humans. Depending upon the pathologic condition to be treated and the patient's condition, the said effective amount may be divided into several sub-units per day or may be administered at more than one day intervals. The patient to be treated may be any warm-blooded animal such as a mammal, preferably a human being, suffering from said TNF-$\alpha$-related disease. Within the framework of this invention, any compound of formula (I), such as extensively described in details hereinabove, or any mixture of such compounds may be administered for the said prevention or treatment.

The following examples are intended to illustrate several embodiments of the present invention, as well as the preparation of the pteridine derivatives.

Example 1- preparation of 2-amino-4-n-pentyloxy-6-styrylpteridine.

A mixture of 1.5 g (5.6 mmoles) 2-amino-6-chloro-4-n-pentyloxypteridine (e.g. available following the procedure disclosed by Mohr et al. in Helv. Chem. Acta (1992) 75:2317), palladium acetate (63 mg, 0.28 mmoles), tri-o-tolylphosphane (682 mg, 2.24 mmoles), cuprous iodide (53 mg, 0.28 mmoles), styrene (1,3 ml., 11.3 mmoles) and triethylamine 3.1 ml, 22 mmoles) was stirred in dry acetonitrile (50 ml) under reflux for 90 hours. It was evaporated and the residue purified by silica gel column chromatography with chloroform. The product fraction was evaporated to give 1.37 g (yield: 72%) of an orange powder exhibiting, after recrystallization from a EtOAc/hexane mixture, a melting point (m.p.) range of 127 -128 ˚C.

Example 2 - preparation of 2-amino-6-(1,2-dibromophenethyl)-4-n-pentyloxy-pteridine.

To a solution of the derivative of example 1 (1.0 g, 2.94 mmoles) in chloroform (50 ml) was added a 2 M bromine solution in chloroform (2.2 ml., 4.4 mmoles) and then the mixture was stirred at room temperature for 7 hours. It was diluted with chloroform (50 ml), washed with a saturated aqueous $Na_2SO_3$ solution (100 ml) and dried over sodium sulfate. After evaporation of the solvents, the residue was treated with toluene, filtered, washed with ether and dried in a vacuum desiccator to give 0.84 g (yield: 57%) of a yellow powder.

Example 3 - preparation of 2-amino-4,7-dimethoxy-6-stryrylpteridine.

A suspension of the derivative of example 2 (0.3 g, 0.6 mmoles) is methanol (10 ml) was treated with 1 M methanolic sodium methoxide (3 ml, 3 mmoles) and then refluxed for 4 hours. It was diluted with chloroform (100 ml), washed with a saturated aqueous ammonium chloride solution and water and then the solution was dried over sodium sulfate. The filtrate was evaporated and the residue was purified by silica gel column chromatography while using chloroform as the eluent. The product fraction was evaporated to give 50 mg (yield: 26%) of a yellow powder with a melting point range of 197-198 ˚C.

Example 4 - preparation of $0^4$-methyl-biopterin (2-amino-4-methoxy-6-(1,2-dihydroxypropyl) pteridine)

To a solution of $N^2$, 1', 2'-O-triacetyl-biopterin (1,0 g, 2.75 mmoles), triphenyl-phosphane (12,08 g, 4.13 mmoles) and methanol (0.15 ml, 3.7 mmoles) in dry dioxane (30 ml) was added diisopropyl azodicarboxylate (0.81 g, 4.1 mmoles). After stirring for 1.5 hour at room temperature, the mixture was evaporated to dryness. The residue was purified by silica gel column chromatography while using an ethylacetate/$CHCl_3$ (1:4) mixture as the eluent. The product fraction was evaporated and dried in vacuum to give 0.4 g (yield: 38%) of $N^2$,1',2'-0-triacetyl-$0^4$-methylbiopterin. Deacetylation of this reaction product (0.28 g, 0.74 mmole) was done by stirring it in absolute methanol (20 ml) and triethylamine (4 ml) for 24 hours. Evaporation to dryness, treatment of the residue with ether, filtration and drying gave 0.172 g (yield: 83%) of $0^4$-methyl-biopterin with a melting point range of 160-161 ˚C.

Example 5 - preparation of 2-amino-4-hydroxylamino-6-phenylpteridine.

A suspension of 2,5,6-triamino-4-methoxypyrimidine dihydrochloride (1 g, 4 mmoles) in methanol (40 ml) was heated to boiling and then a solution of phenylglyoxalmonoxime (1 g, 6.6 mmoles) in methanol (10 ml) was added dropwise. A clear solution is obtained from which on reflux for 2 hours a precipitate was separated out. The solid (hydrochloride salt) was filtered off, suspended in water (30 ml) and then neutralized to pH 8 by concentrated ammonia. The resulting precipitate was collected, washed with water and ethanol and dried at 100 ˚C to give 0.84 g of a yellow powder (yield: 82%).

Examples 6 to 53 - synthesis of 2-amino-4-dialkylamino-6-arylpteridines, 2-amino-4-di(arylalkyl)amino-6-arylpteridines, 2-amino-4-alkylamino-6-arylpteri-dines, 2-amino-4-(N-containing heterocyclic amino)-6-arylpteridines and 2-amino-4-alkoxy-6-arylpteridines

The procedure for the synthesis of the following 2-amino-4-dialkylamino-6-arylpteridines, 2-amino-4-dialkylamino-6-arylpteridines, 2-amino-4-alkylamino-6-arylpteridines, 2-amino-4-(N-containing heterocyclic amino)-6-arylpteridines and 2-amino-4-alkoxy-6-arylpteridines proceeds in three steps:

a) a solution of 2,6-diamino-4-chloro-5-p-chlorophenylazopyrimidine (a compound known from British Patent No. 677,342) (5.0 g, 16.6 mmoles) in DMF (50ml) and 0.12 mole of the appropriate reactant, being selected from the group consisting of secondary alkylamines and arylalkylamines (e.g. dimethyl-amine in ethanol (50%), diethylamine, di-n-propylamine or dibenzylamine), primary amines (e.g. an adamantanamine), heterocyclic amines (e.g. morpholine, piperidine, pyrrolidine, piperazine or N-methyl-piperazine) and alcaline metal alkoxides (e.g. sodium ethoxide or sodium isopropoxide), were heated in an oil bath at 70˚C for 5 hours. Then water (50 ml) was added, cooled and the yellow precipitate collected, washed with water and dried. Recrystallization from ethanol or a DMF/water mixture provided the relevant 2,6-diamino-4-dialkylamino-5-p-chloro-phenylazopyrimidine, 2,6-diamino-4-di(arylalkyl)amino-5-p-chlorophenyl-azopyrimidine, 2,6-diamino-4-alkylamino-5-p-chlorophenylazopyrimidine, 2,6-diamino-4-(N-containing hetero-cyclic amino)-5-p-chlorophenylazo-pyrimidine or 2,6-diamino-4-alkoxy-5-p-chlorophenylazopyrimidine with a yield ranging from 55 to 90%.

b) a suspension of the pyrimidine compound (3.28 g, 10 mmoles) resulting from step (a) in methanol (70 ml) and concentrated ammonia (10 ml) was reduced in a shaking apparatus under a hydrogen atmosphere in the presence of a Raney nickel catalyst (3.5 g) for 2 days. The catalyst was filtered off under argon atmosphere and then the filtrate evaporated in vacuo to dryness. The residue was treated with ether to remove p-chloroaniline, filtered and then the solid stirred in methanolic HCl (10%, 50 ml) overnight. The dihydrochloride salt (obtained with a yield ranging from 85 to 90%) of the relevant 2,5,6-triamino-4-dialkylaminopyrimidine, 2,5,6-triamino-4-alkoxypyrimidine, 2,5,6-triamino-4-di(arylalkyl)aminopyrimidine, 2,5,6-triamino-4-alkylaminopyrimidine or 2,5,6-triamino-4-(N-containing heterocyclic amino) pyrimidine, was collected and dried in a vacuum desiccator over KOH.

c) to a boiling solution of the 2,5,6-triamino-4-substituted pyrimidine dihydrochloride salt (5 mmoles) from step (b) in methanol (20 ml) was added a solution of the relevant arylglyoxalmonoxime (7.5 mmoles) in methanol (10 ml)

dropwise and then the mixture was heated under reflux for 3 hours. After cooling, the suspension or solution was made alkaline by means of concentrated ammonia up to pH 9 and the resulting precipitate was filtered off, washed with water and dried. Recrystallization was done from ethanol and a DMF/water mixture, respectively, such as to provide a yellow solid with a yield ranging from 50 to 85%.

The following compounds were prepared according to the above general procedure:

2-amino-4-dimethylamino-6-phenylpteridine (example 6);
2-amino-4-dimethylamino-6-(4-tolyl) pteridine (example 7);
2-amino-4-dimethylamino-6-(4-methoxyphenyl)pteridine (example 8);
2-amino-4-diethylamino-6-phenylpteridine (example 9);
2-amino-4-diethylamino-6-(4-chlorophenyl)pteridine (example 10);
2-amino-4-diethylamino-6-(4-methoxyphenyl)pteridine (example 11);
2-amino-4-diethylamino-6-(3,4-dimethoxyphenyl)pteridine (example 12);
2-amino-4-dibenzylamino-6-phenylpteridine (example 13);
2-amino-4-dibenzylamino-6-(4-chlorophenyl)pteridine (example 14);
2-amino-4-dibenzylamino-6-(4-methoxyphenyl)pteridine (example 15);
2-amino-4-dibenzylamino-6-(3,4-dimethoxyphenyl)pteridine (example 16);
2-amino-4-dipropylamino-6-phenylpteridine(example 17);
2-amino-4-dipropylamino-6-(4-chlorophenyl)pteridine (example 18);
2-amino-4-dipropylamino-6-(4-methoxyphenyl)pteridine (example 19);
2-amino-4-dipropylamino-6-(3,4-dimethoxyphenyl)pteridine (example 20);
2-amino-4-morpholino-6-phenylpteridine (example 21);
2-amino-4-morpholino-6-(4-chlorophenyl)pteridine (example 22);
2-amino-4-morpholino-6-(4-methoxyphenyl)pteridine (example 23);
2-amino-4-morpholino-6-(3,4-dimethoxyphenyl)pteridine (example 24);
2-amino-4-piperidino-6-phenylpteridine (example 25);
2-amino-4-piperidino-6-(4-chlorophenyl)pteridine (example 26);
2-amino-4-piperidino-6-(4-methoxyphenyl)pteridine (example 27);
2-amino-4-piperidino-6-(3,4-dimethoxyphenyl)pteridine (example 28);
2-amino-4-N-methylpiperazino-6-phenylpteridine (example 29);
2-amino-4-N-methylpiperazino-6-(4-chlorophenyl)pteridine (example 30);
2-amino-4-N-methylpiperazino-6- (4-methoxyphenyl) pteridine (example 31);
2-amino-4-methylpiperazino-6- (3, 4-dimethoxyphenyl) pteridine (example 32);
2-amino-4-pyrrolidino-6- (4-methoxyphenyl) pteridine (example 33);
2-amino-4-piperazino-6-phenylpteridine (example 34);
2-amino-4-piperazino-6- (4-chlorophenyl) pteridine (example 35);
2-amino-4-piperazino-6- (4-methoxyphenyl) pteridine (example 36);
2-amino-4-piperazino-6- (3, 4-dimethoxyphenyl) pteridine (example 37);
2-amino-4-dibenzylamino-6-(3, 4,5-trimethoxyphenyl) pteridine (example 38);
2-amino-4-morpholino-6- (3, 4, 5-trimethoxyphenyl) pteridine (example 39);
2-amino-4-(3-adamantylamino)-6-(3,4,5-trimethoxyphenyl) pteridine (example 40);
2-amino-4-(3-adamantylamino)-6-naphtylpteridine (example 41);
2-amino-4-(4-adamantylamino)-6-(3,4,5-trimethoxyphenyl) pteridine (example 42);
2-amino-4-(4-adamantylamino)-6-naphtylpteridine (example 43);
2-amino-4-morpholino-6-(3,4-formylidene-3,4-dihydroxyphenyl)pteridine (example 44);
2-amino-4-dimethylamino-6-(3,4-formylidene-3,4-dihydroxyphenyl)pteridine (example 45);
2-amino-4-pyrrolidino-6-(3, 4-dimethoxyphenyl) pteridine (example 46);
2-amino-4-dimethylamino-6-(3, 4-dimethoxyphenyl) pteridine (example 47);
2-amino-4-dimethylamino-6-methylpteridine (example 48);
2-amino-4-ethoxy-6-phenylpteridine (example 49);
2-amino-4-propylamino-6-phenylpteridine (example 50);
2-amino-4-propylamino-6-(3, 4-dimethoxyphenyl) pteridine (example 51);
2-acetamido-4-isopropoxy-6-(3,4-dimethoxyphenyl) pteridine (example 52); and
2-amino-4-ethoxy-6-(3,4-dimethoxyphenyl)pteridine (example 53);

Example 54 - synthesis of 2,6-diamino-4-ethoxy-pyrimidine

To a solution of sodium (1.05 g) in ethanol (50 ml) was added 4-chloro-2,6-diaminopyrimidine (6 g, 41.4 mmoles). The resulting solution was heated in a reactor for 6 hours at 160 ˚C. The reaction mixture was cooled down and the precipitated sodium chloride was filtered off. The filtrate was concentrated and precipitated from ethanol (two times), affording the pure title compound as a white solid (4.53 g, 72% yield). The spectral data are identical to those described e.g. by W. Pfleiderer et al. in Chem. Ber. (1961) 94, 12.

Example 55 - synthesis of 2,6-diamino-4-isopropoxy-pyrimidine

The same procedure as in example 54 was followed using isopropanol instead of ethanol. The filtrate was pure enough for further reaction without purification. The spectral data are identical to those described e.g. by W. Pfleiderer et al. in Chem. Ber. (1961) 94, 12.

Example 56 - synthesis of 5-nitroso-2,6-diamino-4-ethoxy-pyrimidine

To a solution of the compound of example 54 (6.13 g, 39.8 mmoles) in 20 % aqueous acetic acid (57 ml) was added dropwise a solution of $NaNO_2$ (3.29 g) in water (13 ml) at 80 ˚C. A pink precipitate was formed which was stirred at 80˚C for an additional 2 hours. The reaction mixture was cooled down in the refrigerator overnight and the resulting precipitate was filtered off, yielding the title compound as a pink powder (4.98 g, yield 68 %). Spectral data are identical with those described e.g. by W. Pfleiderer et al. in Chem. Ber. (1961) 94, 12.

Example 57 - synthesis of 5-nitroso-2,6-diamino-4-isopropoxy-pyrimidine

The same procedure was followed as in example 56 but starting from the compound of example 55. The product has identical spectral data to those described by W. Pfleiderer et al. (cited *supra).*

Example 58 - synthesis of 2,5,6-triamino-4-ethoxy-pyrimidine

To a suspension of the compound of example 56 (7.12 g, 38.9 mmoles) in water (150 ml) at 60 ˚C was added sodium dithionite (46.7 mmol, 8.12 g). Additional sodium dithionite was added till the pink colour completely disappeared and a yellow solution was formed. The solution was stirred at 60 ˚C for another 4 hours. Water was evaporated and the resulting residue was precipitated from a small amount of water, providing the title compound as a yellow powder (4.02 g, yield 61 %). Spectral data are identical with literature data (W. Pfleiderer et al. cited *supra).*

Example 59 - synthesis of 2,5,6-triamino-4-isopropoxy-pyrimidine

The procedure of example 58 was followed, however using the compound of example 57 as the starting material. The spectral data of the product obtained are identical with the literature data (W. Pfleiderer et al. cited *supra).*

Example 60 - synthesis of 2-amino-4-ethoxy-pteridin

To a solution of 2,5,6-triamino-4-ethoxy-pyrimidine (10.54 g, 62.37 mmoles) in ethanol (160 ml) was added glyoxal (40 % solution in water, 2.7 ml, 18.6 mmoles). The reaction mixture was refluxed for 4 hours. Some insoluble material was filtered off. The filtrate was concentrated *in vacuo* and the residue purified by flash chromatography (silica, using a $CH_3OH/CH_2Cl_2$ mixture (5:95) as the eluent), providing the pure title compound (7.34 g, yield: 62 %). The spectral data of the product are identical with the literature data (W. Pfleiderer et al. cited *supra).*

Example 61 - synthesis of 2-amino-4-isopropoxy-pteridin

The procedure of example 60 was repeated, however using isopropanol as the solvent instead of ethanol. The spectral data of the product obtained are identical with the literature data (W. Pfleiderer et al. cited *supra).*

Example 62 - synthesis of 2-amino-4-ethoxypteridine-$N^8$-oxide

To a cooled (0 ˚C) solution of the compound of example 60 (2.47 g, 12.9 mmoles) in trifluoroacetic acid (53 ml) was added dropwise 2.53 ml of a 35 % aqueous $H_2O_2$ solution. The reaction mixture was kept at 4 ˚C for two days in the refrigerator, whereby another 1.25 ml of the same $H_2O_2$ solution was added after 1 day. The solution was concentrated *in vacuo.* The residue was suspended in water and neutralized by the addition of a concentrated ammonia solution. Evaporation of the solvent *in vacuo* and purification of the residue by flash chromatography (silica, using a $CH_3OH/CH_2Cl_2$ mixture (6:94) as the eluent) provided the title compound as a yellow powder (861 mg, yield: 32 %). Mass spectrum data are as follows: m/z (%): 230 ($[M+Na]^+$, 30), 208 ($[M+H]^+$, 100), 180 [$(M+H\text{-ethene})^+$, 10].

Example 63 - synthesis of 2-amino-4-isopropoxypteridine-$N^8$-oxide

The procedure as described in example 62 was followed, however using the compound of example 61 as the starting material. Mass spectrum data are as follows: *m/z* (%): 222 ($[M+H]^+$, 100), 180 ($[M+H\text{-propene}]^+$, 60)

Example 64 - synthesis of 2-amino-6-chloro-4-ethoxypteridine

A suspension of the compound of example 62 (460 mg, 2.22 mmoles) in acetyl chloride (5.5 ml) was stirred at -40 ˚C. Trifluoroacetic acid (1.69 ml) was then added dropwise. The resulting solution was slowly warmed up to 0 ˚C and stirred for an additional 4 hours at 0 ˚C. Reaction was carefully quenched with ice, followed by neutralization with a concentrated ammonia solution (pH = 8). The aqueous phase was extracted with $CH_2Cl_2$ (five times). The combined organic layers were concentrated *in vacuo* and the residue was purified by flash chromatography (silica, using a $CH_3OH/CH_2Cl_2$ mixture (1:99) as the eluent), thus providing the title compound as a yellow powder (360 mg, yield: 72 %). This compound was further characterized as follows:

- mass spectrum: *m/z* (%): 226 ($[M+H]^+$ 100),
- $^1$H-NMR (200 MHz, DMSO-$d_6$): δ 1.42 (3 H, t), 4.52 (2 H, q), 7.42 (2 H, d) and 8.85 (1 H, s) ppm,
- $^{13}$C-NMR (50 MHz, DMSO-$d_6$): δ 14.19, 63.58, 121.74, 140.22, 150.99, 156.13, 161.98 and 165.97 ppm.

Example 65 - synthesis of 2-amino-6-chloro-4-isopropoxypteridine

The procedure as described in example 64 was followed, however starting from the compound of example 63. The mass spectrum data of the resulting compound are as follows: *m/z* (%): 240 ($[M+H]^+$, 55), 198 ($[M+H\text{-propene}]^+$, 100).

Examples 66 to 83 - synthesis of 2-amino-6-aryl-4-ethoxypteridines and 2-amino-6-heteroaryl-4-ethoxypteridines

The general procedure used for preparing 2-amino-6-aryl-4-ethoxy-pteridines is as follows: to a degassed solution of the compound of example 64 (50 mg, 0.22 mmole) in THF (5 ml) was added a degassed solution of sodium carbonate (5 ml of a 0.4 M solution in water), tetrakis(triphenyl-phosphine) palladium (0.013 mmole, 14 mg) and an arylboronic or (examples 72 and 73) heteroarylboronic acid (0.22 mmole). The solution was refluxed for 4 hours. Solvents were concentrated *in vacuo* and the residue was purified by flash chromatography (silica) with an appropriate $CH_3OH/CH_2Cl_2$ mixture (2:98 or 3:97) as the eluent (except for the compound of example 82, which was eluted with an acetone/$CH_2Cl_2$ (7:3) mixture). This procedure provided, with a yield ranging from 16 % to 60 % depending upon the aryl or heteroaryl group (from the arylboronic or heteroarylboronic acid) introduced at the 6-position of the pteridine ring, the following pure final compounds which were characterized by their mass spectrum MS and optionally by their $^1$H-NMR (200 MHz, DMSO-$d_6$) spectrum:

- 2-amino-6-(p-methoxyphenyl)-4-ethoxy-pteridine (example 66): MS 298 ($[M+H]^+$, 100), 270 ($[M+H\text{-ethene}]^+$, 55);
- 2-amino-6-(o-methoxyphenyl)-4-ethoxy-pteridine (example 67): MS 298 ($[M+H]^+$, 100), 270 ($[M+H\text{-ethene}]^+$, 30);
- 2-amino-6-(m-methoxyphenyl)-4-ethoxy-pteridine (example 68): MS 298 ($[M+H]^+$, 100), 270 ($[M+H\text{-ethene}]^+$, 35); $^1$H-NMR: 1.46 (3 H, t), 3.85 (3 H, s), 4.58 (2 H, q), 7.06 (1 H, dd), 7.33 (2 H, br s), 7.46 (1 H, t), 7.68 (1 H, m) and 9.43 (1 H, s) ppm;
- 2-amino-6-(3,4-difluorophenyl)-4-ethoxy-pteridine (example 69): MS 304 ($[M+H]^+$,100), 270 ($[M+H\text{-ethene}]^+$, 35); $^1$H-NMR: 1.45 (3 H, t), 4.57 (2 H, q), 7.42 (2 H, br s), 7.60 (1 H, q), 7.98 (1 H, d), 8.16 (1 H, t) and 9.42 (1 H, s) ppm;
- 2-amino-6-(p-dimethylaminophenyl)-4-ethoxy-pteridine (example 70): MS 311 ($[M+H]^+$,100), 283 ($[M+H\text{-ethene}]^+$, 35);
- 2-amino-6-(p-trifluoromethylphenyl)-4-ethoxy-pteridine (example 71): MS 336 ($[M+H]^+$,100), 308 ($[M+H\text{-ethene}]^+$, 50);
- 2-amino-6-(2-thienyl)-4-ethoxy-pteridine (example 72): MS 274 ($[M+H]^+$, 100), 246 ($[M+H\text{-ethene}]^+$, 40);
- 2-amino-6-(3-thienyl)-4-ethoxy-pteridine (example 73): MS 274 ($[M+H]^+$, 100), 246 ($[M+H\text{-ethene}]^+$, 45);
- 2-amino-6-(3,4-dichlorophenyl)-4-ethoxy-pteridine (example 74): MS 337 ($[M+H]^+$,100); $^1$H-NMR: 1.46 (3 H, t), 4.59 (2 H, q), 7.42 (2 H, br s), 7.81 (1H, d), 8.14 (1 H, dd), 8.37 (1 H, d) and 9.47 (1 H, s) ppm;

- 2-amino-6-(p-cyanophenyl)-4-ethoxy-pteridine (example 75): MS 293 ([M+H]$^+$,100), 265 ([M+H-ethene]$^+$, 65);
- 2-amino-6-(p-ethoxyphenyl)-4-ethoxy-pteridine (example 76): MS 312 ([M+H]$^+$, 100), 284 ([M+H-ethene]$^+$, 70);
- 2-amino-6-(p-fluorophenyl)-4-ethoxy-pteridine (example 77): MS 286 ([M+H]$^+$, 100), 258 ([M+H-ethene)$^+$, 45);
- 2-amino-6-(p-ethylphenyl)-4-ethoxy-pteridine (example 78): MS 296 ([M+H]$^+$, 100), 268 ([M+H-ethene)$^+$, 45);
- 2-amino-6-(p-acetylphenyl)-4-ethoxy-pteridine (example 79): MS 310 ([M+H]$^+$, 100), 282 ([M+H-ethene]$^+$, 60);
- 2-amino-6-(3-methyl-4-fluorophenyl)-4-ethoxy-pteridine (example 80): MS 300 ([M+H]$^+$, 100), 272 ([M+H-ethene]$^+$, 30);
- 2-amino-6-(p-thiomethylphenyl)-4-ethoxy-pteridine (example 81): MS 314 ([M+H]$^+$, 100), 286 ([M+H-ethene]$^+$, 35);
- 2-amino-6-(p-N,N-dimethylbenzamido)-4-ethoxy-pteridine (example 82) MS 338 ([M+H]$^+$, 100), 311 ([M+H-ethene]$^+$, 15); and
- 2-amino-6-(3,4-dimethoxyphenyl)-4-ethoxy-pteridine (example 83): MS 328 ([M+H]$^+$, 100), 300 ([M+H-ethene]$^+$, 40).

Examples 84 to 98 - synthesis of 2-amino-6-aryl-4-isopropoxypteridines and 2-amino-6-heteroaryl-4-isopropoxypteridines

The procedure as described in examples 66-83 was followed while using 2-amino-6-chloro-4-isopropoxypteridine as the starting material, except that longer reaction times were needed (refluxing overnight instead of 4 hours). This procedure provided, with a yield ranging from 10 % to 70 % depending upon the aryl or heteroaryl group introduced at the 6-position of the pteridine ring, the following pure final compounds which were characterized by their mass spectrum:

- 2-amino-6-(3-methyl-4-methoxyphenyl)-4-isopropoxypteridine (example 84): MS 326 ([M+H]$^+$, 100), 284 ([M+H-propene]$^+$, 30);
- 2-amino-6-(3,4-dimethylphenyl)-4-isopropoxypteridine (example 85): MS 310 ([M+H]$^+$, 100), 268 ([M+H-propene]$^+$, 60);
- 2-amino-6-(3-chloro-4-trifluoromethylphenyl)-4-isopropoxypteridine (example 86): MS 384 ([M+H]$^+$, 20), 342 ([M+H-propene]$^+$, 50);
- 2-amino-6-(3-chloro-4-fluorophenyl)-4-isopropoxypteridine (example 87): MS 334 ([M+H]$^+$, 20), 292 ([M+H-propene]$^+$, 50);
- 2-amino-6-(p-N,N-diethylbenzamido)-4-isopropoxypteridine (example 88): MS 381 ([M+H]$^+$, 100);
- 2-amino-6-(p-trifluoromethylphenyl)-4-isopropoxypteridine (example 89): MS 350 ([M+H]$^+$, 100), 308 ([M+H-propene]$^+$, 30);
- 2-amino-6-(3,4-difluorophenyl)-4-isopropoxypteridine (example 90): MS 318 ([M+H]$^+$, 100), 276 ([M+H-propene]$^+$, 50);
- 2-amino-6-(p-methoxyphenyl)-4-isopropoxypteridine (example 91): MS 312 ([M+H]$^+$, 100), 270 ([M+H-propene]$^+$, 50);
- 2-amino-6-(p-ethoxyphenyl)-4-isopropoxypteridine (example 92): MS 326 ([M+H]$^+$, 55), 284 ([M+H-propene]$^+$, 100);
- 2-amino-6-(p-dimethylbenzamido)-4-isopropoxypteridine (example 93): MS 353 ([M+H]$^+$, 75), 311 ([M+H-propene]$^+$, 100);
- 2-amino-6-(3-thienyl)-4-isopropoxypteridine (example 94): MS 288 ([M+H]$^+$, 55), 246 ([M+H-propene]$^+$, 100);
- 2-amino-6-(p-cyanophenyl)-4-isopropoxypteridine (example 95): MS 307 ([M+H]$^+$, 40), 265 ([M+H-propene]$^+$, 100);
- 2-amino-6-(p-benzoic acid methyl ester)-4-isopropoxypteridine (exam-pie 96): MS 340 ([M+H]$^+$, 75), 298 ([M+H-propene]$^+$, 100);
- 2-amino-6-(p-acetylphenyl)-4-isopropoxypteridine (example 97): MS 324 ([M+H]$^+$, 55), 282 ([M+H-propene]$^+$, 100); and
- 2-amino-6-(3,4-dimethoxyphenyl)-4-isopropoxypteridine (example 98): MS 342 ([M+H]$^+$, 100), 300 ([M+H-propene]$^+$, 60).

Example 99 - synthesis of 2,6-diamino-5-nitroso-4-hydroxypyrimidine

To a solution of 2,6-diamino-4-hydroxypyrimidine (12.9 g, 102.2 mmoles) in 200 ml of a 10% acetic acid solution in water at 80 ˚C was added dropwise a solution of $NaNO_2$ (7.05 g, 102.2 mmoles) in 20 ml water. A pink precipitate was formed, which was further stirred for 1 hour at 80 ˚C. The reaction mixture was cooled down in the refrigerator overnight. The precipitate was filtered off and dried over $P_2O_5$, providing the title compound as a pink powder (15.43 g, yield: 97%). The spectral data are in accordance with literature data (Landauer et al. in J. Chem. Soc. (1953) 3721-3722).

Example 100 - synthesis of 2,5,6-triamino-4-hydroxypyrimidine

A suspension of the compound of example 99 (15 g, 96.7 mmoles) in an ammonium sulfide solution (20 % in

water, 200 ml) was stirred overnight at 50 °C. The reaction mixture was cooled down in the refrigerator and the precipitate was filtered off, providing the title compound as a yellow powder (11.33 g, yield: 83 %). The spectral data are identical with literature data (Landauer et al. cited *supra).*

Example 101 - synthesis of 2-amino-6-(3 4-dimethoxyphenyl)pterine

To a boiling solution of the compound of example 100 (2.4 g, 17 mmoles) in methanol (100 ml, with 0.9 N HCl) was added dropwise a solution of 3,4-dimethoxyphenylglyoxal mono-oxime (3.8 g, 18 mmoles) in methanol (100 ml). The reaction mixture was heated under reflux for 4 hours. The precipitate formed was filtered off, washed with water, then ethanol and diethyl ether, and dried over $P_2O_5$ under vacuum, providing the title compound as a yellow powder (4.33 g, yield: 85 %). This compound was further characterized by the following spectra:

- $^1$H-NMR (500 MHz, TFA): δ 4.11 (3 H, s), 4.07 (3 H, s), 7.21 (1 H, d), 7.78 (1 H, dd), 7.81 (1 H, d) and 9.32 (1 H, s) ppm;
- $^{13}$C-NMR (125 MHz, TFA): δ 56.39, 56.7, 111.94, 113.21, 123.22, 127.41, 127.91, 145.92, 149.39, 150.46, 152.47, 153.15, 155.13 and 161.59 ppm.

Example 102 - synthesis of 2-acetylamino-6-(3,4-dimethoxyphenyl)pterine

A suspension of the compound of example 101 (10.46 g, 35 mmoles) in acetic anhydride (600 ml) and acetic acid (200 ml) was refluxed for 1 hour until a clear solution was formed. By cooling down the reaction mixture in the refrigerator, the precipitate formed was filtered off, washed with ethyl acetate and diethyl ether, and then dried over $P_2O_5$ under vacuum, providing the title compound as a yellow powder (9.19 g, yield: 77 %). This compound was further characterized by the following spectra:

- MS: *m*/*z* (%): 300 ([M+H]$^+$, 100);
- $^1$H-NMR (200 MHz, DMSO-$d_6$): δ 2.22 (3 H, s), 3.84 (3 H, s), 3.87 (3 H, s), 7.14 (1 H, d), 7.75 (2 H, m) and 9.51 (1 H, s) ppm.

Example 103 - synthesis of 2-acetylamino-4-(1,2,4-triazolyl)-6-(3,4-dimethoxy-phenyl)pteridine

To a solution of phosphorus oxychloride (1.68 ml, 18 mmoles) and 1,2,4-triazole (4.96 g, 72 mmoles) in dry pyridine (110 ml) was added the compound of example 102 (2.45 g, 7.18 mmoles). The suspension was stirred at room temperature for 4 hours. The precipitate was filtered off, washed with pyridine, toluene and diethyl ether. The resulting solid was dried over $P_2O_5$ under vacuum, providing the title compound as a yellow powder (2 g, yield: 80 %) which afforded the following mass spectrum: 392 ([M+H]$^+$; 100).

Examples 104 and 105 - synthesis of 2-amino-4-mercaptoethyl-6-(3,4-dimethoxyphenyl) pteridine and 2-amino-4-mercaptoisopropyl-6-(3,4-dimethoxyphenyl) pteridine

To a suspension of the compound of example 103 (0.25 mmole, 100 mg) in dioxane (5 ml) was added 1 mmole of either ethanethiol (example 104) or isopropanethiol (example 105) and sodium (12 mg, 0.5 mmole). The suspension was stirred for 24 hours at room temperature. The solvent was concentrated *in vacuo* and the residue purified by flash chromatography (silica, using a $CH_3OH/CH_2Cl_2$ mixture (5:95) as an eluent), followed by purification by preparative TLC, providing the pure title compounds as yellow powders with yields ranging from 20 to 30%. Both compounds were characterized by their mass spectrum as follows:

- 2-amino-4-mercaptoethyl-6-(3,4-dimethoxyphenyl) pteridine: 344 ([M+H]$^+$, 100);
- 2-amino-4-mercaptoisopropyl-6-(3,4-dimethoxyphenyl) pteridine: 357 ([M+H]$^+$, 100).

Example 106 - synthesis of a mixture of 2,4-diamino-6-(p-methoxyphenyl) pteridine and 2.4-diamino-7-(p-methoxyphenyl)pteridine

2,4,5,6-tetra-aminopyrimidine (10 mmoles, 1.4 g) was dissolved in water (50 ml) and the pH was adjusted to 9 with ammonium hydroxide. A solution of 4-methoxyphenylglyoxal (11 mmoles, 1.8 g) in ethanol (10 ml) was added dropwise and the solution was refluxed for 1 hour. The yellow precipitate formed was filtered off and washed with water, ethanol and diethyl ether. NMR analysis reveals the obtention of a mixture (1.2 g, 45 % yield) consisting of 87 % of 2,4-diamino-7-(p-methoxyphenyl)pteridine and 13 % of 2,4-diamino-7-(p-methoxyphenyl)pteridine. $^1$H-NMR (500 MHz, TFA): δ 4.04 (3 H, s), 4.08 (3 H, s), 7.15 (2 H, d), 7.25 (2 H, d), 8.19 (2 H, d), 8.30 (2 H, d), 9.27 (1 H, s) and 9.37 (1 H, s) ppm.

Example 107 - synthesis of a mixture of 2-amino-6-(p-methoxyphenyl)pterin and 2-amino-7-(*p*-methoxyphenyl)pterin

The mixture obtained in example 106 (1.2 g, 4.5 mmoles) was suspended in NaOH 1 N (80 ml) and refluxed till a solution was obtained. The hot solution was treated with acetic acid till pH 5, then cooled down and the resulting precipitate was filtered off and washed with water, ethanol and diethyl ether, providing a mixture of 2-amino-6-(p-methoxyphenyl)pterin and 2-amino-7-(p-methoxyphenyl) pterin as a yellow powder (1 g, yield: 82 %). Mass spectrum: 270 ($[M+H]^+$, 100).

Example 108 - synthesis of 2-acetylamino-6-(p-methoxyphenyl)pterin and 2-acetylamino-7-(p-methoxyphenyl)pterin

A suspension of the mixture obtained in example 107 (7.43 mmoles, 2 g) was suspended in a mixture of acetic anhydride (50 ml) and acetic acid (50 ml). The suspension was refluxed for 4 hours till a clear solution was obtained. Some insoluble material was filtered off and the solution was partly evaporated till precipation starts. Further precipitation was achieved overnight in the refrigerator. The resulting precipitate was filtered off and washed with ethyl acetate and diethyl ether, providing a mixture of 2-acetylamino-6-(p-methoxyphenyl)pterin and 2-acetylamino-7-(p-methoxyphenyl) pterin as a yellow powder (2.1 g, 91 % yield). Mass spectrum: 312 ($[M+H]^+$, 100).

Example 109 - synthesis of 2-acetylamino-4-(1,2,4-triazolyl)-6-(p-methoxy-phenyl) pteridine and 2-acetylamino-4-(1,2,4-triazolyl)-7-(p-methoxyphenyl) pteridine

To a suspension of the mixture obtained in example 108 (1.5 g, 4 mmoles) in dry pyridine (100 ml) was added 1,2,4-triazole (830 mg, 12 mmoles) and 4-chlorophenyl phosphorodichloridate (1 ml, 6 mmoles). The suspension was stirred for 2 days at room temperature under nitrogen. The solvents were removed *in vacuo*. The solid material was suspended in dichloromethane and washed with 2 % HCl. Evaporation of the solvents provided a mixture of 2-acetylamino-4-(1,2,4-triazolyl)-6-(p-methoxyphenyl) pteridine and 2-acetylamino-4-(1,2,4-triazolyl)-7-(p-methoxyphenyl)pteridine.

Example 110 - synthesis of 2-amino-4-isopropoxy-7-(p-methoxyphenyl) pteridine

To a suspension of the mixture obtained in example 109 (180 mg, 0.50 mmole) in isopropanol (8 ml) was added sodium (23 mg, 1 mmole). The suspension was stirred at room temperature overnight. The solvents were evaporated and the residue was purified by preparative TLC (silica, using a methanol/$CH_2Cl_2$ (7:93) mixture as the eluent). At this stage, both regio-isomers obtained were separated, thus providing the pure title compound as a yellow powder (yield: 45 %) which was further characterized by its mass spectrum: 312 ($[M+H]^+$, 65), 270 ($[M+H\text{-propene}]^+$, 100).

Example 111 - synthesis of 2-amino-4-isopropoxy-7-(3.4-dimethoxvphenyl) pteridine

The sequence of reactions described in examples 106 to 110 was followed, however starting from 3,4-dimethoxyphenylglyoxal instead of 4-methoxyphenylglyoxal in the first step. This provided 2-amino-4-isopropoxy-7-(3,4-dimethoxyphenyl) pteridine, a compound which was further characterized by its mass spectrum: 342 ($[M+H]^+$, 55), 300 ($[M+H\text{-propene}]^+$, 75).

Example 112 - synthesis of 2-amino-4-ethoxy-7-(3,4-dimethoxyphenyl) pteridine

The sequence of reactions described in examples 106 to 110 was followed, however starting from 3,4-dimethoxyphenylglyoxal instead of 4-methoxyphenylglyoxal in the first step, and from ethanol instead if isopropanol in the last step. This provided 2-amino-4-ethoxy-7-(3,4-dimethoxyphenyl) pteridine, a compound which was further characterized as follows:

- MS: 328 ($[M+H]^+$, 100), 300 ($[M+H\text{-ethene}]^+$, 40);
- $^1$H-NMR (500 MHz, DMSO-$d_6$): δ 1.44 (3 H, t), 3.86 (3 H, s), 3.88 (3 H, s), 4.54 (2 H, q), 7.13 (1 H, d), 7.16 (2 H, br s), 7.85 (1 H, d), 7.88 (1 H, dd) and 9.06 (1 H, s) ppm;
- $^{13}$C-NMR (125 MHz, DMSO-$d_6$): δ 14.25, 55.67, 55.76, 63.06, 110.39, 111.89, 121.13, 121.25, 128.24, 136.87, 149.28, 151.62, 155.82, 156.72, 162.03 and 166.70 ppm.

Example 113 - synthesis of 2-amino-4-methoxy-7-(3,4-dimethoxyphenyl) pteridine

The sequence of reactions described in examples 106 to 110 was followed, however starting from 3,4-dimethoxyphenylglyoxal instead of 4-methoxyphenylglyoxal in the first step, and from methanol instead if isopropanol in the last step. This provided 2-amino-4-ethoxy-7-(3,4-dimethoxyphenyl) pteridine, a compound which was further characterized by its mass spectrum: 314 ($[M+H]^+$,100), 300 ($[M+H\text{-methane}]^+$, 20).

EXAMPLE 114 - synthesis of 3,4-dimethoxyphenylglyoxalmonoxime (reference)

$SeO_2$ (0.33 mole) was heated to 50°C in a mixture of dioxane (250 ml) and water (10 ml). After dissolution of $SeO_2$, 3,4-dimethoxyacetophenone (0.3 mole) was added and the mixture heated under reflux for 16 hours. The hot solution was filtered in order to remove selenium, the filtrate was evaporated, the oily residue dissolved in $CHCl_3$ (300 ml), then washed with saturated $NaHCO_3$ solution (100 ml) and water. The organic phase was dried over $Na_2S_2O_4$, filtered and evaporated. The yellow oil was distilled in vacuum, the resulting 3,4-dimethoxyphenylglyoxal was dissolved in methanol (50 ml) and water (200 ml), then acetonoxime (0.25 mole) was added and the pH adjusted to 4 by 2 N HCl. The solution was heated to 50°C for 2 hours, then cooled to 0°C and the resulting crystals collected. After washing with cold water and drying in a vacuum desiccator, 3,4-dimethoxyphenylglyoxalmonoxime was obtained with a yield of 71 %, optionally recrystallized from $CHCl_3$ or acetone, and characterized by $^1H$-NMR (200 MHz, DMSO-$d_6$) showing peaks at 3.84 (3 H, s), 7.06 (1 H, d), 7.51 (1 H, s), 7.75 (1 H, d), 8.10 (1 H, s) and 12.51 (1 H, s) ppm.

Example 115 - alternative synthesis of 2-amino-4-isopropoxy-6-(3,4-dimethoxyphenyl) pteridine

To a suspension of the compound of example 59 (1.16 g, 6.34 mmole) in isopropanol (1.25 M HCl, 30 ml) was added the compound of example 114 (6.34 mmole, 1.32 g). The reaction mixture was refluxed for 5 hours, then cooled down and the pH was adjusted to 9 by the addition of an aqueous concentrated solution of $NH_3$. The precipitate was filtered off and further purified by flash chromatography over silica gel, using a isopropanol/$CH_2Cl_2$ (1:99 to 3:97) mixture as the eluent, thus providing as a yellow powder 1.34 g of 2-amino-4-isopropoxy-6-(3,4-dimethoxyphenyl)pteridine, i.e. the compound of example 98 (yield: 62 %).

Example 116 - synthesis of 2,6-diamino-4-(1,2,3,6-tetrahydropyridinyl)-pyrimidine

To a suspension of 6-chloro-2,4-diaminopyrimidine (6 g, 41 mmole) in toluene (50 ml) was added 1,2,3,6-tetrahydropyridine (8.23 ml, 91 mmole). The resulting suspension was heated to reflux until a solution was obtained, then the solution was refluxed for 5 hours. The reaction mixture was cooled down and water was added. The precipitate formed Was filtered off and washed with toluene, providing 2,6-diamino-4-(1,2,3,6-tetrahydropyridinyl)-pyrimidine as a white powder (7.4 g, yield 94 %).

Example 117 - synthesis of 5-nitroso-2,6-diamino-4-(1,2,3,6-tetrahydro-pyridinyl)pyrimidine

To a solution of the compound of example 116 (7.4 g, 38.7 mmole) in water (80 ml) and acetic acid (3.87 ml) was added a solution of sodium nitrite (2.94 g, 42.6 mmole) in water. The pink precipitate formed Was filtered off and washed with water, providing 5-nitroso-2,6-diamino-4-(1,2,3,6-tetrahydro-pyridinyl)pyrimidine (7.83 g) with a yield of 92 %.

Example 118 - synthesis of 2,5,6-triamino-4-(1,2,3,6-tetrahydropyridinyl)-pyrimidine

To a suspension of the compound of example 117 (4 g, 18 mmole) in water (40 ml) was added sodium dithionite (7.9 g, 45 mmole). The suspension was heated to 90 °C until a solution was obtained. After cooling down, the 2,5,6-triamino-4-(1,2,3,6-tetrahydropyridinyl)pyrimidine precipitate formed was filtered off and used as such for further reaction.

Example 119 - synthesis of 2-amino-4-(1,2,3,6-tetrahydropyridinyl)-6-(3,4-dimethoxyphenyl) pteridine

To a solution of the reaction product of example 118 (2.22 g, 10.8 mmole) in methanol (with 1 N HCl, 50 ml) was added the compound of example 114 (2.26 g, 10.8 mmole). The solution was refluxed for 3 hours. The pH of the reaction was adjusted to 8 by the addition of an aqueous concentrated ammonia solution. The resulting precipitate was filtered off and further purified by flash chromatography over silica gel, using a $CH_3OH/CH_2Cl_2$ mixture (3:97) as the eluent. This provided a yellow powder (2.56 g, yield 65 %) of 2-amino-4-(1,2,3,6-tetrahydropyridinyl)-6-(3,4-dimethoxyphenyl)pteridine which was characterized by the following spectra:

- $^1H$-NMR (200 MHz, DMSO-$d_6$): $\delta$ 3,84-3.88 (3 H, s), 4.45 (2 H, s), 4.75 (2 H, s), 5.85-5.94 (1 H, m), 7.12 (2 H, d), 7.40 (2 H, s), 7.66 (1 H, s), 7.72 (2 H, d) and 9.39 (1 H, s) ppm;

- MS: 365 ($[M+H]^+$, 100).

Examples 120 to 128 - synthesis of 2-amino-4-alkylamino-6-aryl-pteridines and 2-amino-4-arylalkylamino-6-aryl-pteridines

To a suspension of 2-acetylamino-4-(1,2,4-triazolyl)-6-[3,4-(dimethoxyphenyl)] pteridine (0.5 mmole) in dioxane (5 ml) was added the desired alkylamine or arylalkylamine (1 mmole). The suspension was stirred at room temperature for 16 hours. The solvent was evaporated *in vacuo* yielding crude 2-acetylamino-4-alkylamino-6-[3,4-(dimethoxyphenyl)] pteridine or 2-acetylamino-4-arylalkylamino-6-[3,4-(dimethoxyphenyl)] pteridine. Deprotection of the acetyl group was achieved by dissolving the crude residue in a mixture of $CH_3OH$ / 20% $K_2CO_3$ in water (1:1). The solution was stirred at room temperature for 16 hours. Evaporation of the solvents *in vacuo,* followed by purification of the residue by preparative TLC (silica, using a $CH_3OH/CH_2Cl_2$ mixture (5:95) as the eluent) afforded the desired compound as a yellow powder in yields ranging from 30 to 65 %, depending upon the starting alkylamine or arylalkylamine.

The following compounds were synthesized according to this procedure and characterized as follows:

- 2-amino-4-(diethanolamino)-6-[[3,4-(dimethoxyphenyl)]pteridine (example 120) synthesized from diethanolamine; MS: *m/z* (%): 387 ($[M+H]^+$, 100);
- 2-amino-4-(benzylamino)-6-[[3,4-(dimethoxyphenyl)]pteridine (example 121) synthesized from benzylamine; MS: *m/z* (%): 389 ($[M+H]^+$, 100);
- 2-amino-4-(phenylethylamino)-6-[[3,4-(dimethoxyphenyl)]pteridine (example 122) synthesized from phenethylamine; MS: *m/z* (%): 403 ($[M+H]^+$, 100);
- 2-amino-4-(4-methyl-piperidine)-6-[[3,4-(dimethoxyphenyl)]pteridine (example 123) synthe-sized from 4-methyl-piperidine; MS: *m/z* (%): 381 ($[M+H]^+$, 100);
- 2-amino-4-(2-thienylmethylamino)-6-[[3,4-(dimethoxyphenyl)]pteridine (example 124) synthe-sized from 2-thienylamine; MS: *m/z* (%): 395 ($[M+H]^+$, 100);
- 2-amino-4-(1,2,3,6-tetrahydropyridino)-6-[[3,4-(dimethoxyphenyl)] pteridine (example 125) synthesized from 1,2,3,6-tetrahydropyridine; MS: *m/z* (%): 365 ($[M+H]^+$, 100);
- 2-amino-4-thiomorpholine-6-[[3,4-(dimethoxyphenyl)]pteridine (example 126) synthesized from thiomorpholine; MS: *m/z* (%): 385 ($[M+H]^+$, 100);
- 2-amino-4-((*R*)-sec-butylamine)-6-[[3,4-(dimethoxyphenyl)]pteridine (example 127) synthe-sized from (*R*)-sec-butylamine; MS: *mlz* (%): 355 ($[M+H]^+$, 100); and
- 2-amino-4-((*S*)-sec-butylamine)-6-[[3,4-(dimethoxyphenyl)]pteridine (example 128) synthe-sized from (*S*)-sec-butylamine; MS: *m*/*z* (%): 355 ($[M+H]^+$, 100).

Examples 129 to 132 - synthesis of 2-substituted 4,6-diamino-5-nitroso-pyrimidines (reference)

To a suspension of 4,6-diamino-2-methylmercapto-5-nitroso-pyrimidine (1 g, 5.41 mmole), which may be prepared and characterised for instance as disclosed by Baddiley et al. in J. Chem. Soc. (1943) 383, in water (25 mi) was added a large excess (162 mmole) of an appropriate amine. After heating the reaction mixture at 65 ˚C during 3 hours, a pink suspension was formed. The reaction mixture was then cooled down to + 4˚C for 4 days. The pink precipitate was filtered off and washed with water, yielding the pure following compounds, each being characterised by its mass spectrum (MS), in yields ranging from 30 to 50%:

- 2-phenylethylamino-4,6-diamino-5-nitroso-pyrimidine (example 129) was obtained from phenylethylamine ; MS: m/z (%): 259 ($[M+H]^+$, 100).
- 2-(2-thienylmethylamino)-4,6-diamino-5-nitroso-pyrimidine (example 130) was obtained from 2-thiophenemethylamine ; MS: m/z (%): 251 ($[M+H]^+$, 100).
- 2-pyrrolidino-4,6-diamino-5-nitroso-pyrimidine (example 131) was obtained from pyrrolidine ; MS: m/z (%): 209 ($[M+H]^+$, 100).
- 2-benzylamino-4,6-diamino-5-nitroso-pyrimidine (example 132) was obtained from benzylamine ; MS: m/z (%): 245 ($[M+H]^+$, 100).

Example 133 to 136 - synthesis of 2-substituted-4,5,6-triamino-pyrimidine sulfates (reference)

To a suspension of a 2-substituted-4,6-diamino-5-nitroso-pyrimidine obtained in one of examples 129 to 132 (1 mmole) in water (25 ml) was added portionwise sodium dithionite (3 mmole). The resulting suspension was refluxed until a yellow solution was formed. A sulfuric acid solution (2.5 ml of a 50 % solution in water) was then added. The reaction mixture was cooled down to + 4˚C for 5 hours. The white precipitate formed was filtered off, yielding the pure

following compounds in yields ranging from 60% to 75%.

- 2-phenylethylamino- 4,5,6-triamino-pyrimidine sulfate (example 133),
- 2-(2-thienylmethylamino)- 4,5,6-triamino-pyrimidine sulfate (example 134),
- 2-pyrrolidino- 4,5,6-triamino-pyrimidine sulfate (example 135), and
- 2-benzylamino- 4,5,6-triamino-pyrimidine sulfate (example 136).

Examples 137 to 140 - synthesis of 2-substituted-4,5,6-triamino-pyrimidine dihydro-chlorides (reference)

To a suspension of a 2-substituted-4,5,6-triamino-pyrimidine sulfate obtained in one of examples 133 to 136 (1 mmole) in water (6 ml) at 80 ˚C was added dropwise a solution of barium chloride dihydrate (0.9 mmole) in water (2 ml). The resulting suspension was stirred for 30 minutes at 80 ˚C, then the reaction mixture was cooled down and barium sulfate was filtered off over Celite. The filtrate was evaporated in vacuo and co-evaporated with toluene yielding each of the following compounds as a yellow powder in yields ranging from 90 % to 98 %:

- 2-phenylethylamino- 4,5,6-triamino-pyrimidine dihydrochloride (example 137),
- 2-(2-thienylmethylamino)- 4,5,6-triamino-pyrimidine dihydrochloride (example 138),
- 2-pyrrolidino- 4,5,6-triamino-pyrimidine dihydrochloride (example 139), and
- 2-benzylamino- 4,5,6-triamino-pyrimidine dihydrochloride (example 140).

Example 141 - synthesis of 3,4-dimethoxyphenylglyoxalmonoxime (reference)

In a mixture of dioxane (250 ml) and water (10 ml), $SeO_2$ (0.33 mole) was heated to 50 ˚C. After solution of $SeO_2$, 3,4-dimethoxyacetophenone was added and the mixture heated under reflux for 16 hours. The hot solution was filtered to remove selenium. The filtrate was evaporated, the oily residue dissolved in $CHCl_3$ (300 ml), then washed with saturated $NaHCO_3$ solution (100 ml) and water. The organic phase was dried over $Na_2S_2O_4$, filtered and evaporated. The yellow oil was distilled in vacuum, the resulting 3,4-dimethoxyphenylglyoxal was dissolved in MeOH (50 ml) and water (200 ml), then acetonoxime (0.25 mol) was added and the pH adjusted to 4 by 2 N HCl. The solution was heated to 50 ˚C for 2 hours, then cooled to 0 ˚C and the resulting crystals collected. After washing with cold water and drying in a vacuum desiccator, 3,4-dimethoxyphenylglyoxalmonooxime was obtained with a yield of 71 %. Recrystallization can be achieved from $CHCl_3$ or acetone. The compound was further characterized by nuclear magnetic resonance spectra as follows: $^1$H-NMR (200 MHz, DMSO-$d_6$): δ 3.80 (3 H, s), 3.84 (3 H, s), 7.06 (1 H, d), 7.51 (1 H, s), 7.75 (1 H, d), 8.10 (1 H, s) and 12.51 (1 H, s) ppm.

Examples 142 to 145 - synthesis of 2-substituted-4-amino-6-(3,4-dimethoxy-phenyl) pteridines (reference)

The following procedure is in accordance with figure 6. To a solution of a 2-substituted-4,5,6-triamino-pyrimidine dihydrochloride obtained in one of examples 137 to 140 (1 mmole) in methanol (15 ml) was added the 3,4-dimethoxyphenylglyoxaloxime obtained according to example 141 (1 mmole). The resulting solution was refluxed for 2 hours, thus forming a yellow suspension. The reaction mixture was cooled down and neutralised by addition of a 33% aqueous ammonia solution until pH 9 was reached. The yellow precipitate was filtered off and further purified by silica gel flash chromatography (eluting with solvent mixture $CH_3OH/CH_2Cl_2$, gradient from 1:99 to 3:97), yielding as a yellow powder each of the pure following compounds, which was characterised by its mass spectrum (MS) and its ultraviolet spectrum (UV):

- 2-phenylethylamino-4-amino-6-(3,4-dimethoxyphenyl) pteridine (example 142) was obtained from the salt of example 75; MS: m/z (%): 403 ($[M+H]^+$, 100), 827 ($[2M+Na]^+$, 20); UV (MeOH, nm): 287, 315, 412.
- 2-(2-thienylmethylamino)- 4-amino-6-(3,4-dimethoxyphenyl) pteridine (example 143) was obtained from the salt of example 76; MS: m/z (%): 394 ($[M+H]^+$, 100); UV (MeOH, nm): 287, 314, 410.
- 2-pyrrolidino-4-amino-6-(3,4-dimethoxyphenyl) pteridine (example 144) was obtained from the salt of example 77; MS: m/z (%): 353 ($[M+H]^+$, 100), 727 ([2M+Na)]+, 10); UV (MeOH, nm): 319, 423.
- 2-benzylamino-4-amino-6-(3,4-dimethoxyphenyl) pteridine (example 145) was obtained from the salt of example 78; MS: m/z (%): 389 ($[M+H]^+$, 100); UV (MeOH, nm): 287, 315, 411.

Example 146 - synthesis of 4,5,6-triamino-2-methylmercaptopyrimidine dihydrochloride (reference)

To a suspension of a 2-methylmercapto-4,5,6-triamino-pyrimidine sulfate (44.3 mmole), which may be prepared and characterised for instance as disclosed by Taylor et al. in J. Am. Chem. Soc. (1952) 74:1644-1647, in water (135

ml) at 80 °C was added dropwise a solution of barium chloride dihydrate (39.8 mmole) in water (25 ml). The suspension was stirred for 30 minutes at 80 °C. The reaction mixture was cooled down and barium sulfate was filtered off over Celite. The filtrate was evaporated in vacuo and co-evaporated with toluene yielding the title compound as a yellow powder (10.2 g, 94 % yield).

Example 147 - synthesis of 4-amino-2-methylmercapto-6-(3,4-dimethoxyphenyl) pteridine (reference)

To a suspension of 4,5,6-triamino-2-methylmercaptopyrimidine dihydrochloride (7.42 mmole, 1.81 g) in methanol (20 ml) was added a solution of 3,4-dimethoxyphenylglyoxaloxime (5.94 mmole, 1.24 g) in methanol. The resulting reaction mixture was refluxed for 3 hours. The reaction mixture was neutralised with concentrated aqueous ammonia until pH 9 was reached. The resulting precipitate was filtered off and further purified by flash chromatography (silica, using an ethyl acetate / hexane mixture in a 4:6 ratio) yielding the pure title compound as a yellow powder which was characterised as follows: MS: m/z (%) 330 ($[M+H]^+$, 100), 681 ($[2M+Na]^+$, 30); UV (MeOH, nm): 292, 397.

Example 148 - synthesis of 4-amino-2-methylmercapto-6-phenyl-pteridine (reference)

A method similar to that of example 147 was used, starting from phenylglyoxal monoxime instead of 3,4-dimethoxyphenylglyoxalmonoxime. The title compound was characterised as follows: MS: m/z (%): 270 ($[M+H]^+$, 100); UV (MeOH, nm): 286, 379.

Example 149 - synthesis of 4-amino-2-morpholino-6-(3,4-dimethoxyphenyl) pteridine (reference)

A solution of the compound of example 147 (100 mg, 0.304 mmole) in morpholine (12 ml) was refluxed overnight. The solvents were removed in vacuo and the residue was purified first by flash chromatography (silica, gradient from 2:98 to 3:97 $CH_3OH$ /$CH_2Cl_2$) and then by preparative TLC (silica, EtOAc/hexane 1 :1) yielding the title compound as a yellow powder (70 mg, 63 % yield) characterised as follows: MS: m/z (%): 369 ($[M+H]^+$, 100), 759 ($[2M+Na]^+$, 20); UV (MeOH, hm): 297, 315, 418.

Example 150 - synthesis of 4-amino-2-piperidino-6-(3,4-dimethoxyphenyl) pteridine (reference)

A method similar to that of example 149 was used, starting from piperidine instead of morpholine. The title compound obtained as a yellow powder (58 mg, 52 %) was characterised as follows: MS: m/z (%): 367 ($[M+H]^+$, 100), 755 ($[2M+Na]^+$, 10); UV (MeOH, nm): 319, 425.

Example 151 - synthesis of 2,4-di-(thienyl-2-methylamino)-6-(3,4-dimethoxy-phenyl) pteridine (reference)

A method similar to that of example 149 was used, starting from thiophenemethylamine instead of morpholine. The title compound obtained as a yellow powder (85 mg, 57 %) was characterised as follows: MS: m/z (%): 491 ($[M+H]^+$, 100); UV (MeOH, nm): 229, 296, 414.

Example 152 - synthesis of 2,5,6-triamino-4-morpholino-pyrimidine dihydro-chloride

In a 10 L 4-neck flask equipped with a mechanical stirrer, a thermometer and a heating mantle was placed 2,6-diamino-4-chloropyrimidine (870.5 g; 6.0 mol) in water (3190 ml). To the stirred resulting white suspension was added morpholine (1113 g; 12.8 moles) in one portion. The temperature raised from 14 to 28°C and the mixture was heated until at 75°C a clear solution was obtained and refluxed for 16 hours. After complete conversion of the starting material (controlled by HPLC analysis) the reaction mixture was cooled to room temperature and $NaNO_2$ (465 g, 6.74 mole) was added in three portions. No heat effect was observed but the product precipitated out of the mixture. During subsequent dropwise addition of acetic acid (600 g, 9.0 moles) the mixture became so thick that faster stirring and additional water (850 mL) were necessary to achieve a well mixable suspension. A temperature raise from 15 to 28°C was measured. After stirring for 2hours, HPLC analysis showed complete conversion. The mixture was then allowed to cool down to room temperature and transferred to a 20L flask. To the acid (pH 4) purple slurry was added water (3500 mL) and then an aqueous NaOH solution (165 g in 825 mL) in order to neutralise to pH 7. The suspension was cooled down to 10-12°C in an ice-water bath and $Na_2S_2O_4$ (4.68 kg, 26.9 moles) was added in portions of about 500 g at 5 minutes intervals. A temperature raise to 30°C was observed. The mixture was left stirring overnight at room temperature before being analysed (HPLC: 99.3% conversion). The suspension was filtered over filter cloth and the wet cake was washed with 2.77 L water leaving 1.5 kg of wet material which was slurried in isopropanol (10.2 L) and to the suspension was added dropwise 36% $HCl/H_2O$ (1.16L) during which the temperature raised from 15 to 30°C. Subsequent heating

of the slurry to 40˚C afforded an almost clear solution and crystallisation started suddenly showing an exotherm to 50˚C. The suspension was cooled down to room temperature before the suspension was filtered over filter cloth. The wet cake was again slurried in isopropanol (5.0 L), again filtered over filter cloth and dried at 40˚C in vacuo (200 mbar) for several days. According to this procedure was obtained 1.2 kg (yield 54.1%) of 2,5,6-triamino-4-morpholino-pyrimidine dihydrochloride with a purity above 99.9% and characterised as follows: UV (MeOH, nm): 225, 254.

Example 153 - synthesis of 4-acetamidophenylglyoxalmonoxime (reference)

$SeO_2$ (34.18 g, 0.308 mole) and 4-acetamidoacetophenone (49.62 g, 0.28 mole) were suspended in a mixture of dioxane (250 ml) and water (10 ml) and the solution was heated to 100˚C for 16 hours. The hot solution was filtered on a paper filter and the filtrate was evaporated to dryness. The oily residue was then partitioned between $CHCl_3$ (400 ml) and a saturated solution of sodium hydrogen-carbonate (200 ml). A precipitation occurs in the aqueous layer. The organic phase was collected and the aqueous layer was filtered over a fritted glass. The solid (4-acetamidoglyoxaldehyde) was washed with water and kept for the next step. The aqueous layer was extracted several times with $CHCl_3$ and all fractions were collected and evaporated to dryness. The oily residue and the precipitate were put together and suspended in a mixture of water (240 ml) and MeOH (60 ml). Then acetonoxime (20.46 g, 0.28 mole) was added and the pH was adjusted to a range of 3 - 4 with 1 N HCl. The solution was heated to 70 ˚C for 1 hour, then cooled to 0 ˚C and the resulting crystals collected. After washing with cold water, then diethylether, drying in a vacuum dessicator over $P_2O_5$ afforded 33.6 g of the desired compound (yield 58%) as yellowish crystals with a purity of 95%, which were characterised as follows: MS: m/z (%): 207 ($[M+H]^+$, 100); W (MeOH, nm): 241.6, 278.3.

Example 154 - synthesis of 3-acetamidophenylglyoxalmonoxime (reference)

$SeO_2$ (36.62 g, 0.33 mole) and 3-acetamidoacetophenone (53.16 g, 0.30 mole) were suspended in a mixture of dioxane (250 ml) and water (10 ml) and the solution was heated to 100˚C for 16 hours. The hot solution was filtered on a paper filter and the filtrate was evaporated to dryness. The oily residue was then suspended in a mixture of water (240 ml) and MeOH (60 ml) and acetonoxime (21.93 g, 0.30 mol) was added and the pH was adjusted to a range of 3 - 4 with 6 N HCl. The solution was heated to 70 ˚C for 2 hours and after cooling, the brownish precipitate was filtered and re-crystallised from toluene to afford 45.0 g of the desired compound (yield 72%) with a purity of 98%, which was characterised as follows: MS: m/z (%): 207 ($[M+H]^+$, 100), 229 ($[M+Na]^+$, 80); UV (MeOH, nm): 239.2.

Example 155 - synthesis of 2-amino-4-morpholino-6-(4-acetanilide) pteridine

2,4,5-triamino-6-morpholinopyrimidine dihydrochloride (14.1 g, 50 mmole) was refluxed in MeOH (300 ml) and the compound of example 153 (11.33 g, 55 mmole) in MeOH (150 ml) was added. The mixture was refluxed for 3 hours and after cooling, the yellowish precipitate was filtered, washed with water, methanol, ether and dried at 110˚C for 3 hours to afford 15.7 g of the desired product (yield 86%) which, after re-crystallisation from DMF/$H_2O$, was obtained with a purity of 97% and characterised as follows: MS: m/z (%): 366 ($[M+H]^+$, 100), 753 ($[2M+Na]^+$ ,15); UV (MeOH, nm): 213, 307, 408.

Example 156 - synthesis of 2-amino-4-morpholino-6-(3-acetanilide) pteridine

Repeating the procedure of example 155 but starting from the compound of example 154, the title pteridine derivative (16.1 g) was obtained with a yield of 88%, and a purity of 97%, and characterised as follows: MS: m/z (%): 366 ($[M+H]^+$, 100), 753 ($[2M+Na]^+$ ,80); UV (MeOH, nm): 220, 294, 402.

Example 157 - synthesis of 2-amino-4-morpholino-6-(4-aminophenyl) pteridine

The crude compound of example 155 (20 mmole) was refluxed in a mixture of MeOH/HCl 6N-1/1 (400 ml) for 2 hours and cooled in an ice bath. The remaining solid was filtered and the pH was adjusted to 10 with NaOH (10N). The orange precipitate was filtered, washed with water, ethanol, ether and dried in a vacuum dessicator over $P_2O_5$ to afford 4.7 g (yield: 73%) of the desired compound as an orange solid with a purity of 98%, which was characterised as follows: MS: m/z (%): 324 ($[M+H]^+$, 100); UV (MeOH, nm): 216, 316, 422; $^1H$ NMR (200 MHz, in DMSO-d6, ppm): 3.78 (br s, 4H); 4.33 (br s, 4H); 5.54 (br s, 2H); 6.63 (br s, 2H); 6.67 (d, 2H, J = 8.4 Hz); 7.75 (d, 2H, J = 8.4 Hz); 9.15 (s, 1H).

Example 158 synthesis of 2-amino-4-morpholino-6-(3-aminochenyl) pteridine

Repeating the procedure of example 157 but starting from the compound of example 156, the title pteridine

derivative (4.88 g) was obtained with a yield of 74% and a purity of 99.34%, and characterised as follows: MS: m/z (%): 324 ([M+H]$^+$, 100); UV (MeOH, nm): 218, 292,403.

Examples 159 to 165 - coupling reaction between a 2-amino-4-morpholino-6-(4-amino-phenyl) pteridine and a carboxylic acid chloride, sulfonyl chloride or carbamoyl chloride

The compound of example 157 (162 mg, 0.5 mmole) was suspended in dioxane (10 ml) and triethylamine was added (84 µl, 0.6 mmole). Then a suitable carboxylic acid chloride or sulfonyl chloride or carbamoyl chloride (0.55 mmole) was added and the mixture was stirred at room temperature until completion of the reaction. After evaporation to dryness, the crude resulting product was purified by flash chromatography on silica gel using a gradient of MeOH (1-5%) in dichloromethane. Yields varied from 50 to 80%. Using this procedure, the following pteridine derivatives were prepared:

- 2-amino-4-morpholino-6-(4-benzoylaminophenyl) pteridine (example 159), purity 98.94%, characterised as follows: MS: m/z (%): 428 ([M+H]$^+$, 100), 877 ([2M+Na]$^+$, 40); UV (MeOH, nm): 313, 408;
- 2-amino-4-morpholino-6-(4-phenoxyacetylaminophenyl) pteridine (example 160), purity 97.18%, characterised as follows: MS: m/z (%): 458 ([M+H]$^+$, 100); UV (MeOH, nm): 305, 407;
- 2-amino-4-morpholino-6-(4-propionylaminophenyl) pteridine (example 161), purity 98.86%, characterised as follows: MS: m/z (%): 380 ([M+H]$^+$, 100), 781 ([2M+Na]$^+$, 20); UV (MeOH, nm): 213, 307, 408;
- 2-amino-4-morpholino-6-(4-furoylaminophenyl) pteridine (example 162), , purity 93.96%,

characterised as follows: MS: m/z (%): 418 ([M+H]$^+$, 100); UV (MeOH, nm): 213, 316, 408;

- 2-amino-4-morpholino-6-(4-cyclohexanoylaminophenyl) pteridine (example 163), purity 96.91%, characterised as follows: MS: m/z (%): 434 ([M+H]$^+$, 100), 889 ([2M+Na]$^+$, 10); UV (MeOH, nm): 308, 408;
- 2-amino-4-morpholino-6-[4-(4-chlorobenzoyl)aminophenyl] pteridine (example 164), purity 96.48%, characterised as follows: MS: m/z (%): 462 ([M+H]$^+$, 100); UV (MeOH, nm): 313.9, 407; and
- 2-amino-4-morpholino-6-(4-benzyloxyacetylaminophenyl) pteridine (example 165), purity 98.45%, characterised as follows: MS: m/z (%): 472 ([M+H]$^+$, 100), 942 ([2M+H]$^+$, 5), 965 ([2M+Na]$^+$, 10); UV (MeOH, nm): 307, 407.

Examples 166 to 180 - coupling reaction between a 2-amino-4-morpholino-6-aminophenyl pteridine and a carboxylic acid chloride, sulfonyl chloride or carbamoyl chloride

The compound of example 157 or example 158 (162 mg, 0.5 mmole) was suspended in pyridine (10 ml). Then a suitable carboxylic acid chloride or sulfonyl chloride or carbamoyl chloride (0.55 mmole) was added and the mixture was stirred at room temperature until completion of the reaction. After evaporation to dryness, the crude resulting product was purified by flash chromatography on silica gel using a gradient of MeOH (1-5%) in dichloromethane. Yields varied from 20 to 80%. Using this procedure, the following pteridine derivatives were prepared:

- 2-amino-4-morpholino-6-(4-isonicotinoylaminophenyl) pteridine (example 166) was characterised as follows: MS: m/z (%): 429 ([M+H]$^+$, 100), 879 ([2M+Na]$^+$, 5); UV (MeOH, nm): 213, 313, 407; purity 95.01%;
- 2-amino-4-morpholino-6-(4-naphtoylaminophenyl) pteridine (example 167) was characterised as follows: MS: m/z (%): 478 ([M+H]$^+$, 100), 977 ([2M+Na]$^+$, 5); UV (MeOH, nm): 213, 313, 407; purity 95.01%;
- 2-amino-4-morpholino-6-(4-methylsulfonylaminophenyl) pteridine (example 168) was characterised as follows: MS: m/z (%): 502 ([M+H]$^+$, 100); UV (MeOH, nm): 301, 422; purity 96.81 %;
- 2-amino-4-morpholino-6-(4-ethylsuccinylaminophenyl) pteridine (example 169) was characterised as follows: MS: m/z (%): 452 ([M+H]$^+$, 100), 925 ([2M+Na]$^+$, 15); UV (MeOH, nm): 213, 308, 408; purity: 98.98%;
- 2-amino-4-morpholino-6-[4-(4-methylbenzoate)aminophenyl) pteridine (example 170) was characterised as follows: MS: m/z (%): 486 ([M+H]$^+$ 100); UV (MeOH, nm): 236, 315, 407; purity 99.57%;
- 2-amino-4-morpholino-6-(3-benzoylaminophenyl) pteridine (example 171) was characterised as follows: MS: m/z (%): 428 ([M+H]$^+$, 100); UV (MeOH, nm): 216, 293, 402; purity: 96.46%;
- 2-amino-4-morpholino-6-(3-benzensulfonylaminophenyl) pteridine (example 172) was characterised as follows: MS: m/z (%): 464 ([M+H]$^+$, 100); UV (MeOH, nm): 216, 295, 402; purity: 97.49%;
- 2-amino-4-morpholino-6-(3-phenoxyacetylaminophenyl) pteridine (example 173) was characterised as follows: MS: m/z (%): 458 ([M+H]$^+$, 100); UV (MeOH, nm): 219, 294, 402; purity 98.96%;
- 2-amino-4-morpholino-6-(3-isonicotinoylaminophenyl) pteridine (example 174) was characterised as follows: MS: m/z (%): 429 ([M+H]$^+$, 100); UV (MeOH, nm): 216, 294, 402; purity 93.92%;
- 2-amino-4-morpholino-6-(3-cyclohexanoylaminophenyl) pteridine (example 175) was characterised as follows: MS:

m/z (%): 434 ([M+H]$^+$, 100); UV (MeOH, nm): 222, 245, 294, 402; purity 99.50%;

- 2-amino-4-morpholino-6-[3-(4-methylbenzoate)aminophenyl] pteridine (example 176) was characterised as follows: MS: m/z (%): 486 ([M+H]$^+$, 100); UV (MeOH, nm): 218, 238, 294, 402; purity 97.44%;
- 2-amino-4-morpholino-6-(3-ethylsuccinylaminophenyl) pteridine (example 177) was characterised as follows: MS: m/z (%): 452 ([M+H]$^+$, 100); UV (MeOH, nm): 222, 245, 294, 402; purity 94.16%;
- 2-amino-4-morpholino-6-(3-ethylmalonylaminophenyl) pteridine (example 178) was characterised as follows: MS: m/z (%): 438 ([M+H]$^+$, 100); UV (MeOH, nm): 220, 244, 294, 402; purity 90.89%;
- 2-amino-4-morpholino-6-(3-benzyloxyacetylaminophenyl) pteridine (example 179) was characterised as follows: MS: m/z (%): 472 ([M+H]$^+$, 100); UV (MeOH, nm): 216, 243, 294, 402; purity 99.70%; and
- 2-amino-4-morpholino-6-(3-ethylsulfonylaminophenyl)pteridine (example 180) was characterised as follows: MS: m/z (%): 4716 ([M+H]$^+$, 100); UV (MeOH, nm): 218, 295, 402; purity 92.54%.

Examples 181 to 184 - coupling reaction between 2-amino-4-moroholino-6-(3-aminophenyl) pteridine and various carboxylic acids

The compound of example 158 (323 mg, 1 mmole), a carboxylic acid (1.1 mmole) and DIEA (2 mmole) were suspended in dry DMF (10 ml) and benzotriazol-1-yloxy tris(dimethylamino)phosphonium hexafluorophosphate) was added (1.1 mmole). The mixture was stirred at room temperature until completion of the reaction and then diluted with water. The aqueous layer was extracted with chloroform and the organic layer was evaporated to dryness. The crude resulting product was purified on silica gel using a gradient of MeOH (1-5%) in dichloromethane. Yields varied from 20 to 50%. The following compounds were made according to this procedure:

- 2-amino-4-morpholino-6-[3-Boc-(L)-phenylalanine-aminophenyl] pteridine (example 181) was characterised as follows: MS: m/z (%): 1141 ([2M+H]$^+$, 10), 571 ([M+H]$^+$, 100), 515 ([M-tBu+H]$^+$, 50), 471 ([M-Boc+H]$^+$, 10); UV (MeOH, nm): 213, 245, 294, 402; purity 96.72%;
- 2-amino-4-morpholino-6-[3-Boc-(D)-phenylalanine-aminophenyl] pteridine (example 182) was characterised as follows: MS: m/z (%): 571 ([M+H]$^+$, 100), 515 ([M-tBu+H]$^+$, 50), 471 ([M-Boc+H]$^+$, 10); UV (MeOH, nm): 213, 245, 294, 402; purity: 95.30%;
- 2-amino-4-morpholino-6-[3-Boc-(L)-tryptophane-aminophenyl] pteridine (example 183) was characterised as follows: MS: m/z (%): 610 ([M+H]$^+$, 100), 554 ([M-tBu+H]$^+$, 50), 510 ([M-Boc+H]$^+$, 10); UV (MeOH, nm): 220, 291, 402; purity 91.74%; and
- 2-amino-4-morpholino-6-[3-Boc-(D)-tryptophane-aminophenyl] pteridine (example 184) was characterised as follows: MS: m/z (%): 610 ([M+H]$^+$, 100), 554 ([M-tBu+H]$^+$, 50), 510 ([M-Boc+H]$^+$, 10); UV (MeOH, nm): 220, 291, 402; purity 84.25%.

Example 185 - synthesis of 2-amino-4-morpholino-6-(4-hydroxyphenyl) pteridine

To a solution of 2,5,6-triamino-4-morpholino-pyrimidine dihydrochloride salt (5.05 g, 17.8 mmole) in methanol (120 ml) was added 4-hydroxyphenylglyoxalmonoxime (2.95 g, 17.8 mmole). The reaction mixture was refluxed for 3 hours and then cooled down to room temperature. The yellow precipitate was filtered off, washed with water yielding the title compound as a chromatographically pure yellow powder which was further characterised as follows: MS: m/z (%): 671 ([2M+Na]$^+$, 5), 325 ([M+H]$^+$, 100); UV (MeOH, nm): 213, 302, 411.

Examples 186 to 194 - synthesis of 2-amino-4-morpholino-6-(4-alkoxyphenyl) pteridines

The following procedure is in accordance with figure 10. To a solution of the compound of example 185 (0.65 mmole, 210 mg) in DMF was added $K_2CO_3$ (0.78 mmole, 108 mg) and an appropriate alkyl halide (0.78 mmole). The reaction was stirred overnight at room temperature. The reaction was quenched with water and extracted with $CH_2Cl_2$. The organic phase was evaporated in vacuo and the residue purified by flash chromatography (silica, gradient from 1: 99 to 3:97 $CH_3OH/CH_2Cl_2$) yielding the title compound as a yellow powder in yields ranging from 15 to 55 %. The following compounds were made using this procedure:

- 2-amino-4-morpholino-6-(4-ethoxyphenyl) pteridine (example 186) was obtained from iodoethane and characterised as follows: MS: m/z (%): 727 ([2M+Na]$^+$, 5), 353 ([M+H]$^+$, 100); UV (MeOH, nm): 211, 302, 410;
- 2-amino-4-morpholino-6-(4-benzyloxyphenyl) pteridine (example 187) was obtained from benzyl bromide and characterised as follows: MS: m/z (%): 727 ([2M+Na]$^+$, 5), 353 ([M+H]$^+$, 100); UV (MeOH, nm): 245,302,410;
- 2-amino-4-morpholino-6-(4-(phenethyloxy)-phenyl) pteridine (example 188) was obtained from phenylethyl bromide and characterised as follows: MS: m/z (%): 428 ([M+H]$^+$, 100); UV (MeOH, nm): 245, 303, 410;

- 2-amino-4-morpholino-6-(4-phenoxy-butyronitrile) pteridine (example 189) was obtained from 4-bromobutyronitrile and characterised as follows: MS: m/z (%): 391 ($[M+H]^+$, 100);
- 2-amino-4-morpholino-6-(4-propoxy-phenyl) pteridine (example 190) was obtained from propyl iodide and characterised as follows: MS: m/z (%): 367 ($[M+H]^+$ 100);
- 2-amino-4-morpholino-6-(4-phenoxy-butyric acid ethyl ester) pteridine (example 191) was obtained from ethyl-4-bromobutyrate and characterised as follows: MS: m/z (%): 439 ($[M^+H]^+$, 100);
- 2-amino-4-morpholino-6-(4-phenoxy-acetic acid ethyl ester) pteridine (example 192) was obtained from ethyl bromoacetate and characterised as follows: MS: m/z (%): 843 ($[2M+H]^+$, 100), 411 ($[M+H]^+$, 100;
- 2-amino-4-morpholino-6-(4-(2-methoxyethoxy)-phenyl) pteridine (example 193) was obtained from 2-bromoethylmethylether and characterised as follows: MS: m/z (%): 787 ($[2M+H]^+$, 25), 383 ($[M+H]^+$, 100); and
- 2-amino-4-morpho)ino-6-(4-butoxy-phenyl)-pteridine (example 194) was obtained from bromobutane and characterised as follows: MS: m/z (%): 381 ($[M+H]^+$, 100).

Example 195 - TNF-α and IL-1 β (reference) assays

Peripheral blood mononuclear cells (herein referred as PBMC), in response to stimulation by lipopolysaccharide (LPS), a gram-negative bacterial endotoxin, produce various chemokines, in particular human TNF-α and IL-1 β. The inhibition of the activation of PBMC can be measured by the level of suppression of the production of TNF-α or IL-1 β by PBMC in response to stimulation by LPS.

Such inhibition measurement was performed as follows: PBMC were isolated from heparinized peripheral blood (Buffy coat) by density gradient centrifugation. LPS is then added to the PMBC suspension in complete medium ($10^6$ cells /ml) at a final concentration of 1 μg/ml. The pteridine derivative to be tested was added at different dilution levels, and the cells were incubated at 37°C for 72 hours. The supernatants were collected, and TNF-α or IL-1β concentrations were measured with respectively an anti-TNF antibody or an anti-IL-1 β antibody in a sandwich ELISA (Duo Set ELISA human TNFα, commercially available from R&D Systems, United Kingdom). The colorimetric reading of the ELISA was measured by a Multiskan RC plate reader (commercially available from ThermoLabsystems, Finland) at 450 nm (reference wavelength: 690 nm). Data analysis was performed with Ascent software 2.6. (also from ThermoLabsystems, Finland): a standard curve (recombinant human TNFα) was drawn and the amount (pg/ml) of each sample on the standard curve was determined.

The % inhibition of human TNF-α production or human IL-1β was calculated using the formula :

$$\% \text{ inhibition} = (\text{pg/ml in sample} - \text{pg/ml min.}) / (\text{pg/ml max.} - \text{pg/ml min.}) - 1$$

wherein:

- min.: pg/ml in culture medium without test compound, and
- max.: pg/ml in culture medium + LPS without test compound.

Tables 1 and 2 hereinafter show the $IC_{50}$ values (expressed in μM) of the tested compounds, being represented by the general formula (I), in these TNF-α and IL-1 assays.

TABLE 1

| Example n° | TNF-α | IL-1 |
|---|---|---|
| 24 | 0.4 | 5.5 |
| 85 | 6.7 | > 40 |
| 98 | 3.5 | > 40 |
| 119 | 0.5 | 15 |

TABLE 2

| Example n° | TNF-α | Example n° | TNF-α | Example n° | TNF-α |
|---|---|---|---|---|---|
| 59 | 8.0 | 83 | 6.6 | 120 | 3.4 |

(continued)

| Example n° | TNF-α | Example n° | TNF-α | Example n° | TNF-α |
|---|---|---|---|---|---|
| 63 | 4.0 | 85 | 6.7 | 121 | 6.5 |
| 65 | 9.1 | 87 | 7.5 | 122 | 7.1 |
| 66 | 4.5 | 89 | 9.1 | 123 | 0.3 |
| 67 | 10.0 | 90 | 6.6 | 124 | 7.2 |
| 68 | 10.0 | 91 | 6.2 | 125 | 0.5 |
| 72 | 8.5 | 92 | 10.0 | 126 | 0.5 |
| 77 | 8.1 | 94 | 7.6 | 127 | 0.2 |
| 78 | 8.5 | 95 | 6.3 | 128 | 0.3 |
| 80 | 6.8 | 97 | 9.1 | 142 | 9.8 |
| 81 | 10.0 | 98 | 3.5 | 143 | 10.0 |
| 145 | 9.3 | 155 | 8.4 | 157 | 2.5 |
| 160 | 4.9 | 166 | 4.4 | 171 | 4.3 |
| 172 | 0.5 | 174 | 7.3 | 175 | 5.0 |
| 177 | 5.5 | 179 | 7.7 | 180 | 10.0 |
| 181 | 2.9 | 185 | 2.1 | 186 | 10.0 |
| 190 | 10.0 | 191 | 0.9 | 192 | 0.6 |
| 193 | 7.2 | | | | |

Example 196 - protection against lethal toxic shock (reference)

When a control group of 14 sham treated (saline injection) C3H mice are injected intraperitoneously with 100 μg LPS per mouse, all animals die within 1-3 days after injection. However when a group (16 animals) of the same C3H mice were treated for 2 days with the pteridine derivative of example 24 (intraperitoneous administration of 20 mg/kg/day; a first injection at the same time as the LPS injection, a second injection 24 hours later), all mice were significantly protected from acute shock related mortality:

- 14 animals permanently survived the challenge of a LPS lethal dose, and
- 2 animals showed a significantly prolonged survival (5 days).

Thus it can be concluded that treatment with a pteridine derivative of the invention provides an unexpectedly nearly complete protection against lethal toxic shock.

Example 197 - protection against a lethal dose of TNF-α

A model of TNF-α induced shock in C57BL/6 male mice was performed as follows. Animals from the control group received an intravenous administration of a lethal dose of TNF-α (10 μg) in the tail. Animals from the test group received three intraperitoneous injections of a pteridine derivative (20 mg/kg/day) respectively 48 hours, 24 hours and immediately before an intravenous injection of TNF-α (10 μg).

Body temperature, a clinical sign of TNF-induced shock, was followed for 48 hours in control mice and in mice receiving the pteridine derivative of example 24: the body temperature of control mice dropped significantly (28.2°C) when compared to mice receiving the test compound (30.1 °C).

The survival rate (at least 50%) of mice that received the pteridine derivatives of the invention in addition to the TNF-α dose (10 μg) was quite substantial, as shown in table 3.

TABLE 3

| Example n° | Total number of animals | Animals surviving 48 hours after TNF-α administration |
|---|---|---|
| control | 9 | 0 |
| 24 | 9 | 7 |
| 98 | 6 | 3 |
| 119 | 6 | 3 |

This model therefore demonstrates that *in vivo* treatment with a pteridine derivative of this invention provides substantial and significant protection against a lethal dose of TNF-α.

Example 198 - reduction of tumor growth while inhibiting TNF-α toxicity

A tumor model of melanoma (B16BL/6) in C57BL6 (B6) mice was performed as follows. On day 1, a group of 18 B6 mice were injected with 1.5 x $10^6$ B16BL/6 melanosarcoma cells. The group was further divided into the following sub-groups 3 days after the tumor cells injection:

- a first control sub-group of 6 mice received vehicle (physiological solution) 3 times a week starting on day 3;
- a second control sub-group of 5 mice received TNF-α (15 μg) on day 5 and, for surviving animals, TNF-α was again administered 3 times a week for 2 weeks;
- a test sub-group of 7 mice intraperitoneously received the pteridine derivative of example 24 at a dose of 20 mg/kg on each of days 3, 4 and 5; the latter third injection on day 5 occurred 2 hours before a first TNF-α intravenous injection (15 μg). Then the test compound (20 mg/kg/day) and TNFα (15 μg/day) were administered each subcutaneously 3 times a week for 2 weeks.

Substantial protection against TNF-α toxicity was achieved by the pteridine derivative of the invention in tumor bearing mice: 5 out of 7 animals of the test sub-group survived, compared to none of the second control group (which all died during day 5).

Furthermore, histological studies after two weeks treatment show that administration of the pteridine derivative of example 24 together with TNF-α leads to a significant reduction of tumor growth: the average tumor size (tumor size was measured as the largest diameter multiplied by the smallest diameter) in the test sub-group was 144 $mm^2$, whereas the average tumor size in the first control group was 439 $mm^2$. These data clearly demonstrate that the pteridine derivative of example 24 effectively protects mice against the toxicity of TNF-α while preserving its anti-tumor effects.

**Claims**

1. Use of a pteridine derivative for the manufacture of a medicament for the prevention or treatment of a disorder in a mammal, the said disorder being selected from the group consisting of toxic effects of TNF-α, alcohol-induced hepatitis, and cachexia,
   the said pteridine derivative having the general formula (1):

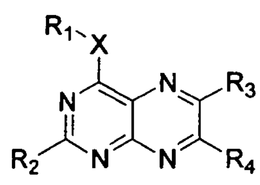

   wherein X represents an oxygen atom or a group with the formula $S(O)_m$ wherein m is an integer from 0 to 2, or a group with the formula NZ and wherein:

   - $R_1$ is selected from the group consisting of methyl, ethyl, isopropyl and pentyl;
   - Z is a group independently defined as $R_1$ or Z is hydrogen or the group NZ together with $R_1$ is either hydroxylamino or an optionally substituted heterocyclic group containing at least one nitrogen atom;

- $R_2$ is selected from the group consisting of amino; acylamino;
- $R_4$ is an atom or a group selected from the group consisting of hydrogen; halogen; $C_{1-7}$ alkyl; $C_{2-7}$ alkenyl; $C_{2-7}$ alkynyl; halo $C_{1-7}$ alkyl; carboxy $C_{1-7}$ alkyl; acetoxy $C_{1-7}$ alkyl; carboxyaryl; $C_{1-7}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; amino; hydroxylamino; mercapto-amino; acylamino; thioacylamino; alkoxyamino; thioalkylamino; acetal; thioacetal; carboxylic acid; carboxylic acid esters, thioesters, halides, anhydrides, amides and thioamides; thiocarboxylic acid; thiocarboxylic acid esters, thioesters, halides, anhydrides, amides and thioamides; hydroxyl; sulfhydryl; nitro; cyano; carbamoyl; thiocarbamoyl, ureido; thio-ureido; alkylamino; cycloalkylamino; alkenylamino; cycloalkenylamino; alkynyl-amino; arylamino; arylalkylamino; hydroxyalkylamino; mercapto-alkylamino; heterocyclic amino; heterocyclic-substituted alkylamino; oximino; alkyloximino; hydrazino; alkylhydrazino; phenylhydrazino; cysteinyl acid, esters, thioesters, halides, anhydrides, amides and thioamides thereof; aryl groups optionally substituted with one or more substituents selected from the group consisting of halogen, $C_{1-7}$ alkyl, $C_{1-7}$ alkoxy; optionally substituted heterocyclic radicals; aromatic or heterocyclic substituents substituted with an aliphatic spacer between the pteridine ring and the aromatic or heterocyclic substituent, whereby said aliphatic spacer is a branched or straight, saturated or unsaturated aliphatic chain of 1 to 4 carbon atoms; branched or straight, saturated or unsaturated aliphatic chains of 1 to 7 carbon atoms; and
- $R_3$ is an atom or a group defined as $R_4$, or $R_3$ together with $R_4$ forms a homocyclic or heterocyclic radical;

and/or being a pharmaceutically acceptable addition salt thereof and/or a stereoisomer thereof and/or a mono- or a di-*N*-oxide thereof and/or a solvate and/or a dihydro- or tetrahydropteridine derivative thereof.

**2.** Use according to claim 1, wherein $R_4$ is hydrogen or methoxy.

**3.** Use according to claim 1, wherein $R_3$ is 3-thienyl, 2-thienyl or a phenyl group with one or more substituents.

**4.** Use according to claim 1, wherein:

- X is NZ,
- Z is selected from the group consisting of hydrogen, methyl, ethyl, n-propyl and benzyl, and
- $R_1$ is selected from the group consisting of methyl, ethyl, n-propyl and benzyl.

**5.** Use according to claim 1, wherein X is NZ and wherein the group NZ together with $R_1$ is selected from the group consisting of tetrahydropyridinyl, hydroxylamino, morpholinyl, piperidinyl, piperazinyl, 1,2,4-triazolyl and N-methylpiperazinyl.

**6.** Use of a pteridine derivative for the manufacture of a medicament for the prevention or treatment of a disorder in a mammal, the said disorder being selected from the group consisting of toxic effects of TNF-α, alcohol-induced hepatitis, and cachexia, the said pteridine derivative having the general formula (I):

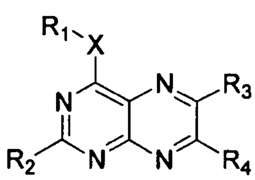

wherein X represents an oxygen atom or a group with the formula $S(O)_m$ wherein m is an integer from 0 to 2, or a group with the formula NZ and wherein:

- $R_1$ is selected from the group consisting of methyl, ethyl, isopropyl and pentyl;
- Z is a group independently defined as $R_1$ or Z is hydrogen or the group NZ together with $R_1$ is either hydroxylamino or an optionally substituted heterocyclic group containing at least one nitrogen atom;
- $R_2$ is selected from the group consisting of amino; acylamino;
- $R_4$ is an atom or a group selected from the group consisting of hydrogen; halogen; $C_{1-7}$ alkyl; $C_{2-7}$ alkenyl; $C_{2-7}$ alkynyl; halo $C_{1-7}$ alkyl; carboxy $C_{1-7}$ alkyl; acetoxy $C_{1-7}$ alkyl; carboxyaryl; $C_{1-7}$ alkoxy; $C_{3-10}$ cycloalkoxy; aryloxy; arylalkyloxy; oxyheterocyclic; heterocyclic-substituted alkyloxy; thio $C_{1-7}$ alkyl; thio $C_{3-10}$ cycloalkyl; thioaryl; thioheterocyclic; arylalkylthio; heterocyclic-substituted alkylthio; amino; hydroxylamino; mercapto-amino; acylamino; thioacylamino; alkoxyamino; thioalkylamino; acetal; thioacetal; carboxylic acid; carboxylic acid esters, thioesters, halides, anhydrides, amides and thioamides; thiocarboxylic acid; thiocarboxylic acid esters, thioesters, halides, anhydrides, amides and thioamides; hydroxyl; sulfhydryl; nitro; cyano; carbamoyl; thiocarbamoyl, ureido; thio-ureido; alkylamino; cycloalkylamino; alkenylamino; cycloalkenylamino; alkynyl-amino; arylamino; arylalkylamino; hydroxyalkylamino; mercapto-alkylamino; heterocyclic amino; heterocyclic-substituted alkylamino; oximino; alkyloximino; hydrazino; alkylhydrazino; phenylhydrazino; cysteinyl acid, esters, thioesters, halides, anhydrides, amides and thioamides thereof; aryl groups optionally substituted with one or more substituents selected from the group consisting of halogen, $C_{1-7}$ alkyl, $C_{1-7}$ alkoxy; optionally substituted heterocyclic radicals; aromatic or heterocyclic substituents substituted with an aliphatic spacer between the pteridine ring and the aromatic or heterocyclic substituent, whereby said aliphatic spacer is a branched or straight, saturated or unsaturated aliphatic chain of 1 to 4 carbon atoms; branched or straight, saturated or unsaturated aliphatic chains of 1 to 7 carbon atoms; and

- $R_3$ is a phenyl group with one or more substituents each independently selected from the group consisting of fluoro, methoxy, ethoxy, trifluoromethyl, dimethylamino, chloro, cyano, methyl, ethyl, carboxymethyl, methylthio, dimethylcarboxamido, diethylcarboxamido and methylcarboxylate,

and/or being a pharmaceutically acceptable addition salt thereof and/or a stereoisomer thereof and/or a mono- or a di-*N*-oxide thereof and/or a solvate and/or a dihydro- or tetrahydropteridine derivative thereof.

**7.** Use of a pteridine derivative for the manufacture of a medicament for the prevention or treatment of a disorder in a mammal, the said disorder being selected from the group consisting of toxic effects of TNF-α, alcohol-induced hepatitis, and cachexia, wherein the pteridine derivative is a compound selected from the group consisting of:

- 2-amino-4-ethoxypteridine
- 2-amino-4-ethoxy-6-chloro-pteridine
- 2-amino-4-ethoxy-6-(4-methoxyphenyl)-pteridine
- 2-amino-4-ethoxy-6-(2-methoxyphenyl)-pteridine
- 2-amino-4-ethoxy-6-(3-methoxyphenyl)-pteridine
- 2-amino-4-ethoxy-6-(3,4-difluorophenyl)-pteridine
- 2-amino-4-ethoxy-6-(4-dimethylaminophenyl)-pteridine
- 2-amino-4-ethoxy-6-(4-trifluoromethylphenyl)-pteridine
- 2-amino-4-ethoxy-6-(2-thienyl)-pteridine
- 2-amino-4-ethoxy-6-(3-thienyl)-pteridine
- 2-amino-4-ethoxy-6-(3,4-dichlorophenyl)-pteridine
- 2-amino-4-ethoxy-6-(4-cyanophenyl)-pteridine
- 2-amino-4-ethoxy-6-(4-ethoxyphenyl)-pteridine
- 2-amino-4-ethoxy-6-(4-fluorophenyl)-pteridine
- 2-amino-4-ethoxy-6-(4-ethylphenyl)-pteridine
- 2-amino-4-ethoxy-6-(4-acetylphenyl)-pteridine
- 2-amino-4-ethoxy-6-(3-fluoro-4-methylphenyl)-pteridine
- 2-amino-4-ethoxy-6-(4-methylthiophenyl)-pteridine
- 2-amino-4-ethoxy-6-(4-N,N-dimethylbenzamido)-pteridine
- 2-amino-4-isopropoxypteridine
- 2-amino-4-isopropoxy-6-chloropteridine
- 2-amino-4-isopropoxy-6-(3-methyl-4-methoxyphenyl)-pteridine
- 2-amino-4-isopropoxy-6-(3,4-dimethylphenyl)-pteridine
- 2-amino-4-isopropoxy-6-(3-chloro-4-trifluoromethylphenyl)-pteridine
- 2-amino-4-isopropoxy-6-(3-chloro-4-fluorophenyl)-pteridine
- 2-amino-4-isopropoxy-6-(4-N,N-diethylbenzamido)-pteridine
- 2-amino-4-isopropoxy-6-(4-trifluoromethylphenyl)-pteridine
- 2-amino-4-isopropoxy-6-(3,4-difluorophenyl)-pteridine
- 2-amino-4-isopropoxy-6-(4-methoxyphenyl)-pteridine
- 2-amino-4-isopropoxy-6-(4-ethoxyphenyl)-pteridine
- 2-amino-4-isopropoxy-6-(4-N,N-dimethylbenzamido)-pteridine
- 2-amino-4-isopropoxy-6-(3-thienyl)-pteridine
- 2-amino-4-isopropoxy-6-(4-cyanophenyl)-pteridine
- 2-amino-4-isopropoxy-6-(4-benzoic acid methyl ester)-pteridine
- 2-amino-4-isopropoxy-6-(4-acetylphenyl)-pteridine

- 2-amino-4-isopropoxy-6-(3,4-dimethoxyphenyl)-pteridine
- 2-amino-4-ethylthio-6-(3,4-dimethoxyphenyl)-pteridine
- 2-amino-4-isopropylthio-6-(3,4-dimethoxyphenyl)-pteridine
- 2-amino-4-pentoxy-6-styrylpteridine,
- 2-amino-4-n-pentoxy-6-(1,2-dibromo-2-phenylethyl)-pteridine,
- 2-amino-4-methoxy-6-styryl-7-methoxypteridine,
- 2-amino-4-dimethylamino-6-phenylpteridine,
- 2-amino-4-dimethylamino-6-(4-tolyl)pteridine,
- 2-amino-4-dimethylamino-6-(4-methoxyphenyl)pteridine,
- 2-amino-4-diethylamino-6-phenylpteridine,
- 2-amino-4-diethylamino-6-(4-chlorophenyl)pteridine,
- 2-amino-4-diethylamino-6-(4-methoxyphenyl)pteridine,
- 2-amino-4-diethylamino-6-(3,4-dimethoxyphenyl)pteridine,
- 2-amino-4-dipropylamino-6-phenylpteridine,
- 2-amino-4-dipropylamino-6-(4-chlorophenyl)pteridine,
- 2-amino-4-dipropylamino-6-(4-methoxyphenyl)pteridine,
- 2-amino-4-dipropylamino-6-(3,4-dimethoxyphenyl)pteridine,
- 2-amino-4-morpholino-6-phenylpteridine.
- 2-amino-4-morpholino-6-(4-chlorophenyl)pteridine,
- 2-amino-4-morpholino-6-(4-methoxyphenyl)pteridine,
- 2-amino-4-morpholino-6-(3,4-dimethoxyphenyl)pteridine,
- 2-amino-4-piperidino-6-phenylpteridine,
- 2-amino-4-piperidino-6-(4-chlorophenyl) pteridine,
- 2-amino-4-piperidino-6-(4-methoxyphenyl)pteridine,
- 2-amino-4-piperidino-6-(3,4-dimethoxyphenyl)pteridine,
- 2-amino-4-N-methylpiperazino-6-phenylpteridine,
- 2-amino-4-N-methylpiperazino-6-(4-chlorophenyl)pteridine,
- 2-amino-4-N-methylpiperazino-6-(4-methoxyphenyl)pteridine,
- 2-amino-4-methylpiperazino-6-(3,4-dimethoxyphenyl)pteridine,
- 2-amino-4-pyrrolidino-6-(4-methoxyphenyl)pteridine,
- 2-amino-4-piperazino-6-phenylpteridine,
- 2-amino-4-piperazino-6-(4-chlorophenyl)pteridine,
- 2-amino-4-piperazino-6-(4-methoxyphenyl)pteridine,
- 2-amino-4-piperazino-6-(3,4-dimethoxyphenyl)pteridine,
- 2-amino-4-morpholino-6-(3,4,5-trimethoxyphenyl)pteridine,
- 2-amino-4-morpholino-6-(3,4-formylidene-3,4-dihydroxyphenyl)pteridine,
- 2-amino-4-dimethylamino-6-(3,4-formylidene-3,4-dihydroxyphenyl) pteridine,
- 2-amino-4-pyrrolidino-6-(3,4,dimethoxyphenyl)pteridine,
- 2-amino-4-dimethylamino-6-(3,4-dimethoxyphenyl)pteridine,
- 2-amino-4-dimethylamino-6-methylpteridine,
- 2-amino-4-ethoxy-6-phenylpteridine,
- 2-amino-4-propylamino-6-phenylpteridine,
- 2-amino-4-propylamino-6-(3,4-dimethoxyphenyl)pteridine,
- 2-acetamido-4-isopropoxy-6-(3,4-dimethoxyphenyl)pteridine,
- 2-amino-4-ethoxy-6-(3,4-dimethoxyphenyl)pteridine,
- 2-amino-4-(1,2,3,6-tetrahydropyridinyl)-6-(3,4-dimethoxyphenyl)pteridine,
- 2-amino-4-ethoxy-pteridine,
- 2-amino-4-ethoxypteridine-$N^8$-oxide,
- 2-amino-4-isopropoxypteridine-$N^8$-oxide,
- 2-amino-6-chloro-4-ethoxypteridine,
- 2-amino-6-chloro-4-isopropoxypteridine,
- 2-amino-6-(p-methoxyphenyl)-4-ethoxy-pteridine;
- 2-amino-6-(o-methoxyphenyl)-4-ethoxy-pteridine;
- 2-amino-6-(m-methoxyphenyl)-4-ethoxy-pteridine;
- 2-amino-6-(3,4-difluorophenyl)-4-ethoxy-pteridine;
- 2-amino-6-(p-dimethylaminophenyl)-4-ethoxy-pteridine;
- 2-amino-6-(p-trifluoromethylphenyl)-4-ethoxy-pteridine;
- 2-amino-6-(2-thienyl)-4-ethoxy-pteridine;

- 2-amino-6-(3-thienyl)-4-ethoxy-pteridine;
- 2-amino-6-(3,4-dichlorophenyl)-4-ethoxy-pteridine;
- 2-amino-6-(p-cyanophenyl)-4-ethoxy-pteridine;
- 2-amino-6-(p-ethoxyphenyl)-4-ethoxy-pteridine;
- 2-amino-6-(p-fluorophenyl)-4-ethoxy-pteridine;
- 2-amino-6-(p-ethylphenyl)-4-ethoxy-pteridine;
- 2-amino-6-(p-acetylphenyl)-4-ethoxy-pteridine;
- 2-amino-6-(3-methyl-4-fluorophenyl)-4-ethoxy-pteridine;
- 2-amino-6-(p-thiomethylphenyl)-4-ethoxy-pteridine;
- 2-amino-6-(p-N,N-dimethylbenzamido)-4-ethoxy-pteridine;
- 2-amino-6-(3,4-dimethoxyphenyl)-4-ethoxy-pteridine,
- 2-amino-6-(3-methyl-4-methoxyphenyl)-4-isopropoxypteridine;
- 2-amino-6-(3,4-dimethylphenyl)-4-isopropoxypteridine;
- 2-amino-6-(3-chloro-4-trifluoromethylphenyl)-4-isopropoxypteridine;
- 2-amino-6-(3-chloro-4-fluorophenyl)-4-isopropoxypteridine;
- 2-amino-6-(p-N,N-diethylbenzamido)-4-isopropoxypteridine;
- 2-amino-6-(p-trifluoromethylphenyl)-4-isopropoxypteridine;
- 2-amino-6-(3,4-difluorophenyl)-4-isopropoxypteridine;
- 2-amino-6-(p-methoxyphenyl)-4-isopropoxypteridine;
- 2-amino-6-(p-ethoxyphenyl)-4-isopropoxypteridine;
- 2-amino-6-(p-dimethylbenzamido)-4-isopropoxypteridine;
- 2-amino-6-(3-thienyl)-4-isopropoxypteridine;
- 2-amino-6-(p-cyanophenyl)-4-isopropoxypteridine;
- 2-amino-6-(p-benzoic acid methyl ester)-4-isopropoxypteridine;
- 2-amino-6-(p-acetylphenyl)-4-isopropoxypteridine;
- 2-amino-6-(3,4-dimethoxyphenyl)-4-isopropoxypteridine,
- 2-amino-4-mercaptoethyl-6-(3,4-dimethoxyphenyl) pteridine;
- 2-amino-4-mercaptoisopropyl-6-(3,4-dimethoxyphenyl) pteridine,
- 2-acetylamino-4-(1,2,4-triazolyl)-6-(p-methoxyphenyl) pteridine,
- 2-acetylamino-4-(1,2,4-triazolyl)-7-(p-methoxyphenyl)pteridine,
- 2-amino-4-isopropoxy-7-(p-methoxyphenyl) pteridine,
- 2-amino-4-isopropoxy-7-(3,4-dimethoxyphenyl) pteridine,
- 2-amino-4-ethoxy-7-(3,4-dimethoxyphenyl) pteridine,
- 2-amino-4-methoxy-7-(3,4-dimethoxyphenyl) pteridine,
- 2-amino-4-(1,2,3,6-tetrahydropyridinyl)-6-(3,4-dimethoxyphenyl)pteridine,
- 2-amino-4-(diethanolamino)-6-[[3,4-(dimethoxyphenyl)]pteridine,
- 2-amino-4-thiomorpholino-6-[[3,4-(dimethoxyphenyl)]pteridine,
- 2-amino-4-morpholino-6-(4-acetanilide) pteridine,
- 2-amino-4-morpholino-6-(3-acetanilide) pteridine,
- 2-amino-4-morpholino-6-(4-aminophenyl) pteridine,
- 2-amino-4-morpholino-6-(3-aminophenyl) pteridine,
- 2-amino-4-morpholino-6-(4-benzoylaminophenyl) pteridine;
- 2-amino-4-morpholino-6-(4-phenoxyacetylaminophenyl) pteridine;
- 2-amino-4-morpholino-6-(4-propionylaminophenyl) pteridine;
- 2-amino-4-morpholino-6-(4-furoylaminophenyl) pteridine;
- 2-amino-4-morpholino-6-(4-cyclohexanoylaminophenyl) pteridine;
- 2-amino-4-morpholino-6-[4-(4-chlorobenzoyl)aminophenyl] pteridine;
- 2-amino-4-morpholino-6-(4-benzyloxyacetylaminophenyl) pteridine,
- 2-amino-4-morpholino-6-(4-isonicotinoylaminophenyl) pteridine;
- 2-amino-4-morpholino-6-(4-naphtoylaminophenyl) pteridine;
- 2-amino-4-morpholino-6-(4-methylsulfonylaminophenyl) pteridine;
- 2-amino-4-morpholino-6-(4-ethylsuccinylaminophenyl) pteridine;
- 2-amino-4-morpholino-6-[4-(4-methylbenzoate)aminophenyl) pteridine;
- 2-amino-4-morpholino-6-(3-benzoylaminophenyl) pteridine;
- 2-amino-4-morpholino-6-(3-benzensulfonylaminophenyl) pteridine;
- 2-amino-4-morpholino-6-(3-phenoxyacetylaminophenyl) pteridine;
- 2-amino-4-morpholino-6-(3-isonicotinoylaminophenyl) pteridine;
- 2-amino-4-morpholino-6-(3-cyclohexanoylaminophenyl) pteridine;

- 2-amino-4-morpholino-6-[3-(4-methylbenzoate)aminophenyl] pteridine;
- 2-amino-4-morpholino-6-(3-ethylsuccinylaminophenyl) pteridine;
- 2-amino-4-morpholino-6-(3-ethylmalonylaminophenyl) pteridine;
- 2-amino-4-morpholino-6-(3-benzyloxyacetylaminophenyl) pteridine;
- 2-amino-4-morpholino-6-(3-ethylsulfonylaminophenyl)pteridine,
- 2-amino-4-morpholino-6-[3-Boc-(L)-phenylalanine-aminophenyl] pteridine;
- 2-amino-4-morpholino-6-[3-Boc-(D)-phenylalanine-aminophenyl] pteridine;
- 2-amino-4-morpholino-6-[3-Boc-(L)-tryptophane-aminophenyl] pteridine;
- 2-amino-4-morpholino-6-[3-Boc-(D)-tryptophane-aminophenyl] pteridine,
- 2-amino-4-morpholino-6-(4-hydroxyphenyl) pteridine,
- 2-amino-4-morphofino-6-(4-ethoxyphenyl) pteridine;
- 2-amino-4-morpholino-6-(4-benzyloxyphenyl) pteridine;
- 2-amino-4-morpholino-6-(4-(phenethyloxy)-phenyl) pteridine;
- 2-amino-4-morpholino-6-(4-phenoxy-butyronitrile) pteridine;
- 2-amino-4-morpholino-6-(4-propoxy-phenyl) pteridine;
- 2-amino-4-morpholino-6-(4-phenoxy-butyric acid ethyl ester) pteridine;
- 2-amino-4-morpholino-6-(4-phenoxy-acetic acid ethyl ester) pteridine
- 2-amino-4-morpholino-6-(4-(2-methoxyethoxy)-phenyl) pteridine; and
- 2-amino-4-morpholino-6-(4-butoxy-phenyl)-pteridine.

## Patentansprüche

**1.** Verwendung eines Pteridinderivates zur Herstellung eines Medikaments zur Vorbeugung von oder Behandlung einer Erkrankung in einem Säugetier, wobei die Erkrankung ausgewählt ist aus der Gruppe, bestehend aus toxischen Wirkungen von TNF-α, alkoholinduzierter Hepatitis und Kachexie, wobei das Pteridinderivat die allgemeine Formel (I) besitzt:

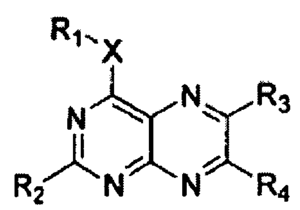

worin X ein Sauerstoffatom oder eine Gruppe mit der Formel $S(O)_m$ darstellt, wobei m eine ganze Zahl von 0 bis 2 ist, oder eine Gruppe mit der Formel NZ und worin:

- $R_1$ ausgewählt ist, bestehend aus Methyl, Ethyl, Isopropyl und Pentyl;
- Z eine Gruppe ist, die unabhängig voneinander definiert ist als $R_1$ oder Z Wasserstoff ist oder die Gruppe NZ zusammen mit $R_1$ entweder Hydroxylamino oder eine wahlweise substituierte heterocyclische Gruppe ist, die mindestens ein Stickstoffatom enthält;
- $R_2$ ausgewählt ist aus der Gruppe, bestehend aus Amino; Acylamino;
- $R_4$ ein Atom oder eine Gruppe ist, ausgewählt aus der Gruppe, bestehend aus Wasserstoff; Halogen; $C_{1-7}$ Alkyl; $C_{2-7}$ Alkenyl; $C_{2-7}$ Alkinyl; Halo $C_{1-7}$ Alkyl; Carboxy $C_{1-7}$ Alkyl; Acetoxy $C_{1-7}$ Alkyl; Carboxyaryl; $C_{1-7}$ Alkoxy; $C_{3-10}$ Cycloalkoxy; Aryloxy; Arylalkyloxy; Oxyheterocyclic; heterocyclisch-substituiertes Alkyloxy; Thio $C_{1-7}$ Alkyl; Thio $C_{3-10}$ Cycloalkyl; Thioaryl; thioheterocyclisch; Arylalkylthio; heterocyclisch-substituertes Alkylthio; Amino; Hydroxylamino; Mercapto-Amino; Acylamino; Thioacylamino; Alkoxyamino; Thioalkylamino; Acetal; Thioacetal; Carbonsäure; Carbonsäure-Estern, Thioestern, Halogeniden, Anhydriden, Amiden und Thioamiden; Thiocarboxylsäure; Thiocarboxylsäure-Estern; Thioestern, Halogeniden, Anhydriden, Amiden und Thioamiden; Hydroxyl; Sulfhydryl; Nitro; Cyano; Carbamoyl; Thiocarbamoyl; Ureido; Thio-Ureido; Alkylamino; Cycloalkylamino; Alkenylamino; Cycloalkenylamino; Alkinyl-Amino; Arylamino; Arylalkylamino; Hydroxyalkylamino; Mercapto-Alkylamino; Heterocyclisches Amino; Heterocyklisch-substituiertes Alkylamino; Oximino; Alkyloximino; Hydrazino; Alkylhydrazino; Phenylhydrazino; Cysteinylsäure, Ester, Thioester, Halogenide, Anhydride, Amide und Thioamide davon; Arylgruppen, die wahlweise mit einem oder mehreren Substituenten substituiert sind, ausgewählt aus der Gruppe bestehend aus Halogen, $C_{1-7}$ Alkyl, $C_{1-7}$ Alkoxy; wahlweise substituierte heterocyclische Rest; aromatische oder heterocyclische Substituenten, substituiert mit aliphatischen Spacern zwischen dem Pteridinring und dem aromatischen oder heterocyclischen Substituienten, wobei deren aliphatischer Spacer eine verzweigte oder geradkettige, gesättigte oder ungesättigte aliphatische Kette mit 1 bis 4 Kohlenstoffatomen ist; eine verzweigte oder geradkettige, gesättigte oder ungesättigte aliphatischen Kette mit 1 bis 7 Kohlenstoffatomen; ist und

- $R_3$ ein Atom oder eine Gruppe ist definiert als $R_4$ oder $R_3$ zusammen mit $R_4$ einen homocyclischen oder heterocyclischen Rest ausbildet;

und/oder ein pharmazeutisch annehmbares Additionssalz davon und/oder Stereoisomer davon und/oder ein mono- oder ein di-N-Oxid davon und/oder ein Solvat und/oder ein Dihydro- oder Tetrahydropteridinderivat davon.

**2.** Verwendung gemäß Anspruch 1, wobei $R_4$ ein Wasserstoff oder Methoxy ist.

**3.** Verwendung gemäß Anspruch 1, wobei $R_3$ 3-Thienyl, 2-Thienyl oder eine Phenylgruppe mit einem oder mehreren Substituienten ist.

**4.** Verwendung gemäß Anspruch 1, wobei:

- X NZ ist,
- Z ausgewählt ist aus der Gruppe, bestehend aus Wasserstoff, Methyl, Ethyl, N-Propyl und Benzyl, und
- $R_1$ ausgewählt ist aus der Gruppe, bestehend aus Methyl, Ethyl, N-Propyl und Benzyl.

**5.** Verwendung gemäß Anspruch 1, wobei X NZ ist und wobei die Gruppe NZ zusammen mit $R_1$ ausgewählt ist aus der Gruppe, bestehend aus Tetrahydropyridinyl, Hydroxylamino, Morpholinyl, Piperidinyl, Piperazinyl, 1,2,4-triazolyl und N-Methylpiperazinyl.

**6.** Verwendung eines Pteridinderivates zur Herstellung eines Medikamentes zur Vorbeugung gegen oder Behandlung von einer Erkrankung eines Säugetieres, wobei die Erkrankung ausgewählt ist aus der Gruppe, bestehend aus toxischen Wirkungen von TNF-$\alpha$, alkoholinduzierter Hepatitis und Kachexie, wobei das Pteridinderivat die allgemeine Formel (I) besitzt:

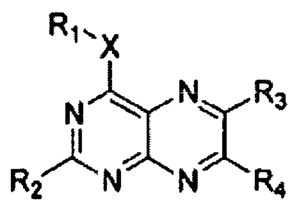

wobei X ein Sauerstoffatom oder eine Gruppe mit der Formel $S(O)_m$ darstellt, wobei $_m$ eine ganze Zahl von 0 bis 2 ist, oder eine Gruppe mit der Formel NZ und wobei:

- $R_1$ ausgewählt ist, bestehend aus Methyl, Ethyl, Isopropyl und Pentyl;
- Z eine Gruppe ist, die unabhängig voneinander definiert ist als $R_1$ oder Z ist Wasserstoff ist oder die Gruppe NZ zusammen mit $R_1$ entweder Hydroxylamino oder eine wahlweise substituierte heterocyclische Gruppe ist, die mindestens ein Stickstoffatom enthält;
- $R_2$ ausgewählt ist aus der Gruppe, bestehend aus Amino; Acylamino;
- $R_4$ ein Atom oder eine Gruppe ist, ausgewählt aus der Gruppe, bestehend aus Wasserstoff; Halogen; $C_{1-7}$ Alkyl; $C_{2-7}$ Alkenyl; $C_{2-7}$ Alkinyl; Halo $C_{1-7}$ Alkyl; Carboxy $C_{1-7}$ Alkyl; Acetoxy $C_{1-7}$ Alkyl; Carboxyaryl; $C_{1-7}$ Alkoxy; $C_{3-10}$ Cycloalkoxy; Aryloxy; Arylalkyloxy; Oxyheterocyclisch; heterocyclisch-substituierte Alkyloxy; Thio $C_{1-7}$ Alkyl; Thio $C_{3-10}$ Cycloalkyl; Thioaryl; thioheterocyclisch; Arylalkylthio; heterocyclisch-substituerte Alkylthio; Amino; Hydroxylamino; Mercapto-Amino; Acylamino; Thioacylamino; Alkoxyamino; Thioalkylamino; Acetal; Thioacetal; Carbonsäuren; Carbonsäure-Estern; Thioestern, Halogeniden; Anhydrides; Amiden und Thioamiden; Thiocarboxylsäure; Thiocarboxylsäure-Estern; Thioestern; Halogeniden; Anhydriden; Amiden und Thioamiden; Hydroxyl; Sulfhydryl; Nitro; Cyano; Carbamoyl; Thiocarbamoyl; Ureido; Thio-Ureido; Alkylamino; Cycloalkylamino; Alkenylamino; Cycloalkenylamino; Alkinyl-Amino; Arylamino; Arylalkylamino; Hydroxyalkylamino; Mercapto-Alkylamino; Heterocyclisches Amino; Heterocyklisch-substituiertes Alkylamino; Oximino; Alkyloximino; Hydrazino; Alkylhydrazino; Phenylhydrazino; Cysteinylsäure, Estern, Thioestern, Halogeniden, Anhydriden, Amiden und Thioamiden davon; Arylgruppen, die wahlweise mit einem oder mehreren Substituenten substituiert sind, ausgewählt aus der Gruppe bestehend aus Halogen, $C_{1-7}$ Alkyl, $C_{1-7}$ Alkoxy; wahlweise substituierte heterocyclische Reste; aromatische oder heterocyclische Substituenten, substituiert mit aliphatischen Spacern zwischen dem Pteridinring und dem aromatischen oder heterocyclischen Substituenten, wobei deren aliphatischer Spacer eine verzweigte oder geradkettige, gesättigte oder ungesättigte aliphatische Kette mit 1 bis 4 Kohlenstoffatomen; verzweigte oder geradkettige, gesättigte oder ungesättigte aliphatische Ketten mit 1 bis 7 Kohlenstoffatomen; ist und

- $R_3$ ist eine Phenylgruppe mit ein oder mehreren Substituenten ist, jeweils unabhängig voneinander ausgewählt aus der Gruppe, bestehend aus Fluor, Methoxy, Ethoxy, Trifluormethyl, Dimethylamino, Chlor, Cyano, Methyl, Ethyl, Carboxymethyl, Methylthio, Dimethylcarboxamido, Diethylcarboxamido und Methylcarboxylat,

und/oder einem pharmazeutisch annehmbaren Additionssalz davon und/oder einem Stereoisomer davon und/oder einem mono- oder einem di-N-Oxid davon und/oder einem Solvat und/oder einem Dihydro- oder Thetrahydropteridinderivat davon.

**7.** Verwendung eines Pteridinderivates zur Herstellung eines Medikamentes zur Vorbeugung gegen oder Behandlung von einer Krankheit eines Säugetiers, wobei die Krankheit ausgewählt ist aus der Gruppe, bestehend aus den toxischen Wirkungen von TNF-$\alpha$, alkoholinduzierter Hepatitis und Kachexie, wobei das Pteridinderivat eine Verbindung ist, ausgewählt aus der Gruppe, bestehend aus:

- 2-Amino-4-ethoxypteridin,
- 2-Amino-4-ethoxy-6-chlor-pteridin,
- 2-Amino-4-ethoxy-6-(4-methoxyphenyl)-pteridin,
- 2-Amino-4-ethoxy-6-(2-methoxyphenyl)-pteridin,
- 2-Amino-4-ethoxy-6-(3-methoxyphenyl)-pteridin,
- 2-Amino-4-ethoxy-6-(3,4-difluorophenyl)-pteridin,
- 2-Amino-4-ethoxy-6-(4-dimethylaminophenyl)-pteridin,
- 2-Amino-4-ethoxy-6-(4-trifluoromethylphenyl)-pteridin,
- 2-Amino-4-ethoxy-6-(2-thienyl)-pteridin,
- 2-Amino-4-ethoxy-6-(3-thienyl)- pteridin,
- 2-Amino-4-ethoxy-6-(3,4-dichlorophenyl)-pteridin,
- 2-Amino-4-ethoxy-6-(4-cyanophenyl)-pteridin,
- 2-Amino-4-ethoxy-6-(4-ethoxyphenyl)-pteridin,
- 2-Amino-4-ethoxy-6-(4-fluorophenyl)-pteridin,
- 2-Amino-4-ethoxy-6-(4-ethylphenyl)-pteridin,
- 2-Amino-4-ethoxy-6-(4-acetylphenyl)-pteridin,
- 2-Amino-4-ethoxy-6-(3-fluoro-4-methylphenyl)-pteridin,
- 2-Amino-4-ethoxy-6-(4-methylthiophenyl)-pteridin,
- 2-Amino-4-ethoxy-6-(4-N,N-dimethylbenzamido)-pteridin,
- 2-Amino-4-isopropoxypteridin,
- 2-Amino-4-isopropoxy-6-Chlorpteridin,
- 2-Amino-4-isopropoxy-6-(3-methyl-4-methoxyphenyl)-pteridin,
- 2-Amino-4-isopropoxy-6-(3,4-dimethylphenyl)-pteridin,
- 2-Amino-4-isopropoxy-6-(3-chlor-4-trifluoromethylphenyl)-pteridin,
- 2-Amino-4-isopropoxy-6-(3-chlor-4-fluorophenyl)-pteridin,
- 2-Amino-4-isopropoxy-6-(4-N,N-diethylbenzamido)-pteridin,
- 2-Amino-4-isopropoxy-6-(4-trifluoromethylphenyl)-pteridin,
- 2-Amino-4-isopropoxy-6-(3,4-difluorphenyl)-pteridin,
- 2-Amino-4-isopropoxy-6-(4-methoxyphenyl)-pteridin,
- 2-Amino-4-isopropoxy-6-(4-ethoxyphenyl)-pteridin,
- 2-Amino-4-isopropoxy-6-(4-N,N-dimethylbenzamido)-pteridin,
- 2-Amino-4-isopropoxy-6-(3-thienyl)-pteridin,
- 2-Amino-4-isopropoxy-6-(4-cyanophenyl)-pteridin,
- 2-Amino-4-isopropoxy-6-(4-benzoesäuremethylester)-pteridin,

- 2-Amino-4-isopropoxy-6-(4-acetylphenyl)-pteridin,
- 2-Amino-4-isopropoxy-6-(3,4-dimethoxyphenyl)-pteridin,
- 2-Amino-4-ethylthio-6-(3,4-dimethoxyphenyl)-pteridin,
- 2-Amino-4-isopropylthio-6-(3,4-dimethoxyphenyl)-pteridin,
- 2-Amino-4-pentoxy-6-styrylpteridin,
- 2-Amino-4-n-pentoxy-6-(1,2-dibromo-2-phenylethyl)-pteridin,
- 2-Amino-4-methoxy-6-styryl-7-methoxypteridin,
- 2-Amino-4-dimethylamino-6-phenylpteridin,
- 2-Amino-4-dimethylamino-6-(4-tolyl)pteridin,
- 2-Amino-4-dimethylamino-6-(4-methoxyphenyl)pteridin,
- 2-Amino-4-diethylamino-6-phenylpterdin,
- 2-Amino-4-diethylamino-6-(4-chlorophenyl)pterdin,
- 2-Amino-4-diethylamino-6-(4-methoxyphenyl)pteridin,
- 2-Amino-4-diethylamino-6-(3,4-dimethoxyphenyl)pteridin,
- 2-Amino-4-dipropylamino-6-phenylpterdin,
- 2-Amino-4-dipropylamino-6-(4-chlorphenyl)pteridin,
- 2-Amino-4-dipropylamino-6-(4-methoxyphenyl)pteridin,
- 2-Amino-4-dipropylamino-6-(3,4-dimethoxyphenyl)pteridin,
- 2-Amino-4-morpholino-6-phenylpterdin,
- 2-Amino-4-morpholino-6-(4-chlorphenyl)pteridin,
- 2-Amino-4-morpholino-6-(4-methoxyphenyl)pteridin,
- 2-Amino-4-morpholino-6-(3,4-dimethoxyphenyl)pteridin,
- 2-Amino-4-piperidino-6-phenylpterdin,
- 2-Amino-4-piperidino-6-(4-chlorphenyl)pteridin,
- 2-Amino-4-piperidino-6-(4-methoxyphenyl)pteridin,
- 2-Amino-4-piperidino-6-(3,4-dimethoxyphenyl)pteridin,
- 2-Amino-4-N-methylpiperazino-6-phenylpterdin,
- 2-Amino-4-N-methylpiperazino-6-(4-chlorphenyl)pteridin,
- 2-Amino-4-N-methylpiperazino-6-(4-methoxyphenyl)pteridin,
- 2-Amino-4-methylpiperazino-6-(3,4-dimethoxyphenyl)pteridin,
- 2-Amino-4-pyrrolidono-6-(4-methoxyphenyl)pteridin,
- 2-Amino-4-piperazino-6-phenylperdin,
- 2-Amino-4-piperazino-6-(4-chlorphenyl)pteridin,
- 2-Amino-4-piperazino-6-(4-methoxyphenyl)pteridin,
- 2-Amino-4-piperazino-6-(3,4-dimethoxyphenyl)pteridin,
- 2-Amino-4-morpholino-6-(3,4,5-trimethoxyphenyl)pteridin,
- 2-Amino-4-morpholino-6-(3,4-formyliden-3,4-dihydroxyphenyl)pteridin,
- 2-Amino-4-dimethylamino-6-(3,4-formyliden-3,4-dihydroxyphenyl)pteridin,
- 2-Amino-4-pyrrolidino-6-(3,4-dimethoxyphenyl)pteridin,
- 2-Amino-4-dimethylamino-6-(3,4-dimethoxyphenyl)pteridin,
- 2-Amino-4-dimethylamino-6-methylpteridin,
- 2-Amino-4-ethoxy-6-phenylpteridin,
- 2-Amino-4-propylamino-6-phenylpteridin,
- 2-Amino-4-propylamino-6-(3,4-dimethoxyphenyl)pteridin,
- 2-Acetamino-4-isopropoxy-6-(3,4-dimethoxyphenyl)pteridin,
- 2-Amino-4-ethoxy-(3,4-dimethoxyphenyl)pteridin,
- 2-Amino-4-(1,2,3,6-thetrahydropyridinyl)-6-(3,4-dimethoxyphenyl)pteridin,
- 2-Amino-4-ethoxy-pteridin,
- 2-Amino-4-ethoxypteridin-$N^8$-oxid,
- 2-Amino-4-isopropoxypteridin-$N^8$-oxid,
- 2-Amino-6-chlor-4-ethoxypteridin,
- 2-Amino-6-chlor-4-isopropoxypteridin,
- 2-Amino-6-(p-methoxyphenyl)-4-ethoxy-pteridin,
- 2-Amino-6-(o-methoxyphenyl)-4-ethoxy-pteridin,
- 2-Amino-6-(m-methoxyphenyl)-4-ethoxy-pteridin,
- 2-Amino-6-(3,4-difluorphenyl)-4-ethoxy-pteridin,
- 2-Amino-6-(p-dimethylaminophenyl)-4-ethoxy-pteridin,
- 2-Amino-6-(p-trifluormethylphenyl)-4-ethoxy-pteridin,

- 2-Amino-6-(2-thienyl)-4-ethoxy-pteridin,
- 2-Amino-6-(3-thienyl)-4-ethoxy-pteridin,
- 2-Amino-6-(3,4-dichlorphenyl)-4-ethoxy-pteridin,
- 2-Amino-6-(p-cyanophenyl)-4-ethoxy-pteridin,
- 2-Amino-6-(p-ethoxyphenyl)-4-ethoxy-pteridin,
- 2-Amino-6-(p-fluorphenyl)-4-ethoxy-pteridin,
- 2-Amino-6-(p-ethylphenyl)-4-ethoxy-pteridin,
- 2-Amino-6-(p-acetylphenyl)-4-ethoxy-pteridin,
- 2-Amino-6-(3-methyl-4-fluorphenyl)-4-ethoxy-pteridin,
- 2-Amino-6-(p-thiomethylphenyl)-4-ethoxy-pteridin,
- 2-Amino-6-(p-N,N-dimethylbenzamino)-4-ethoxy-pteridin,
- 2-Amino-6-(3,4-dimethoxyphenyl)-4-ethoxy-pteridin,
- 2-Amino-6-(3-methyl-4-methoxyphenyl)-4-isopropoxypteridin,
- 2-Amino-6-(3,4-dimethylphenyl)-4-isopropoxypteridin,
- 2-Amino-6-(3-chlor-4-trifluormethylphenyl)-4-isopropoxypteridin,
- 2-Amino-6-(3-chlor-4-fluorphenyl)-4-isopropoxypteridin,
- 2-Amino-6-(p-N,N-diethylbenzamino)-4-isopropoxypteridin,
- 2-Amino-6-(p-trifluormethylphenyl)-4-isopropoxypteridin,
- 2-Amino-6-(3,4-difluorphenyl)-4-isopropoxypteridin,
- 2-Amino-6-(p-methoxyphenyl)-4-isopropoxypteridin,
- 2-Amino-6-(p-ethoxyphenyl)-4-isopropoxypteridin,
- 2-Amino-6-(p-dimethylbenzamido)-4-isopropoxypteridin,
- 2-Amino-6-(3-thienyl)-4-isopropoxypteridin,
- 2-Amino-6-(p-cyanophenyl)-4-isopropoxypteridin,
- 2-Amino-6-(p-benzoesäure methyl ester)-4-isopropoxypteridin,
- 2-Amino-6-(p-acetylphenyl)-4-isopropoxypteridin,
- 2-Amino-6-(3,4-dimethoxyphenyl)-4-isopropoxypteridin,
- 2-Amino-4-mercaptoethyl-6-(3,4-dimethoxyphenyl)pteridin,
- 2-Amino-4-mercaptoisopropyl-6-(3,4-dimethoxyphenyl)pteridin,
- 2-Acetylamino-4-(1,2,4-triazolyl)-6-(p-methoxyphenyl)pteridin,
- 2-Acetylamino-4-(1,2,4-triazolyl)-7-(p-methoxyphenyl)pteridin,
- 2-Amino-4-isopropoxy-7-(p-methoxyphenyl)pteridin,
- 2-Amino-4-isopropoxy-7-(3,4-dimethoxyphenyl)pteridin,
- 2-Amino-4-ethoxy-7-(3,4-dimethoxyphenyl)pteridin,
- 2-Amino-4-methoxy-7-(3,4-dimethoxyphenyl)pteridin,
- 2-Amino-4-(1,2,3,6-tetrahydropyridinyl)-6-(3,4-dimethoxyphenyl)pteridin,
- 2-Amino-4-(diethanolamino)-6-[[3,4-(dimethoxyphenyl)]pteridin,
- 2-Amino-4-thiomorpholino-6-[[3,4-(dimethoxyphenyl)]pteridin,
- 2-Amino-4-morpholino-6-(4-acetanilid)pteridin,
- 2-Amino-4-morpholino-6-(3-acetanilid)pteridin,
- 2-Amino-4-morpholino-6-(4-aminophenyl)pteridin,
- 2-Amino-4-morpholino-6-(3-aminophenyl)pteridin,
- 2-Amino-4-morpholino-6-(4-benzoylaminophenyl)pteridin,
- 2-Amino-4-morpholino-6-(4-phenoxyacetylaminophenyl)pteridin,
- 2-Amino-4-morpholino-6-(4-propionylaminophenyl)pteridin,
- 2-Amino-4-morpholino-6-(4-furoylaminophenyl)pteridin,
- 2-Amino-4-morpholino-6-(4-cyclohexanoylaminophenyl)pteridin,
- 2-Amino-4-morpholino-6-[4-(4-chlorbenzoyl)aminophenyl]pteridin,
- 2-Amino-4-morpholino-6-(4-benzyloxyacetylaminophenyl)pteridin,
- 2-Amino-4-morpholino-6-(4-isonicotinoylaminophenyl)pteridin,
- 2-Amino-4-morpholino-6-(4-naphtoylaminophenyl)pteridin,
- 2-Amino-4-morpholino-6-(4-methylsulfonylaiminophenyl)pteridin,
- 2-Amino-4-morpholino-6-(4-ethylsuccinylaminophenyl)pteridin,
- 2-Amino-4-morpholino-6-[4-/4-methylbenzoate)aminophenyl]pteridin,
- 2-Amino-4-morpholino-6-(3-benzoylaminophenyl)pteridin,
- 2-Amino-4-morpholino-6-(3-benzolsulfonylaminophenyl)pteridin,
- 2-Amino-4-morpholino-6-(3-phenoxyacetylaminophenyl)pteridin,
- 2-Amino-4-morpholino-6-(3-isonicotinoylaminophenyl)pteridin,

- 2-Amino-4-morpholino-6-(3-cyclohexanoylaminophenyl)pteridin,
- 2-Amino-4-morpholino-6-[3-(4-cyclohexanoylaminophenyl]pteridin,
- 2-Amino-4-morpholino-6-(3-ethylsuccinylaminophenyl)pteridin,
- 2-Amino-4-morpholino-6-(3-ethylmalonylaminophenyl)pteridin,
- 2-Amino-4-morpholino-6-(3-benzyloxyacetylaminophenyl)pteridin,
- 2-Amino-4-morpholino-6-(3-ethylsulfonylaminophenyl)pteridin,
- 2-Amino-4-morpholino-6-[3-Boc-(L)-phenylalanin-aminophenyl]pteridin,
- 2-Amino-4-morpholino-6-[3-Boc-(D)-phenylalanin-aminophenyl]pteridin,
- 2-Amino-4-morpholino-6-[3-Boc-(L)-tryptophan-aminophenyl]pteridin,
- 2-Amino-4-morpholino-6-[3-Boc-(D)-tryptophan-aminophenyl]pteridin,
- 2-Amino-4-morpholino-6-(4-hydroxyphenyl)pteridin,
- 2-Amino-4-morpholino-6-(4-ethoxyphenyl)pteridin,
- 2-Amino-4-morpholino-6-(4-benzyloxyphenyl)pteridin,
- 2-Amino-4-morpholino-6-(4-(phenethyloxy)-phenyl)pteridin,
- 2-Amino-4-morpholino-6-(4-phenoxy-butyronitril)pteridin,
- 2-Amino-4-morpholino-6-(4-propoxy-phenyl)pteridin,
- 2-Amino-4-morpholino-6-(4-phenoxy-buttersäureethylester)pteridin,
- 2-Amino-4-morpholino-6-(4-phenoxy-essigsäureethylester)pteridin,
- 2-Amino-4-morpholino-6-(4-(2-methoxyethoxy)-phenyl)pteridin, und
- 2-Amino-4-morpholino-6-(4-butoxy-phenyl)pteridin.

**Revendications**

**1.** Utilisation d'un dérivé de la ptéridine pour la fabrication d'un médicament pour la prévention ou le traitement d'un trouble chez les mammifères, ledit trouble étant choisi parmi le groupe constitué par les effets toxiques de la TNF-α, l'hépatite induite par l'alcool et la cachexie,
ledit dérivé de la ptéridine ayant la formule générale (I) :

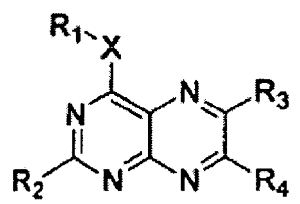

dans laquelle X représente un atome d'oxygène ou un groupe avec la formule $S(O)_m$ dans laquelle m est un entier de 0 à 2, ou un groupe avec la formule NZ et dans laquelle :

- $R_1$ est choisi parmi le groupe constitué par le groupe méthyle, le groupe éthyle, le groupe isopropyle et le groupe pentyle ;
- Z est un groupe défini de manière indépendante tel que $R_1$ ou Z est un groupe hydrogène ou le groupe NZ avec $R_1$ est soit un groupe hydroxylamino soit un groupe hétérocyclique substitué de manière facultative contenant au moins un atome d'azote ;
- $R_2$ est choisi parmi le groupe constitué par un groupe amino ; un groupe acylamino ;
- $R_4$ est un atome ou un groupe choisi parmi le groupe constitué par un atome d'hydrogène ; un atome d'halogène ; un groupe alkyle $C_{1-7}$ ; un groupe alcényle $C_{2-7}$ ; un groupe alcynyle $C_{2-7}$; un groupe haloalkyle $C_{1-7}$ ; un groupe carboxyalkyle $C_{1-7}$ ; un groupe acétoxyalkyle $C_{1-7}$ ; un groupe carboxyaryle ; un groupe alcoxy $C_{1-7}$ ; un groupe cycloalcoxy $C_{3-10}$ ; un groupe aryloxy ; un arylalkyloxy ; un groupe oxy hétérocyclique ; un groupe alkyloxy substitué par un groupe hétérocyclique ; un groupe thioalkyle $C_{1-7}$ ; un groupe thiocycloalkyle $C_{3-10}$ ; un groupe thioaryle ; un groupe thio hétérocyclique ; un groupe arylalkylthio ; un groupe alkylthio substitué par un groupe hétérocyclique ; un groupe amino ; un groupe hydroxylamino ; un groupe mercaptoamino ; un groupe acylamino ; un groupe thioacylamino ; un groupe alcoxyamino ; un groupe thioalkylamino ; un groupe acétal ; un groupe thioacétal ; des groupes esters, thioesters, halides, anhydrides, amides et thioamides d'acides carboxyliques ; un groupe hydroxyle ; un groupe sulfhydryle ; un groupe nitro ; un groupe cyano ; un groupe carbamoyle ; un groupe thiocarbamoyle ; un groupe uréido ; un groupe thiouréido ; un groupe alkylamino ; un

groupe cycloalkylamino ; un groupe alcénylamino ; un groupe cycloalcénylamino ; un groupe alcynylamino ; un groupe arylamino ; un groupe aryl-alkylamino ; un groupe hydroxyalkylamino ; un groupe mercaptoalkylamino et un groupe amino hétérocyclique ; un groupe alkylamino substitué par un groupe hétérocyclique ; un groupe oximino ; un groupe alkyloximino ; un groupe hydrazino ; un groupe alkylhydrazino ; un groupe phénylhydrazino ; un groupe acide cystéinyle, les groupes esters, thioesters, halides, anhydrides, amides et thioamides de celui-ci ; les groupes aryles substitués de manière facultative par un ou plusieurs substituants choisis parmi le groupe constitué par un groupe halogène, un groupe alkyle $C_{1-7}$, un groupe alcoxy $C_{1-7}$ ; des radicaux hétérocycliques substitués de manière facultative ; des substituants aromatiques ou hétérocycliques substitués par un espaceur aliphatique entre le cycle ptéridine et le substituant aromatique ou hétérocyclique, dans lesquels ledit espaceur aliphatique est une chaîne aliphatique de 1 à 4 atomes de carbone ramifiée ou linéaire, saturée ou insaturée ; des chaînes aliphatiques de 1 à 7 atomes de carbone ramifiées ou linéaires, saturées ou insaturées ; et

- $R_3$ est un atome ou un groupe défini tel que $R_4$ ou $R_3$ avec $R_4$ forment un radical homocycle ou hétérocycle ;

et/ou étant un sel d'addition de ceux-ci et/ou un stéréoisomère de ceux-ci et/ou un mono- ou un di-N-oxyde de ceux-ci et/ou un solvate et/ou un dérivé dihydro- ou tétrahydroptéridine de ceux-ci acceptables d'un point de vue pharmaceutique.

**2.** Utilisation selon la revendication 1, dans laquelle $R_4$ est un groupe hydrogène ou un groupe méthoxy.

**3.** Utilisation selon la revendication 1, dans laquelle $R_3$ est un groupe 3-thiényle, un groupe 2-thiényle ou un groupe phényle avec un ou plusieurs substituants.

**4.** Utilisation selon la revendication 1, dans laquelle :

- X est NZ,
- Z est choisi parmi le groupe constitué par un groupe hydrogène, un groupe méthyle, un groupe éthyle, un groupe n-propyle et un groupe benzyle et
- $R_1$ est choisi parmi le groupe constitué par un groupe méthyle, un groupe éthyle, un groupe n-propyle et un groupe benzyle.

**5.** Utilisation selon la revendication 1, dans laquelle X est NZ et dans laquelle le groupe NZ avec $R_1$ est choisi parmi le groupe constitué par un groupe tétrahydropyridinyle, un groupe hydroxylamino, un groupe morpholinyle, un groupe pipéridinyle, un groupe pipérazinyle, un groupe 1,2,4-triazolyle et un groupe N-méthylpipérazinyle.

**6.** Utilisation d'un dérivé de la ptéridine pour la fabrication d'un médicament pour la prévention ou le traitement d'un trouble chez les mammifères, ledit trouble étant choisi parmi le groupe constitué par les effets toxiques de la TNF-α, l'hépatite induite par l'alcool et la cachexie,
ledit dérivé de la ptéridine ayant la formule générale (I) :

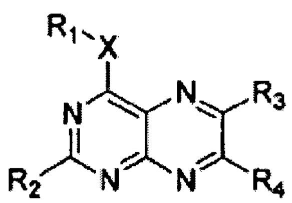

dans laquelle X représente un atome d'oxygène ou un groupe avec la formule $S(O)_m$ dans laquelle m est un entier de 0 à 2, ou un groupe avec la formule NZ et dans laquelle .

- $R_1$ est choisi parmi le groupe constitué par un groupe méthyle, un groupe éthyle, un groupe isopropyle et un groupe pentyle ;
- Z est un groupe défini de manière indépendante tel que $R_1$ ou Z est un groupe hydrogène ou le groupe NZ avec $R_1$ est soit un groupe hydroxylamino soit un groupe hétérocyclique substitué de manière facultative contenant au moins un atome d'azote ;
- $R_2$ est choisi parmi le groupe constitué par un groupe amino ; un groupe acylamino ;

- $R_4$ est un atome ou un groupe choisi parmi le groupe constitué par un atome d'hydrogène ; un atome d'halogène ; un groupe alkyle $C_{1-7}$ ; un groupe alcényle $C_{2-7}$ ; un groupe alcynyle $C_{2-7}$ un groupe haloalkyle $C_{1-7}$ ; un groupe carboxyalkyle $C_{1-7}$ ; un groupe acétoxyalkyle $C_{1-7}$ ; un groupe carboxyaryle ; un groupe alcoxy $C_{1-7}$ ; un groupe cycloalcoxy $C_{3-10}$ ; un groupe aryloxy ; un groupe arylalkyloxy ; un groupe oxy hétérocyclique ; un groupe alkyloxy substitué par un groupe hétérocyclique ; un groupe thioalkyle $C_{1-7}$ ; un groupe thiocycloalkyle $C_{3-10}$ ; un groupe thioaryle ; un groupe thio hétérocyclique ; un groupe arylalkylthio ; un groupe alkylthio substitué par un groupe hétérocyclique ; un groupe amino; un groupe hydroxylamino ; un groupe mercaptoamino ; un groupe acylamino ; un groupe thioacylamino, un groupe alcoxyamino, un groupe thioalkylamino ; un groupe acétal ; un groupe thioacétal ; un groupe acide carboxylique ; des groupes esters, thioesters, halides, anhydrides, amides et thioamides d'acides carboxyliques ; un groupe hydroxyle ; un groupe sulfhydryle ; un groupe nitro ; un groupe cyano ; un groupe carbamoyle ; un groupe thiocarbamoyle ; un groupe uréido ; un groupe thiouréido ; un groupe alkylamino ; un groupe cycloalkylamino ; un groupe alcénylamino ; un groupe cycloalcénylamino ; un groupe alcynylamino ; un groupe arylamino ; un groupe arylalkylamino ; un groupe hydroxyalkylamino ; un groupe mercaptoalkylamino ; un groupe amino hétérocyclique ; un groupe alkylamino substitué par un groupe hétérocyclique ; un groupe oximino ; un groupe alkyloximino ; un groupe hydrazino ; un groupe alkylhydrazino ; un groupe phénylhydrazino ; un groupe acide cystéinyle, des groupes esters, thioesters, halides, anhydrides, amides et thioamides de celui-ci ; des groupes aryles substitués de manière facultative par un ou plusieurs substituants choisi parmi le groupe constitué par un groupe halogène, un groupe alkyle $C_{1-7}$, un groupe alcoxy $C_{1-7}$ ; des radicaux hétérocycliques substitués de manière facultative ; des substituants aromatiques ou hétérocycliques substitués par un espaceur aliphatique entre le cycle ptéridine et le substituant aromatique ou hétérocyclique, dans lesquels ledit espaceur aliphatique est une chaîne aliphatique de 1 à 4 atomes de carbone ramifiée ou linéaire, saturée ou insaturée ; des chaînes aliphatiques de 1 à 7 atomes de carbone ramifiées ou linéaires, saturées ou insaturées; et
- $R_3$ est un groupe phényle avec un ou plusieurs substituants chacun choisi de manière indépendante parmi le groupe constitué par un groupe fluoro, un groupe méthoxy, un groupe éthoxy, un groupe trifluorométhyle, un groupe diméthylamino, un groupe chloro, un groupe cyano, un groupe méthyle, un groupe éthyle, un groupe carboxyméthyle, un groupe méthyltio, un groupe diméthylcarboxamido, un groupe diéthylcarboxamido et un groupe méthylcarboxylate,

et/ou étant un sel d'addition de ceux-ci et/ou un stéréoisomère de ceux-ci et/ou un mono- ou un di-N-oxyde de ceux-ci et/ou un solvate et/ou un dérivé dihydro- ou tétrahydroptéridine de ceux-ci acceptables d'un point de vue pharmaceutique.

**7.** Utilisation d'un dérivé de la ptéridine pour la fabrication d'un médicament pour la prévention ou le traitement d'un trouble chez les mammifères, ledit trouble étant choisi parmi le groupe constitué par les effets toxiques de la TNF-$\alpha$, l'hépatite induite par l'alcool et la cachexie, dans lequel le dérivé de la ptéridine est un composé choisi parmi le groupe constitué par :

- le 2-amino-4-éthoxyptéridine
- le 2-amino-4-éthoxy-6-chloro-ptéridine
- le 2-amino-4-éthoxy-6-(4-méthoxyphényl)-ptéridine
- le 2-amino-4-éthoxy-6-(2-méthoxyphényl)-ptéridine
- le 2-amino-4-éthoxy-6-(3-méthoxyphényl)-ptéridine
- le 2-amino-4-éthoxy-6-(3,4-difluorophényl)-ptéridine
- le 2-amino-4-éthoxy-6-(4-diméthylaminophényl)-ptéridine
- le 2-amino-4-éthoxy-6-(4-trifluorométhylphényl)-ptéridine
- le 2-amino-4-éthoxy-6-(2-thiényl)-ptéridine
- le 2-amino-4-éthoxy-6-(3-thiényl)-ptéridine
- le 2-amino-4-éthoxy-6-(3,4-dichlorophényl)-ptéridine
- le 2-amino-4-éthoxy-6-(4-cyanophényl)-ptéridine
- le 2-amino-4-éthoxy-6-(4-éthoxyphényl)-ptéridine
- le 2-amino-4-éthoxy-6-(4-fluorophényl)-ptéridine
- le 2-amino-4-éthoxy-6-(4-éthylphényl)-ptéridine
- le 2-amino-4-éthoxy-6-(4-acétylphényl)-ptéridine
- le 2-amino-4-éthoxy-6-(3-fluoro-4-méthylphényl)-ptéridine
- le 2-amino-4-éthoxy-6-(4-méthylthiophényl)-ptéridine
- le 2-amino-4-éthoxy-6-(4-N,N-diméthylbenzamido)-ptéridine
- le 2-amino-4-isopropoxyptéridine

- le 2-amino-4-isopropoxy-6-chloroptéridine
- le 2-amino-4-isopropoxy-6-(3-méthyl-4-méthoxyphényl)-ptéridine,
- le 2-amino-4-isopropoxy-6-(3,4-diméthylphényl)-ptéridine,
- le 2-amino-4-isopropoxy-6-(3-chloro-4-trifluorométhylphényl)-ptéridine
- le 2-amino-4-isopropoxy-6-(3-chloro-4-fluorophényl)-ptéridine
- le 2-amino-4-isopropoxy-6-(4-N,N-diéthylbenzamido)-ptéridine
- le 2-amino-4-isopropoxy-6-(4-trifluorométhylphényl)-ptéridine
- le 2-amino-4-isopropoxy-6-(3,4-difluorophényl)-ptéridine
- le 2-amino-4-isopropoxy-6-(4-méthoxyphényl)-ptéridine
- le 2-amino-4-isopropoxy-6-(4-éthoxyphényl)-ptéridine
- le 2-amino-4-isopropoxy-6-(4-N,N-diméthylbenzamido)-ptéridine
- le 2-amino-4-isopropoxy-6-(3-thiényl)-ptéridine
- le 2-amino-4-isopropoxy-6-(4-cyanophényl)-ptéridine
- le 2-amino-4-isopropoxy-6-(4-ester méthylique de l'acide benzoïque)-ptéridine
- le 2-amino-4-isopropoxy-6-(4-acétylphényl)-ptéridine
- le 2-amino-4-isopropoxy-6-(3,4-diméthoxyphényl)-ptéridine
- le 2-amino-4-éthylthio-6-(3,4-diméthoxyphényl)-ptéridine
- le 2-amino-4-isopropylthio-6-(3,4-diméthoxyphényl)-ptéridine
- le 2-amino-4-pentoxy-6-styrylptéridine,
- le 2-amino-4-n-pentoxy-6-(1,2-dibromo-2-phényléthyl)-ptéridine,
- le 2-amino-4-méthoxy-6-styryl-7-méthoxyptéridine,
- le 2-amino-4-diméthylamino-6-phénylptéridine,
- le 2-amino-4-diméthylamino-6-(4-tolyl)ptéridine,
- le 2-amino-4-diméthylamino-6-(4-méthoxyphényl)ptéridine,
- le 2-amino-4-diéthylamino-6-phénylptéridine,
- le 2-amino-4-diéthylamino-6-(4-chlorophényl)ptéridine,
- le 2-amino-4-diéthylamino-6-(4-méthoxyphényl)ptéridine,
- le 2-amino-4-diéthylamino-6-(3,4-diméthoxyphényl)ptéridine,
- le 2-amino-4-dipropylamino-6-phénylptéridine,
- le 2-amino-4-dipropylamino-6-(4-chlorophényl)ptéridine,
- le 2-amino-4-dipropylamino-6-(4-méthoxyphényl)ptéridine,
- le 2-amino-4-dipropylamino-6-(3,4-diméthoxyphényl)ptéridine,
- le 2-amino-4-morpholino-6-phénylptéridine,
- le 2-amino-4-morpholino-6-(4-chlorophényl)ptéridine,
- le 2-amino-4-morpholino-6-(4-méthoxyphényl)ptéridine,
- le 2-amino-4-morpholino-6-(3,4-diméthoxyphényl)ptéridine,
- le 2-amino-4-pipéridino-6-phénylptéridine,
- le 2-amino-4-pipéridino-6-(4-chlorophényl)ptéridine,
- le 2-amino-4-pipéridino-6-(4-méthoxyphényl)ptéridine,
- le 2-amino-4-pipéridino-6-(3,4-diméthoxyphényl)ptéridine,
- le 2-amino-4-N-méthylpipérazino-6-phénylptéridine,
- le 2-amino-4-N-méthylpipérazino-6-(4-chlorophényl)ptéridine,
- le 2-amino-4-N-méthylpipérazino-6-(4-méthoxyphényl)ptéridine,
- le 2-amino-4-méthylpipérazino-6-(3,4-diméthoxyphényl)ptéridine,
- le 2-amino-4-pyrrolidino-6-(4-méthoxyphényl)ptéridine,
- le 2-amino-4-piperazino-6-phénylptéridine,
- le 2-amino-4-piperazino-6-(4-chlorophényl)ptéridine,
- le 2-amino-4-piperazino-6-(4-méthoxyphényl)ptéridine,
- le 2-amino-4-piperazino-6-(3,4-diméthoxyphényl)ptéridine,
- le 2-amino-4-morpholino-6-(3,4,5-triméthoxyphényl)ptéridine,
- le 2-amino-4-morpholino-6-(3,4-formilidène-3,4-dihydroxyphényl)ptéridine,
- le 2-amino-4-diméthylamino-6-(3,4-formylidène-3,4-dihydroxyphényl)ptéridine,
- le 2-amino-4-pyrrolidino-6-(3,4,diméthoxyphényl)ptéridine,
- le 2-amino-4-diméthylamino-6-(3,4-diméthoxyphényl)ptéridine,
- le 2-amino-4-diméthylamino-6-méthylptéridine,
- le 2-amino-4-éthoxy-6-phénylptéridine,
- le 2-amino-4-propylamino-6-phénylptéridine,
- le 2-amino-4-propylamino-6-(3,4-diméthoxyphényl)ptéridine,

- le 2-acétamido-4-isopropoxy-6-(3,4-diméthoxyphényl)ptéridine,
- le 2-amino-4-éthoxy-6-(3,4-diméthoxyphényl)ptéridine,
- le 2-amino-4-(1,2,3,6-tétrahydropyridinyl)-6-(3,4-diméthoxyphényl)ptéridine,
- le 2-amino-4-éthoxyptéridine
- le 2-amino-4-éthoxyptéridine $N^8$-oxyde
- le 2-amino-4-isopropoxyptéridine $N^8$-oxyde
- le 2-amino-6-chloro-4-éthoxyptéridine
- le 2-amino-6-chloro-4-isopropoxyptéridine
- le 2-amino-6-(p-méthoxyphényl)-4-éthoxy-ptéridine ;
- le 2-amino-6-(o-méthoxyphényl)-4-éthoxy-ptéridine ;
- le 2-amino-6-(m-méthoxyphényl)-4-éthoxy-ptéridine ;
- le 2-amino-6-(3,4-difluorophényl)-4-éthoxy-ptéridine ;
- le 2-amino-6-(p-diméthylaminophényl)-4-éthoxy-ptéridine ;
- le 2-amino-6-(p-trifluorométhylphényl)-4-éthoxy-ptéridine ;
- le 2-amino-6-(2-thiényl)-4-éthoxy-ptéridine ;
- le 2-amino-6-(3-thiényl)-4-éthoxy-ptéridine ;
- le 2-amino-6-(3,4-dichlorophényl)-4-éthoxy-ptéridine ;
- le 2-amino-6-(p-cyanophényl)-4-éthoxy-ptéridine ;
- le 2-amino-6-(p-éthoxyphényl)-4-éthoxy-ptéridine ;
- le 2-amino-6-(p-fluorophényl)-4-éthoxy-ptéridine ;
- le 2-amino-6-(p-éthylphényl)-4-éthoxy-ptéridine ;
- le 2-amino-6-(p-acétylphényl)-4-éthoxy-ptéridine ;
- le 2-amino-6-(3-méthyl-4-fluorophényl)-4-éthoxy-ptéridine ;
- le 2-amino-6-(p-thiométhylphényl)-4-éthoxy-ptéridine ;
- le 2-amino-6-(p-N,N-diméthylbenzamido)-4-éthoxy-ptéridine ;
- le 2-amino-6-(3,4-diméthoxyphényl)-4-éthoxy-ptéridine ;
- le 2-amino-6-(3-méthyl-4-méthoxyphényl)-4-isopropoxyptéridine
- le 2-amino-6-(3,4-diméthylphényl)-4-isopropoxyptéridine ;
- le 2-amino-6-(3-chloro-4-trifluorométhylphényl)-4-isopropoxyptéridine ;
- le 2-amino-6-(3-chloro-4-fluorophényl)-4-isopropoxyptéridine
- le 2-amino-6-(p-N,N-diéthylbenzamido)-4-isopropoxyptéridine ;
- le 2-amino-6-(p-trifluorométhylphényl)-4-isopropoxyptéridine ;
- le 2-amino-6-(3,4-difluorophényl)-4-isopropoxyptéridine ;
- le 2-amino-6-(p-méthoxyphényl)-4-isopropoxyptéridine ;
- le 2-amino-6-(p-éthoxyphényl)-4-isopropoxyptéridine ;
- le 2-amino-6-(p-diméthylbenzamido)-4-isopropoxyptéridine ;
- le 2-amino-6-(3-thiényl)-4-isopropoxyptéridine ;
- le 2-amino-6-(p-cyanophényl)-4-isopropoxyptéridine ;
- le 2-amino-6-(ester méthylique de l'acide p-benzoïque)-isopropoxyptéridine ;
- le 2-amino-6-(p-acétylphényl)-4-isopropoxyptéridine ;
- le 2-amino-6-(3,4-diméthoxyphényl)-4-isopropoxyptéridine ;
- le 2-amino-4-mercaptoéthyl-6-(3,4-diméthoxyphényl)ptéridine ;
- le 2-amino-4-mercaptoisopropyl-6-(3,4-diméthoxyphényl)ptéridine,
- le 2-acétylamino-4-(1,2,4-triazolyl)-6-(p-méthoxyphényl)ptéridine,
- le 2-acétylamino-4-(1,2,4-triazolyl)-7-(p-méthoxyphényl)ptéridine,
- le 2-amino-4-isopropoxy-7-(p-méthoxyphényl)ptéridine,
- le 2-amino-4-isopropoxy-7-(3,4-diméthoxyphényl)ptéridine,
- le 2-amino-4-éthoxy-7-(3,4-diméthoxyphényl)ptéridine,
- le 2-amino-4-méthoxy-7-(3,4-diméthoxyphényl)ptéridine,
- le 2-amino-4-(1,2,3,6-tétrahydropyridinyl)-6-(3,4-diméthoxyphényl)ptéridine,
- le 2-amino-4-(diéthylamino)-6-[[3,4-(diméthoxyphényl)]ptéridine,
- le 2-amino-4-thiomorpholino-6-[[3,4-(diméthoxyphényl)]ptéridine,
- le 2-amino-4-morpholino-6-(4-acétanilide)ptéridine,
- le 2-amino-4-morpholino-6-(3-acétanilide)ptéridine,
- le 2-amino-4-morpholino-6-(4-aminophényl)ptéridine,
- le 2-amino-4-morpholino-6-(3-aminophényl)ptéridine,
- le 2-amino-4-morpholino-6-(4-benzoylaminophényl)ptéridine ;
- le 2-amino-4-morpholino-6-(4-phénoxyacétylaminophényl)ptéridine ;

- le 2-amino-4-morpholino-6-(4-propionylaminophényl)ptéridine ;
- le 2-amino-4-morpholino-6-(4-furoylaminophényl)ptéridine ;
- le 2-amino-4-morpholino-6-(4-cyclohexanoylaminophényl)ptéridine ;
- le 2-amino-4-morpholino-6-[4-(4-chlorobenzoyl)aminophényl]ptéridine
- le 2-amino-4-morpholino-6-(4-benzyloxyacétylaminophényl)ptéridine ;
- le 2-amino-4-morpholino-6-(4-isonicotinoylaminophényl)ptéridine ;
- le 2-amino-4-morpholino-6-(4-naphtoylaminophényl)ptéridine ;
- le 2-amino-4-morpholino-6-(4-méthylsulfonylaminophényl)ptéridine ;
- le 2-amino-4-morpholino-6-(4-éthylsuccinylaminophényl)ptéridine ;
- le 2-amino-4-morpholino-6-[4-(4-méthylbenzoate)aminophényl]ptéridine ;
- le 2-amino-4-morpholino-6-(3-benzoylaminophényl)ptéridine
- le 2-amino-4-morpholino-6-(3-benzènesufonylaminophényl)ptéridine ;
- le 2-amino-4-morpholino-6-(3-phénoxyacétylaminophényl)ptéridine ;
- le 2-amino-4-morpholino-6-(3-isonicotinoylaminophényl)ptéridine,
- le 2-amino-4-morpholino-6-(3-cyclohexanoylaminophényl)ptéridine ;
- le 2-amino-4-morpholino-6-[3-(4-méthylbenzoate)aminophényl]ptéridine ;
- le 2-amino-4-morpholino-6-(3-éthylsuccinylaminophényl)ptéridine ;
- le 2-amino-4-morpholino-6-(3-éthylmalonylaminophényl)ptéridine ;
- le 2-amino-4-morpholino-6-(3-benzyloxyacétylaminophényl)ptéridine ;
- le 2-amino-4-morpholino-6-(3-éthylsulfonylaminophényl)ptéridine ;
- le 2-amino-4-morpholino-6-[3-Boc-(L)-phénylalanineaminophényl]ptéridine ;
- le 2-amino-4-morpholino-6-[3-Boc-(D)-phénylalanineaminophényl]ptéridine ;
- le 2-amino-4-morpholino-6-[3-Boc-(L)-tryptophaneaminophényl]ptéridine ;
- le 2-amino-4-morpholino-6-[3-Boc-(D)-tryptophaneaminophényl]ptéridine ;
- le 2-amino-4-morpholino-6-(4-hydroxyphényl)ptéridine ;
- le 2-amino-4-morpholino-6-(4-éthoxyphényl)ptéridine ;
- le 2-amino-4-morpholino-6-(4-benzyloxyphényl)ptéridine ;
- le 2-amino-4-morpholino-6-(4-(phénéthyloxy)-phényl)ptéridine ;
- le 2-amino-4-morpholino-6-(4-phénoxy-butyronitrile)ptéridine ;
- le 2-amino-4-morpholino-6-(4-propoxyphényl)ptéridine ;
- le 2-amino-4-morpholino-6-(4-ester éthylique de l'acide phénoxybutyrique)ptéridine ;
- le 2-amino-4-morpholino-6-(4-ester éthylique de l'acide phénoxyacétique)ptéridine
- le 2-amino-4-morpholino-6-(4-(2-méthoxyéthoxy)-phényl)ptéridine ;et
- le 2-amino-4-morpholino-6-(4-butoxyphényl)-ptéridine.

## Figure 1

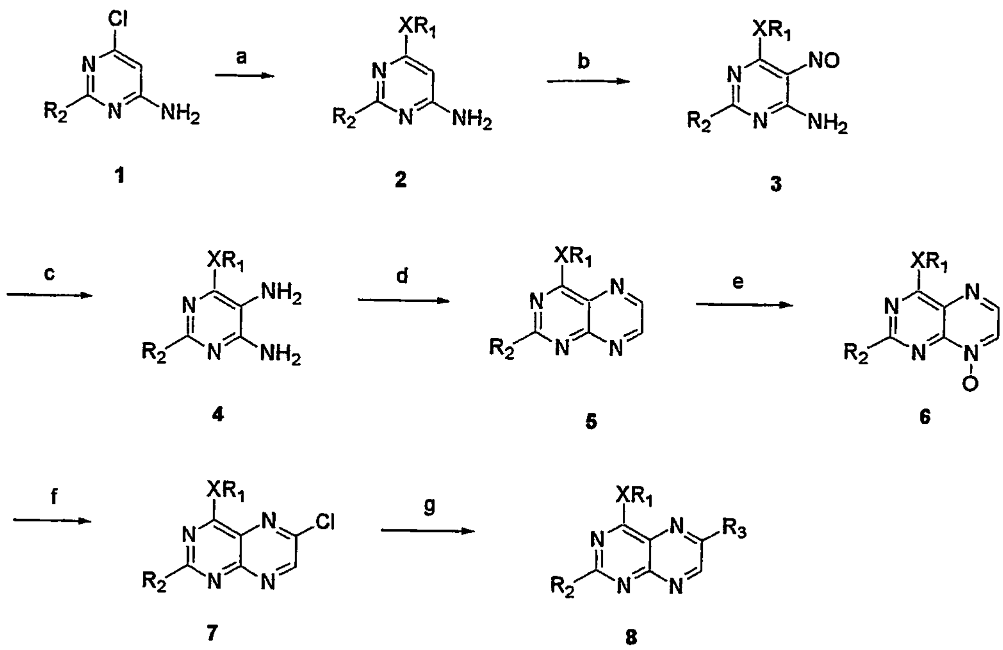


1 2 3 4 5 6 7 8

Figure 2

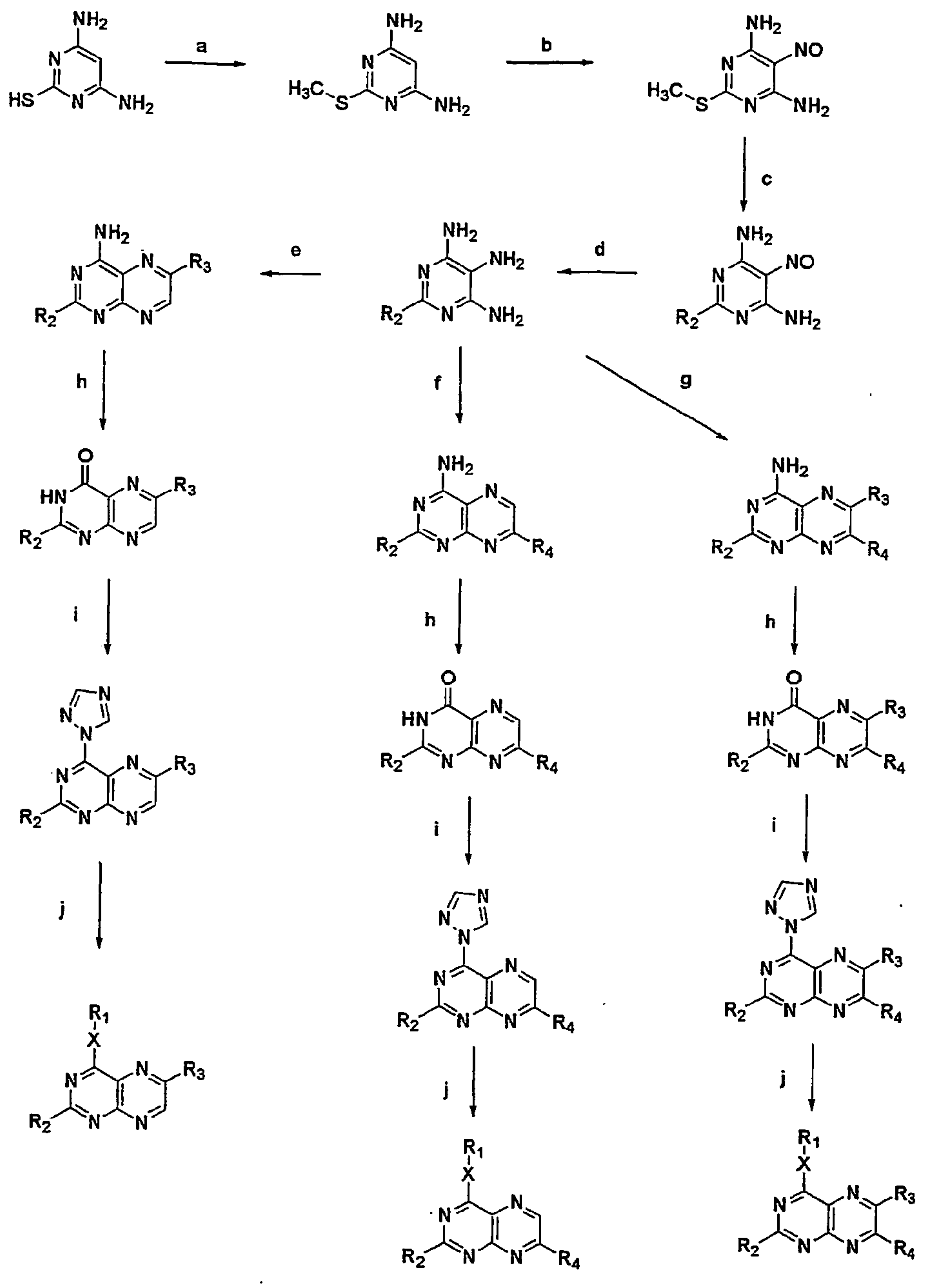

Figure 3

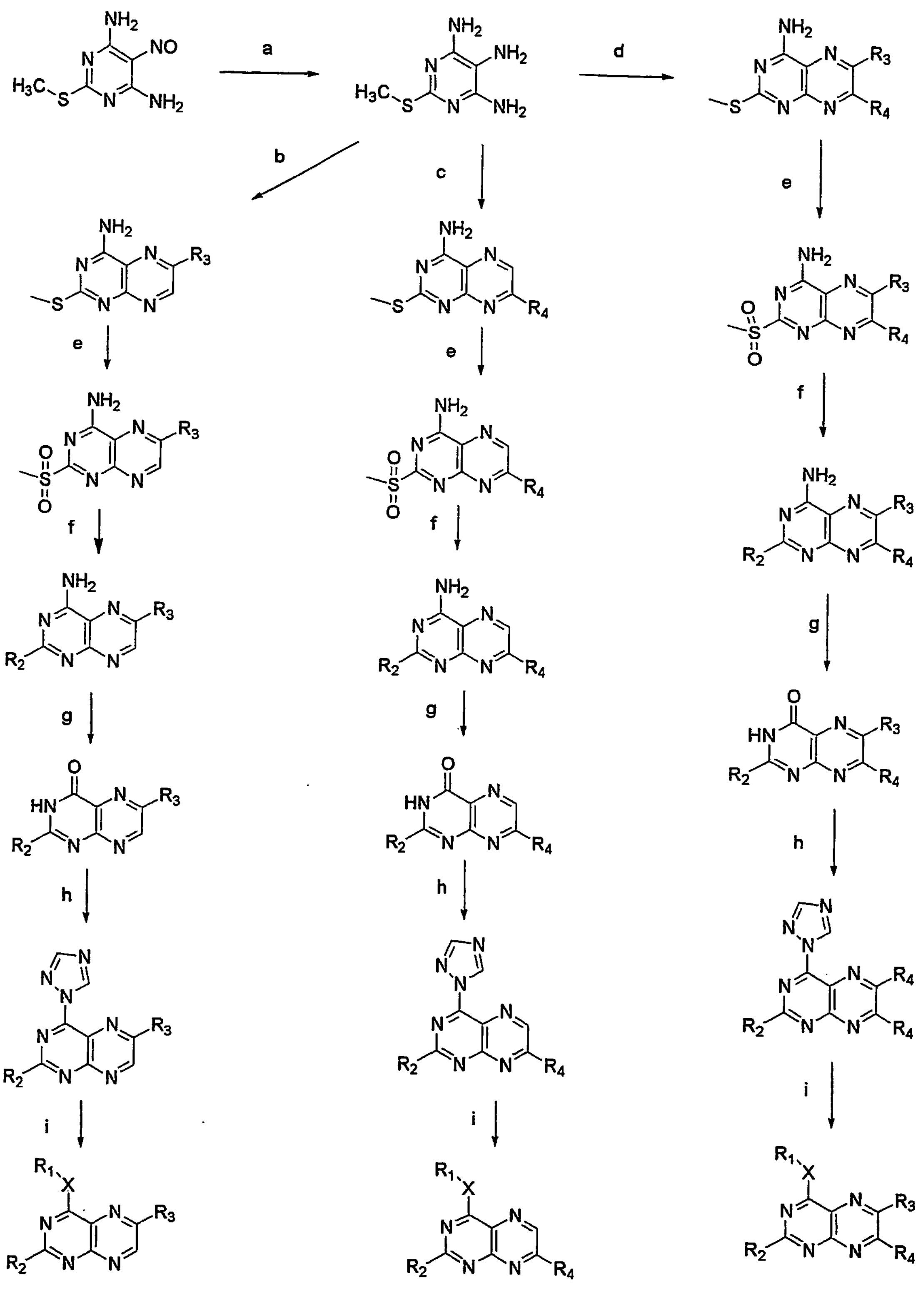

Figure 4

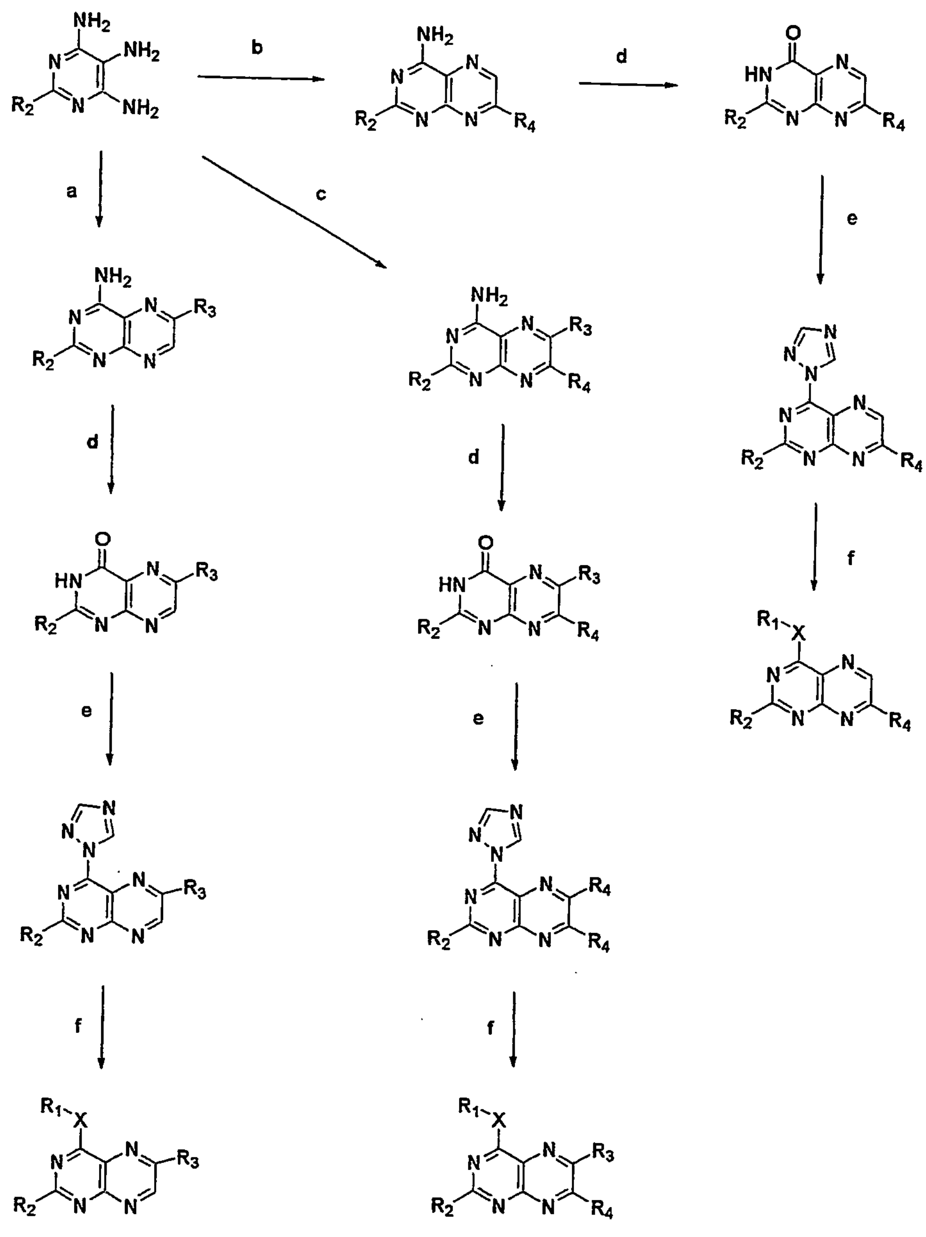

Figure 5

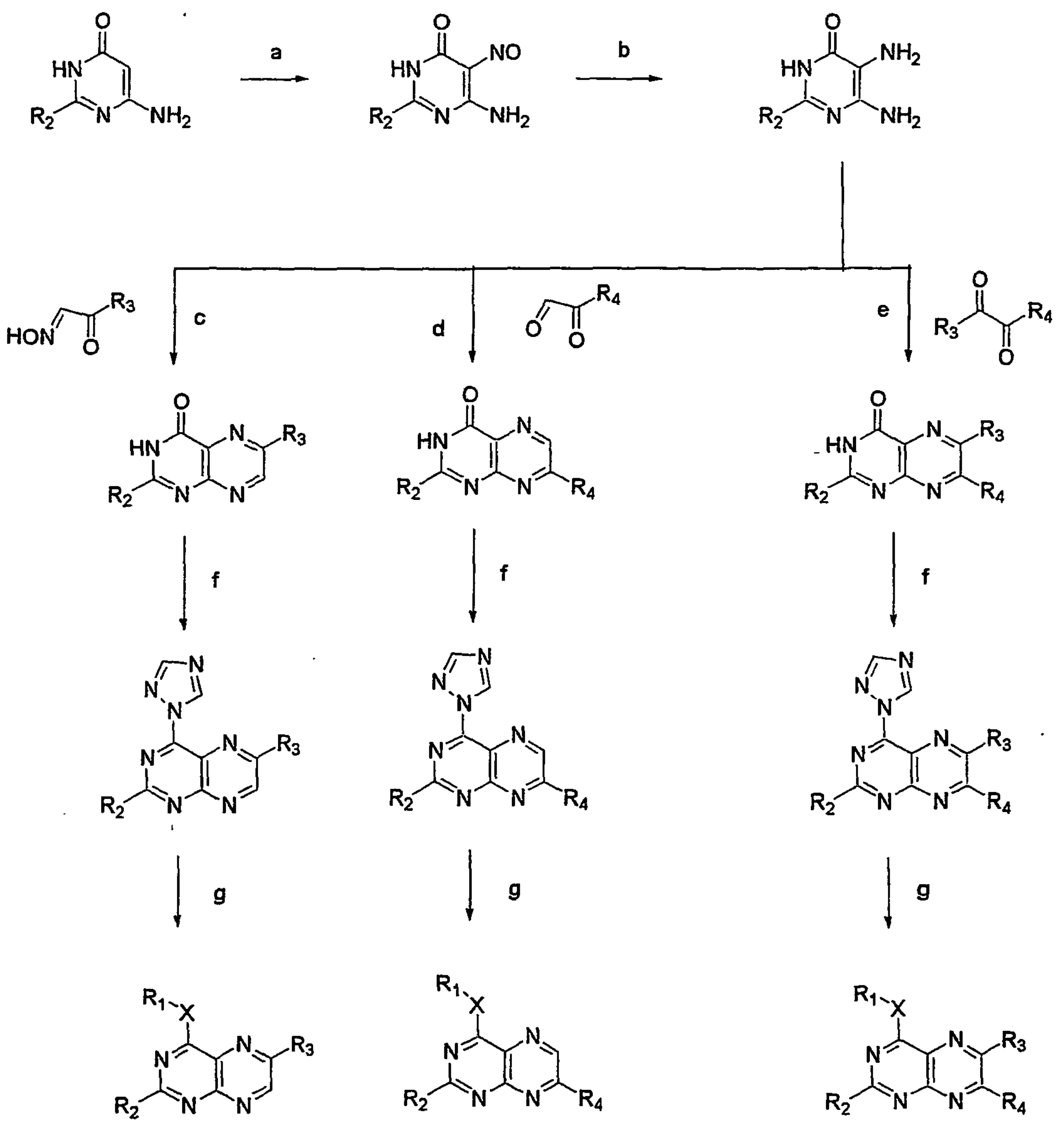

Figure 6 (reference)

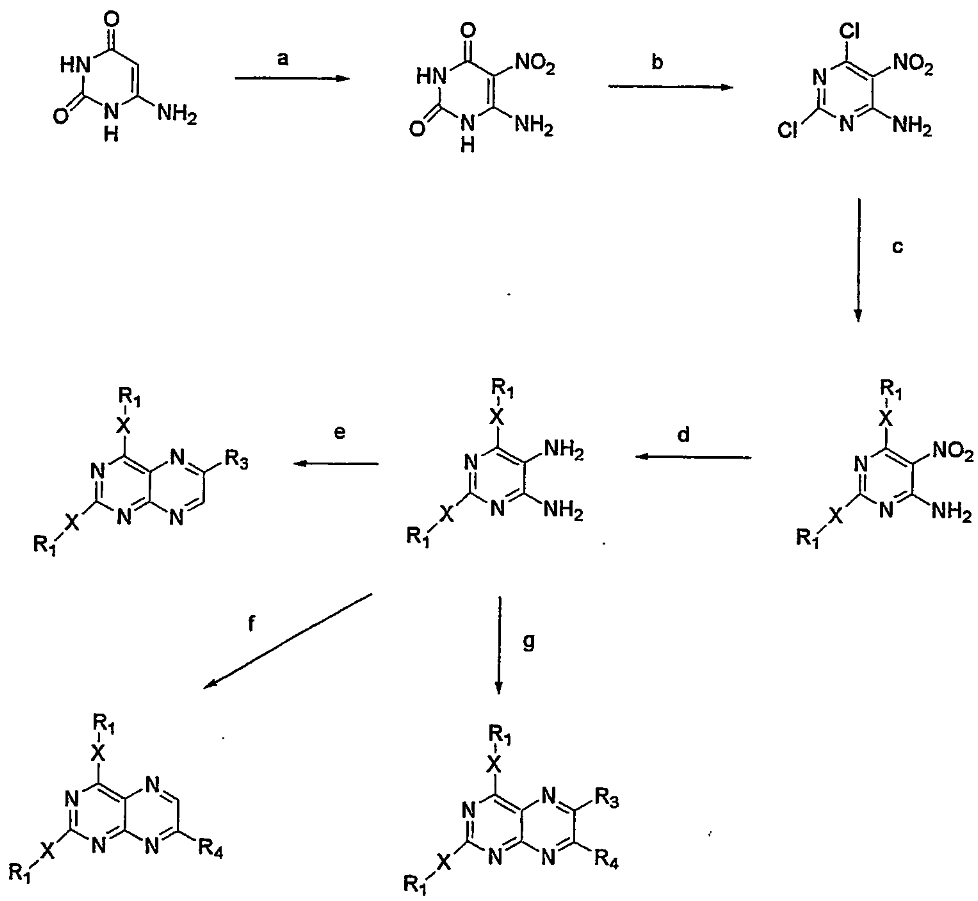

Figure 7

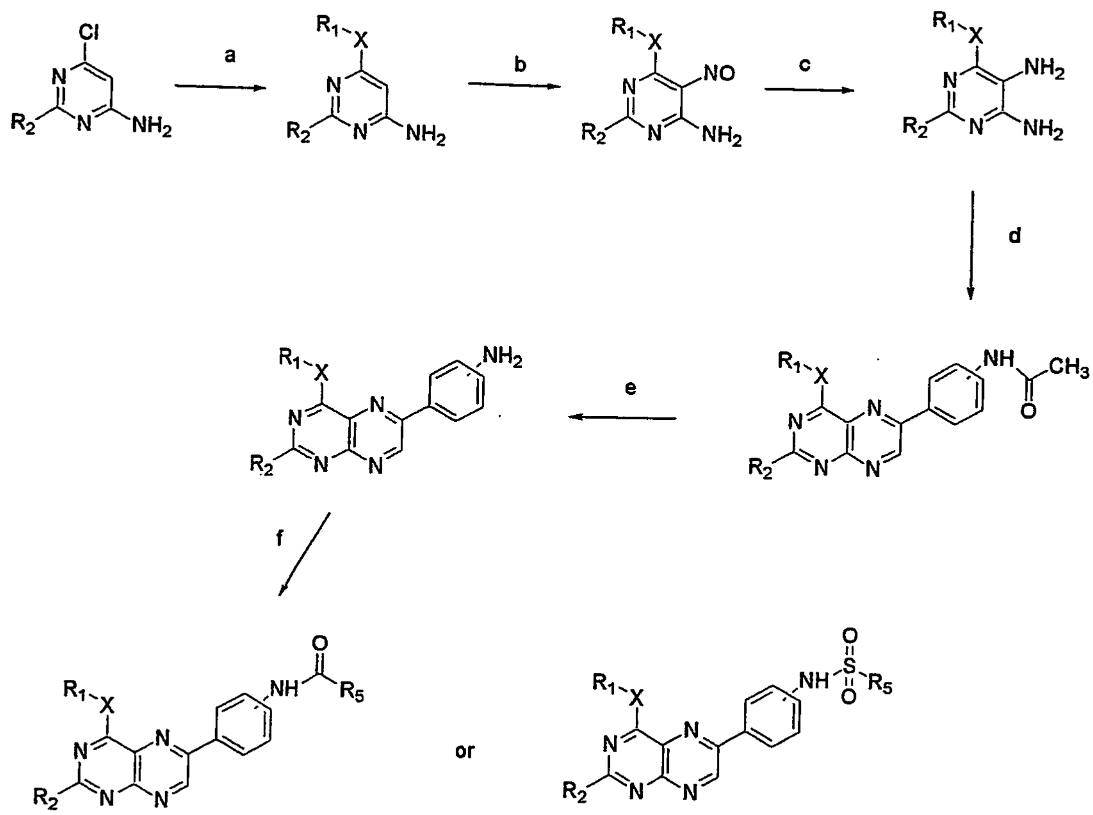


or

Figure 8

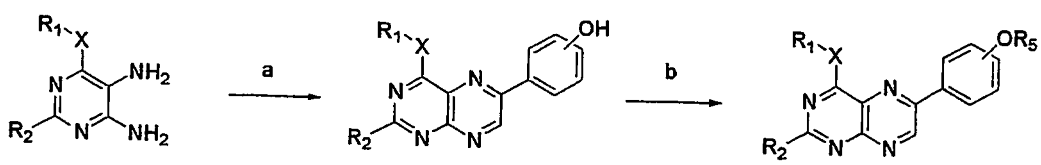

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2512572 A
- WO 0039129 A
- WO 0121619 A
- US 2581889 A
- GB 677342 A


### Non-patent literature cited in the description


- **NENOVA et al.** *Archives of Hellenic Medicine,* 2000, vol. 17, 619-621
- **SAARINEN et al.** *Cancer Research,* 1990, vol. 50, 592-595
- **RAUTONEN et al.** *AIDS,* 1991, vol. 5, 1319-1325
- **SWAPAN et al.** *Journal of Virology,* 2002, vol. 76, 11710-11714
- McCutcheon's Detergents and Emulsifiers Annual. MC Publishing Crop, 1981
- Tensid-Taschenbuch. Hanser Verlag, 1981
- Encyclopaedia of Surfactants. Chemical Publishing Co, 1981
- **MOHR et al.** *Helv. Chem. Acta,* 1992, vol. 75, 2317
- **W. PFLEIDERER et al.** *Chem. Ber.,* 1961, vol. 94, 12
- **LANDAUER et al.** *J. Chem. Soc.,* 1953, 3721-3722
- **BADDILEY et al.** *J. Chem. Soc.,* 1943, vol. 383
- **TAYLOR et al.** *J. Am. Chem. Soc.,* 1952, vol. 74, 1644-1647